# EUROPÄISCHE PATENTSCHRIFT

(11) **EP 1 881 967 B1**
(45) Veröffentlichungstag und Bekanntmachung des Hinweises auf die Patenterteilung: **28.04.2010**
(21) Anmeldenummer: 06753695.3
(22) Anmeldetag: 18.05.2006
(51) Int. Cl.: C07D 261/20, C07D 413/12, A61K 31/423, A61K 31/4439, A61P 29/00

(54) **SUBSTITUIERTE BENZO (D) ISOAXOL-3-YL-AMIN-VERBINDUNGEN ALS ANALGETIKA**
SUBSTITUTED BENZO(D)ISOXAZOL-3-YL AMINE COMPOUNDS AS ANALGESICS
COMPOSES BENZO(D)ISOAXOL-3-YL-AMINE SUBSTITUES UTILISES COMME ANALGESIQUES

(30) Priorität: 18.05.2005 DE 102005023589; 16.08.2005 DE 102005038947
(43) Veröffentlichungstag der Anmeldung: 30.01.2008
(73) Patentinhaber: Grünenthal GmbH, 52078 Aachen (DE)
(72) Erfinder: MERLA, Beatrix, 52078 Aachen (DE); FRANK, Robert, 52070 Aachen (DE); BAHRENBERG, Gregor, 52078 Aachen (DE); SCHRÖDER, Wolfgang, 52074 Aachen (DE); ZEMOLKA, Saskia, 78465 Konstanz (DE)
(86) Internationale Anmeldenummer: PCT/EP2006/004700
(87) Internationale Veröffentlichungsnummer: WO 2006/122800

(56) Entgegenhaltungen:
- WO-A-02/066036
- WO-A-03/040113
- US-A1- 2002 156 120
- US-A1- 2002 193 597

## Beschreibung

Die vorliegende Erfindung betrifft substituierte Benzo[d]isoxazol-3-yl-amin-Verbindungen, Verfahren zu ihrer Herstellung, Arzneimittel enthaltend diese Verbindungen sowie die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein pathophysiologisches Merkmal von chronischen Schmerzen besteht in der Überregbarkeit von Neuronen. Die neuronale Erregbarkeit wird entscheidend von der Aktivität von K⁺ Kanälen beeinflusst, da diese das Ruhemembranpotential der Zelle und somit die Erregbarkeitsschwelle maßgeblich bestimmen. Heteromere K⁺ Kanäle vom molekularen Subtyp KCNQ2/3 (Kv7.2/7.3) sind in Neuronen verschiedener Regionen des zentralen (Hippocampus, Amygdala) und peripheren (Hinterwurzelganglien) Nervensystems exprimiert und regulieren deren Erregbarkeit. Die Aktivierung von KCNQ2/3 K⁺ Kanälen führt zu einer Hyperpolarisation der Zellmembran und damit einhergehend zu einer Abnahme der elektrischen Erregbarkeit dieser Neurone. KCNQ2/3 exprimierende Neurone der Hinterwurzelganglien sind an der Übertragung nociceptiver Erregungen von der Peripherie ins Rückenmark beteiligt (Passmore et al., 2003). Dementsprechend konnte für den KCNQ2/3 Agonisten Retigabine eine analgetische Wirksamkeit in präklinischen Neuropathie- und Entzündungsschmerzmodellen nachgewiesen werden (Blackburn-Munro and Jensen, 2003; Passmore et al., 2003; Dost et al., 2004). Der KCNQ2/3 K⁺ Kanal stellt somit einen geeigneten Ansatzpunkt zur Behandlung von Schmerz; insbesondere von Schmerz ausgewählt aus der Gruppe bestehend aus chronischem Schmerz, neuropathischem Schmerz, inflammatorischem Schmerz und muskulärem Schmerz (Nielsen et al., 2004), insbesondere von neuropathischem und inflammatorischem Schmerz dar. Darüber hinaus ist der KCNQ2/3 K⁺ Kanal ein geeignetes Target für die Therapie einer Vielzahl weiterer Erkrankungen wie beispielsweise Migräne (US2002/0128277), kognitive Erkrankungen (Gribkoff, 2003), Angstzuständen (Korsgaard et al., 2005), Epilepsie (Wickenden et al., 2004) und Harninkontinenz (Streng et al., 2004).

Aus US 2002/193597 sind bereits Benzo[d]isoxazolyl-3-yl-amine und deren Eignung zur Modulation von KCNQ K⁺ Kanälen bekannt. Diese weisen an der in 3 Position exozyklisch gebundenen Aminogruppe umittelbar angebundene Ringsysteme auf.

Eine Aufgabe der vorliegenden Erfindung bestand nun darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch KCNQ2/3 K⁺ Kanäle vermittelt werden.

Überraschenderweise wurde nun gefunden, dass substituierte Benzo[d]isoxazol-3-yl-amin-Verbindungen der nachstehend angegebenen allgemeinen Formel I sich zur Behandlung von Schmerzen eignen und auch eine ausgezeichnete Affinität zum KCNQ2/3 K⁺ Kanal aufweisen und sich daher zur Behandlung von Störungen oder Krankheiten eignen, die zumindest teilweise durch KCNQ2/3 K⁺ Kanäle vermittelt werden.

Ein Gegenstand der vorliegenden Erfindung sind daher substituierte Benzo[d]isoxazol-3-yl-amin-Verbindungen der allgemeinen Formel I, 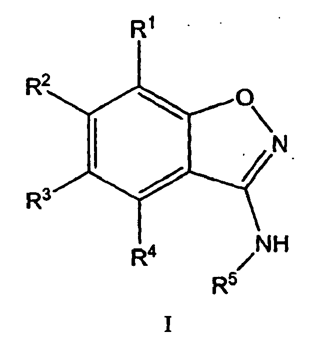 R¹, R², R³ und R⁴, unabhängig voneinander, jeweils für
H; F; Cl; Br; I; -CN; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; C₁₋₁₀-Alkyl, C₂₋₁₀-Alkenyl, C₂₋₁₀-Alkinyl;
R⁵ für
-C(=S)NR²¹R²² oder (CHR⁶)ₙ-R²⁵
mit n = 1, 2 oder 3,
wobei R⁶ für H, C₃₋₈-Cycloalkyl oder C₁₋₆-Alkyl steht und R²⁵ Aryl oder Heteroaryl bedeutet
R⁷ und R⁸, unabhängig voneinander, jeweils für
H, -C(=O)R¹⁴ oder C₁₋₁₀-Alkyl stehen, oder
R⁷ und R⁸ zusammen mit dem sie verbindenden Stickstoffatom als Ringglied einen Rest bilden, der ausgewählt ist aus Morpholin, Piperidin oder Pyrrolidin;
R⁹, R¹⁰, R¹¹ und R¹⁶ unabhängig voneinander, jeweils für
H; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
R¹² und R¹³, unabhängig voneinander, jeweils für
H oder für einen C₁₋₁₀-Alkyl-Rest; oder
R¹² und R¹³ zusammen mit dem sie verbindenden Stickstoffatom als Ringglied einen Rest bilden, der ausgewählt ist aus Morpholin, Piperidin oder Pyrrolidin;
R¹⁴ für
-NR⁷R⁸; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
R¹⁵ und R¹⁷, unabhängig voneinander, jeweils für
C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
R²¹ und R²², unabhängig voneinander, jeweils für H,
C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
wobei
die vorstehend genannten C₁₋₁₀-Alkyl-, C₂₋₁₀-Alkenyl- und C₂₋₁₀-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; COOH; COOC₁₋₄-Alkyl; -CN; -OH; -SH; -O-C₁₋₂-Alkyl; -S-C₁₋₂-Alkyl und -NH₂ substituiert sein können,
die vorstehend genannten C₃₋₈-Cycloalkyl-Reste jeweils ggf. mit 1, 2, 3, 4 oder 5 Substituenten unabhängig voneinander ausgewählt aus der Gruppe bestehend aus F; Cl; Br; -CN; -OH; -SH; -C₁₋₅-Alkyl; -O-C₁₋₂-Alkyl; -S-C₁₋₂-Alkyl und -NH₂ substituiert sein können,
die vorstehend genannten Heterocycloalkyl-Reste jeweils 4-, 5-, 6- oder 7-gliedrig sind, 1 oder 2 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; -CN; -OH; -SH; -C₁₋₅-Alkyl; -O-C₁₋₂-Alkyl; -S-C₁₋₂-Alkyl und -NH₂ substituiert sein können,
die vorstehend genannten Aryl-Reste jeweils für einen Phenyl-, Anthracenyl- oder Naphthyl-Rest stehen, der mit 1, 2, 3, 4 oder 5 Substituenten unabhängig voneinander ausgewählt aus der Gruppe bestehend aus F; Cl; Br; I; -CF₃;
-OCF₃; -SCF₃; C(=O)C₁₋₅-Alkyl; 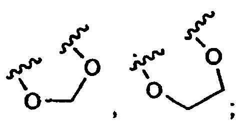 -NO₂; Cyclohexyl; -O(C=O)C₁₋₂-Alkyl;
-SF₅; -CN; -OH; -SH; -C₁₋₅-Alkyl; -O-C₁₋₅-Alkyl; -S-C₁₋₅-Alkyl; -C(=O)-OH; -C(=O)-OC₁₋₅-Alkyl; -NH₂; -N(H)(C₁₋₅-Alkyl); -N(C₁₋₅-Alkyl)(C₁₋₅-Alkyl); -C(=O)NH₂; -C(=O)N(H)(C₁₋₅-Alkyl); -C(=O)N(C₁₋₅-Alkyl)(C₁₋₅-Alkyl); -S(=O)₂NH₂; -S(=O)₂N(H)(C₁₋₅-Alkyl); -S(=O)₂N(C₁₋₅-Alkyl)(C₁₋₅-Alkyl); -S(=O)₂-Phenyl; -S(=O)₂-C₁₋₅-Alkyl; Phenyl; Phenoxy, Benzyl; Benzyloxy; Thiophenyl (Thienyl), Furanyl und Pyridinyl substituiert sein können,
die vorstehend genannten Heteroaryl-Reste jeweils 5- oder 6-gliedrig sind, 1, 2 oder 3 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; I; -CF₃; -OCF₃; -SCF₃; -SF₅; -CN; -OH; -SH; -C₁₋₅-Alkyl; -O-C₁₋₅-Alkyl; -S-C₁₋₅-Alkyl; -C(=O)-OH; -C(=O)-OC₁₋₅-Alkyl; -NH₂; -N(H)(C₁₋₅-Alkyl); -N(C₁₋₅-Alkyl)(C₁₋₅-Alkyl); -C(=O)NH₂; -C(=O)N(H)(C₁₋₅-Alkyl); -C(=O)N(C₁₋₅-Alkyl)(C₁₋₅-Alkyl); -S(=O)₂NH₂; -S(=O)₂N(H)(C₁₋₅-Alkyl); -S(=O)₂N(C₁₋₅-Alkyl)(C₁₋₅-Alkyl); -S(=O)₂-Phenyl; -S(=O)₂-C₁₋₅-Alkyl; Phenyl; Phenoxy, Benzyl; Thiophenyl (Thienyl), Furanyl und Pyridinyl substituiert sein können,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren:

Als (hetero)cycloaliphatische Reste seien beispielsweise Cyclopropyl, Cyclobutyl, Cyclopentyl, Cyclohexyl, Cycloheptyl, Cyclooctyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl genannt.

Als Aryl-Reste seien beispielsweise Phenyl und Naphthyl (umfassend 1-Naphthyl und 2-Naphthyl) genannt.

Als Heteroaryl-Reste seien beispielsweise Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinoxalinyl, Chinolinyl und Isochinolinyl genannt.

Alkenyl-Reste weisen wenigstens eine, vorzugsweise 1, 2, 3 oder 4 C-C-Doppelbindungen und Alkinyl-Reste wenigstens eine, vorzugsweise 1, 2, 3 oder 4 C-C-Dreifachbindungen auf.

Bevorzugt sind Alkyl-Reste ausgewählt aus der Gruppe bestehend aus Methyl, Ethyl, n-Propyl, Isopropyl, n-Butyl, sec-Butyl, isobutyl, tert-Butyl, n-Pentyl, sec-Pentyl, neoPentyl und n-Hexyl, die ggf. mit 1, 2, 3, 4, oder 5 Substituenten unabhängig voneinander ausgewählt aus der Gruppe bestehend aus F, Cl, Br, -CN, -OH, -NH₂, -SH, -OCH₃, -O-C₂H₅, -SCH₃, -S-C₂H₅, substituiert sein können.

Bevorzugt sind ferner Alkenyl-Reste ausgewählt aus der Gruppe bestehend aus Vinyl, 1-Propenyl, 2-Propenyl, 1-Butenyl, 2-Butenyl, 3-Butenyl, 2-Methyl-buten-2-yl, 1-Pentenyl, 2-Pentenyl, 3-Pentenyl und 4-Pentenyl, die ggf. mit 1, 2 oder 3 Substituenten unabhängig voneinander ausgewählt aus der Gruppe bestehend aus F, Cl, Br, -CN, -OH, -NH₂, -SH, -OCH₃, -O-C₂H₅, -SCH₃, -S-C₂H₅, substituiert sein können.

Weiterhin bevorzugt sind Alkinyl-Reste ausgewählt aus der Gruppe bestehend aus Ethinyl, 1-Propinyl, 2-Propinyl, 1-Butinyl, 2-Butinyl und 3-Butinyl, die ggf. mit 1, 2 oder 3 Substituenten unabhängig voneinander ausgewählt aus der Gruppe bestehend aus F, Cl, Br, -CN, -OH, -NH₂, -SH, -OCH₃, -O-C₂H₅, -SCH₃, -S-C₂H₅, substituiert sein können.

Bevorzugt sind erfindungsgemäße Verbindungen gemäß Formel I, R¹, R², R³ und R⁴, unabhängig voneinander, jeweils für
H; F; Cl; Br; I; -CN; -NR⁷R⁸; -OR⁹; -SR¹⁰; C₁₋₄-Alkyl, C₂₋₄-Alkenyl oder C₂₋₄-Alkinyl;
R⁵ für
-C(=S)NR²¹R²²
oder
-(CHR⁶)ₙ-R²⁵
mit n = 1, 2 oder 3
wobei R⁶ für H oder C₁₋₆-Alkyl steht
und R²⁵ Aryl oder Heteroaryl bedeutet
R⁷ und R⁸, unabhängig voneinander, jeweils für
H, -C(=O)R¹⁵ oder C₁₋₄-Alkyl stehen, oder
R⁷ und R⁸ zusammen mit dem sie verbindenden Stickstoffatom als Ringglied einen Rest bilden, der ausgewählt ist aus Morpholin, Piperidin oder Pyrrolidin;
R⁹, R¹⁰ unabhängig voneinander, jeweils für
H; C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl; -(C_{1, 2 oder 3}-Alkylen)-Heteroaryl;
R²¹ und R²², unabhängig voneinander, jeweils für
H; C₁₋₁₀-Alkyl, C₂₋₁₀-Alkenyl, C₂₋₁₀-Alkinyl; C₃₋₈-Cycloalkyl; -(C_{1, 2 oder 3}-Alkylen)-C₃₋₈-Cycloalkyl; Heterocycloalkyl; -( C_{1, 2 oder 3}-Alkylen)-Heterocycloalkyl; Aryl; Heteroaryl; -(C_{1, 2 oder 3}-Alkylen)-Aryl; -(C_{1, 2 oder 3}-Alkylen)-Heteroaryl;
wobei
die vorstehend genannten C₁₋₁₀-Alkyl-, C₂₋₁₀-Alkenyl- und C₂₋₁₀-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; -CN; COOH; COOC₁₋₄-Alkyl; -OH; -SH; -O-C₁₋₂-Alkyl; -S-C₁₋₂-Alkyl und -NH₂ substituiert sein können,
die vorstehend genannten C₁₋₄-Alkyl-, C₂₋₄-Alkenyl- und C₂₋₄-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; -CN; -OH; -OCH₃ und -NH₂ substituiert sein können,
die vorstehend genannten C₃₋₈-Cycloalkyl-Reste jeweils ggf. mit 1, 2, 3, 4 oder 5 Substituenten unabhängig voneinander ausgewählt aus der Gruppe bestehend aus F; Cl; Br; -CN; -OH; -CH₃; -C₂H₅; -OCH₃; und -NH₂ substituiert sein können,
die vorstehend genannten Heterocycloalkyl-Reste jeweils 4-, 5-, 6- oder 7-gliedrig sind, 1 oder 2 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; -CN; -OH; -CH₃; -C₂H₅; -OCH₃; und -NH₂ substituiert sein können,
die vorstehend genannten Aryl-Reste jeweils für einen Phenyl-, Anthracenyl- oder Naphthyl-Rest stehen, der mit 1, 2, 3, 4 oder 5 Substituenten unabhängig voneinander ausgewählt aus der Gruppe bestehend aus F; Cl; Br; -CF₃; -OCF₃; -SCF₃; 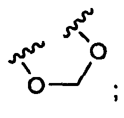 -NO₂, -C(=O)C₁₋₂-Alkyl; Cyclohexyl; -O(C=O)C₁₋₂-Alkyl;
-SF₅; -CN; -OH; Methyl; Ethyl; n-Propyl; iso-Propyl; n-Butyl; iso-Butyl; sec-Butyl; tert.-Butyl; Methoxy; Ethoxy; -NH₂; -N(CH₃)₂; -N(C₂H₅)₂; Phenyl; Benzyloxy; Phenoxy, Benzyl; Thiophenyl (Thienyl), Furanyl und Pyridinyl substituiert sein können;
die vorstehend genannten Heteroaryl-Reste jeweils für einen Furanyl-, Thienyl- (Thiophenyl-) oder Pyridinyl-Rest stehen und ggf. mit 1, 2, 3, 4 oder 5 Substituenten unabhängig voneinander ausgewählt aus der Gruppe bestehend F; Cl; Br; -CF₃; -OCF₃; -SCF₃; -SF₅; -CN; -OH; Methyl; Ethyl; n-Propyl; iso-Propyl; n-Butyl; iso-Butyl; sec-Butyl; tert.-Butyl; Methoxy; Ethoxy; -NH₂; -N(CH₃)₂; -N(C₂H₅)₂; Phenyl; Phenoxy, Benzyl; Thiophenyl (Thienyl), Furanyl und Pyridinyl substituiert sein können,

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Bevorzugt sind Verbindungen, worin R⁵ -C(=S)NR²¹R²² bedeutet.

Bevorzugt sind auch Verbindungen, worin R⁵ -(CHR⁶)ₙ-R²⁵ bedeutet.

Weiterhin bevorzugt sind Verbindungen, bei denen n 1 bedeutet.

Besonders bevorzugt sind Verbindungen, worin R²¹ für H und R²² für Benzyl, Phenyl, Pyridyl, Naphthyl oder Phenethyl, unsubstituiert oder einfach oder mehrfach substituiert mit Methyl, Ethyl, Isopropyl, Cl, F, Br, NO₂, Acetyl, CN, COOH, COOC₁₋₄-Alkyl, Methoxy, Ethoxy, N(CH₃)₂, N(C₂H₅)₂, 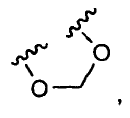 CF₃, SCH₃; oder für C₁₋₁₀-Alkyl, verzweigt oder unverzweigt, gesättigt oder ungesättigt, unsubstituiert oder substituiert mit OCH₃, COOCH₃ oder COOC₂H₅; C₃₋₆-Cycloalkyl, wobei die Cycloalkylgruppe über eine CH₂-Gruppe verknüpft sein kann; C₅₋₆-Heterocycloalkyl wobei die Heterocycloalkylgruppe über eine CH₂-Gruppe verknüpft sein kann; steht.

Ganz besonders bevorzugt sind Verbindungen worin R²¹ für H und R²² für Benzyl, Phenyl, 2-Methylphenyl, Phenethyl, 2-Isoropylphenyl, 2-Chlorphenyl, 4-Fluorbenzyl, 1-(4-Fluorphenyl)ethyl, 4-Chlorbenzyl, 4-Chlor-phenethyl, 4-Nitrophenyl, 4-Acetyl-phenyl, 3-Carboxyphenyl, 3-Methylbenzoat, 4-Ethylbenzoat, 2,6-Diethylphenyl, 3-Chlor-4-methyl-phenyl, 2-Methoxyethyl, 3-Methoxypropyl, Cyclopentyl, Cyclohexyl, 3-Pyridyl, 4-Dimethylaminophenyl, 4-Diethylaminophenyl, CH₂COOCH₃, CH(CH₃)COOC₂H₅, CH(CH₃)CH₂COOC₂H₅, Cyclohexylmethyl, 4-Ethoxyphenyl, 3,4-Dimethoxyphenyl, 1-Naphthyl, 3,4,5-Trimethoxyphenyl, 2,3,4,5,6-Pentafluorphenyl, Benzodioxol, 4-Fluorphenyl, Methyl, Ethyl, Propyl, Isopropyl, tert.-Butyl, Allyl, 2-Methylprop-2-enyl, 2-Nitrophenyl, 3-Trifluormethylphenyl, 2-Trifluormethylphenyl, 4-Trifluormethylphenyl, Cyclopropyl, 2-Methylsulfanylphenyl, 3-Methylsulfanylphenyl, 4-Methylsulfanylphenyl, 3,5-Dimethylphenyl, Ethylmorpholin, ((4-Propyl)-cyclohexyl)phenyl, 4-Brom-2-trifluormethyl-phenyl, n-Octyl, n-Nonanyl, Tetrahydrofurylmethyl, 2-Ethylphenyl, 4-Cyanophenyl, 3-Cyanophenyl, 2,6-Diisopropylphenyl, n-Pentyl, n-Hexyl, sec-Butyl, Propylmorpholin, 5-Chlor-2-methoxyphenyl, 4-Chlor-3-trifluormethylphenyl, 3-Chlorphenyl, 1-Phenylethyl, CH(CH₃)COOCH₃, 2-Methoxyphenyl, 3-Methoxyphenyl, 4-Methoxyphenyl, CH₂CH₂COOC₂H₅, 2-Methylbenzoat, 4-Methylbenzoat, 2-Ethylbenzoat, 2-Fluorphenyl, 2-Methoxy-5-chlorphenyl oder 2,4-Dimethoxyphenyl steht.

Weiterhin bevorzugt sind Verbindungen, worin R⁶ H bedeutet.

Ebenfalls bevorzugt sind Verbindungen, worin R⁶ CH₃ bedeutet.

Besonders bevorzugt sind auch Verbindungen, worin R²⁵ Phenyl, Anthracenyl, Pyridyl, Thienyl oder Furyl bedeutet, jeweils unsubstituiert oder einfach oder mehrfach substituiert mit CF₃, SCF₃, C₁₋₄-Alkyl, Cl, NO₂, O-Acetyl, OCH₃, F, O-Phenyl, OCF₃, Br, O-Benzyl, O-Allyl, Phenyl, I, CN oder OH, vorzugsweise Phenyl unsubstituiert oder substituiert mit den genannten Resten.

Ganz besonders bevorzugt sind Verbindungen, worin R²⁵ für 4-Trifluormethylphenyl, 4-SCF₃-Phenyl, 2-Methylphenyl, Phenyl, Anthracenyl, 4-Cl-Phenyl, 4-OCF₃-Phenyl, 4-n-Butylphenyl, 3(3-CF₃-Phenoxy)-phenyl, 4-OCHF₂-phenyl, 3,5-Dimethylphenyl, 3-Brom-4-methoxyphenyl, 4-Benzyloxy-3,5-dimethylphenyl, 3-Nitrophenyl, 3-Methoxy-4-(acetylmethyl)-phenyl, 2,4,5-Trimethoxyphenyl, 4-Ethylphenyl, 3,4-Dichlorphenyl, 2,3,5-Trifluorphenyl, 4-Phenoxyphenyl, 3-Chlor-4-fluorphenyl, 3-Benzyloxy-phenyl, 3-Brom-4,5-dimethoxyphenyl, 3-Fluor-2-methylphenyl, 2-Chlor-3-trifluormethylphenyl, 3-Chlor-2-fluor-5-trifluormethylphenyl, 2-Fluor-4-trifluormethylphenyl, 4-(Allyloxy)-phenyl, 2-(Benzyloxy)-4,5-dimethoxyphenyl, 2-Phenyl-phenyl, 2,3,4-Trifluorphenyl, 2-Fluor-5-trifluorphenyl, 4-Methoxy-3-methylphenyl, 2-Fluor-3-chlorphenyl, 3,4-Difluorphenyl, 2,6-Dichlorphenyl, 3-lodphenyl, 3-lod-4,5-dimethoxyphenyl, 2-Cyanophenyl, 4-Hydroxyphenyl, 3,4-Dimethylphenyl oder 3-OCF₃-Phenyl steht.

Am meisten bevorzugt sind erfindungsgemäße Verbindungen ausgewählt aus der Gruppe bestehend aus
Benzo[d]isoxazol-3-yl-[1-(4-trifluormethyl-phenyl)-ethyl]-amin
Benzo[d]isoxazol-3-yl-[1-(4-trifluormethylsulfanyl-phenyl)-ethyl]-amin
1-Benzo[d]isoxazol-3-yl-3-benzyl-thioharnstoff
1-Benzo[d]isoxazol-3-yl-3-phenyl-thiohamstoff
1-Benzo[d]isoxazol-3-yl-3-o-tolyl-thioharnstoff
1-Benzo[d]isoxazol-3-yl-3-phenethyl-thioharnstoff
1-Benzo[d]isoxazol-3-yl-3-(2-isopropyl-phenyl)-thioharnstoff
1-Benzo[d]isoxazol-3-yl-3-(2-chlor-phenyl)-thioharnstoff
1-Benzo[d]isoxazol-3-yl-3-(4-fluor-benzyl)-thioharnstoff
1-Benzo[d]isoxazol-3-yl-3-[1-(4-fluor-phenyl)-ethyl]-thioharnstoff
1-Benzo[d]isoxazol-3-yl-3-(4-chlor-benzyl)-thioharnstoff
1-Benzo[d]isoxazol-3-yl-3-[2-(4-chlor-phenyl)-ethyl]-thioharnstoff
1-Benzo[d]isoxazol-3-yl-3-(4-nitro-phenyl)-thioharnstoff
1-(4-Acetyl-phenyl)-3-benzo[d]isoxazol-3-yl-thioharnstoff
3-(3-Benzo[d]isoxazol-3-yl-thioureido)-benzoesäure
3-(3-Benzo[d]isoxazol-3-yl-thioureido)-benzoesäuremethylester
4-(3-Benzo[d]isoxazol-3-yl-thioureido)-benzoesäureethylester
1-Benzo[d]isoxazol-3-yl-3-(2,6-diethyl-phenyl)-thioharnstoff
1-Benzo[d]isoxazol-3-yl-3-(3-chlor-4-methyl-phenyl)-thioharnstoff
1-Benzo[d]isoxazol-3-yl-3-(2-methoxy-ethyl)-thioharnstoff
1-Benzo[d]isoxazol-3-yl-3-(3-methoxy-propyl)-thioharnstoff
1-Benzo[d]isoxazol-3-yl-3-cyclopentyl-thioharnstoff
1-Benzo[d]isoxazol-3-yl-3-cyclohexyl-thioharnstoff
1-Benzo[d]isoxazol-3-yl-3-pyridin-3-yl-thiohamstoff
1-Benzo[d]isoxazol-3-yl-3-(4-dimethylamino-phenyl)-thioharnstoff
1-Benzo[d]isoxazol-3-yl-3-(4-diethylamino-phenyl)-thioharnstoff
(3-Benzo[d]isoxazol-3-yl-thioureido)-essigsäuremethylester
2-(3-Benzo[d]isoxazol-3-yl-thioureido)-propionsäureethylester
3-(3-Benzo[d]isoxazol-3-yl-thioureido)-buttersäureethylester
1-Benzo[d]isoxazol-3-yl-3-cyclohexylmethyl-thioharnstoff
1-Benzo[d]isoxazol-3-yl-3-(4-ethoxy-phenyl)-thioharnstoff
1-Benzo[d]isoxazol-3-yl-3-(3,4-dimethoxy-phenyl)-thioharnstoff
1-Benzo[d]isoxazol-3-yl-3-(3,4,5-trimethoxy-phenyl)-thioharnstoff
1-Benzo[d]isoxazol-3-yl-3-pentafluorphenyl-thioharnstoff
1-Benzo[d]isoxazol-3-yl-3-naphthalen-1-yl-thioharnstoff
1-Benzo[1,3]dioxol-5-ylmethyl-3-benzo[d]isoxazol-3-yl-thiohamstoff
1-Benzo[d]isoxazol-3-yl-3-(4-fluor-phenyl)-thioharnstoff
1-Benzo[d]isoxazol-3-yl-3-methyl-thioharnstoff
1-Benzo[d]isoxazol-3-yl-3-ethyl-thioharnstoff
1-Benzo[d]isoxazol-3-yl-3-propyl-thioharnstoff
1-Benzo[d]isoxazol-3-yl-3-isopropyl-thioharnstoff
1-Benzo[d]isoxazol-3-yl-3-tert-butyl-thioharnstoff
1-Allyl-3-benzo[d]isoxazol-3-yl-thioharnstoff
1-Benzo[d]isoxazol-3-yl-3-(2-methyl-allyl)-thioharnstoff
1-Benzo[d]isoxazol-3-yl-3-(2-nitro-phenyl)-thioharnstoff
1-Benzo[d]isoxazol-3-yl-3-(2-trifluormethyl-phenyl)-thioharnstoff
1-Benzo[d]isoxazol-3-yl-3-(3-trifluormethyl-phenyl)-thioharnstoff
1-Benzo[d]isoxazol-3-yl-3-(4-trifluormethyl-phenyl)-thioharnstoff
1-Benzo[d]isoxazol-3-yl-3-cyclopropyl-thioharnstoff 2-[3-(4-Fluor-benzo[d]isoxazol-3-yl)-thioureido]-propionsäuremethylester
1-(4-Chlor-benzo[d]isoxazol-3-yl)-3-o-tolyl-thioharnstoff
1-Benzyl-3-(4-chlor-benzo[d]isoxazol-3-yl)-thioharnstoff
1-(4-Chlor-benzo[d]isoxazol-3-yl)-3-(1-phenyl-ethyl)-thioharnstoff
1-(4-Dimethylamino-benzo[d]isoxazol-3-yl)-3-isobutyl-thioharnstoff
1-(4-Dimethylamino-benzo[d]isoxazol-3-yl)-3-p-tolyl-thioharnstoff
1-(3-Chlor-phenyl)-3-(4-dimethylamino-benzo[d]isoxazol-3-yl)-thioharnstoff
1-(4-Dimethylamino-benzo[d]isoxazol-3-yl)-3-(3-methoxy-phenyl)-thioharnstoff
1-(4-Dimethylamino-benzo[d]isoxazol-3-yl)-3-(2-methylsulfanyl-phenyl)-thioharnstoff
1-(4-Dimethylamino-benzo[d]isoxazol-3-yl)-3-(3-methylsulfanyl-phenyl)-thioharnstoff
1-(4-Dimethylamino-benzo[d]isoxazol-3-yl)-3-(4-methylsulfanyl-phenyl)-thioharnstoff
1-(4-Dimethylamino-benzo[d]isoxazol-3-yl)-3-(2-methoxy-phenyl)-thioharnstoff
1-(4-Dimethylamino-benzo[d]isoxazol-3-yl)-3-(4-methoxy-phenyl)-thioharnstoff
1-(4-Dimethylamino-benzo[d]isoxazol-3-yl)-3-(3,5-dimethyl-phenyl)-thioharnstoff
1-Benzyl-3-(4-dimethylamino-benzo[d]isoxazol-3-yl)-thioharnstoff
1-(4-Dimethylamino-benzo[d]isoxazol-3-yl)-3-(3-methoxy-propyl)-thioharnstoff
3-[3-(4-Dimethylamino-benzo[d]isoxazol-3-yl)-thioureido]-propionsäurethylester
2-[3-(4-Dimethylamino-benzo[d]isoxazol-3-yl)-thioureido]-propionsäurethylester
3-[3-(4-Dimethylamino-benzo[d]isoxazol-3-yl)-thioureido]-buttersäureethylester
3-[3-(4-Dimethylamino-benzo[d]isoxazol-3-yl)-thioureido]-benzoesäure
1-(4-Dimethylamino-benzo[d]isoxazol-3-yl)-3-(4-ethoxy-phenyl)-thioharnstoff
2-[3-(4-Dimethylamino-benzo[d]isoxazol-3-yl)-thioureido]-benzoesäuremethylester
3-[3-(4-Dimethylamino-benzo[d]isoxazol-3-yl)-thioureido]-benzoesäuremethylester
4-[3-(4-Dimethylamino-benzo[d]isoxazol-3-yl)-thioureido]-benzoesäuremethylester
2-[3-(4-Dimethylamino-benzo[d]isoxazol-3-yl)-thioureido]-benzoesäureethylester
4-[3-(4-Dimethylamino-benzo[d]isoxazol-3-yl)-thioureido]-benzoesäureethylester
1-(4-Acetyl-phenyl)-3-(4-dimethylamino-benzo[d]isoxazol-3-yl)-thioharnstoff
1-(2-Chlor-phenyl)-3-(4-methoxy-benzo[d]isoxazol-3-yl)-thioharnstoff
1-(4-Diethylamino-phenyl)-3-(4-methoxy-benzo[d]isoxazol-3-yl)-thioharnstoff
1-(4-Methoxy-benzo[d]isoxazol-3-yl)-3-(2-morpholin-4-yl-ethyl)-thioharnstoff
2-{3-[4-(2,2,2-trifluor-ethoxy)-benzo[d]isoxazol-3-yl]-thioureido}-propionsäuremethylester
1-Benzo[1,3]dioxol-5-ylmethyl-3-[4-(2,2,2-trifluor-ethoxy)-benzo[d]isoxazol-3-yl]-thioharnstoff
1-[4-(4-Propyl-cyclohexyl)-phenyl]-3-[4-(2,2,2-trifluor-ethoxy)-benzo[d]isoxazol-3-yl]-thioharnstoff
1-(4-Brom-2-trifluormethyl-phenyl)-3-[4-(2,2,2-trifluor-ethoxy)-benzo[d]isoxazol-3-yl]-thioharnstoff
1-(4-Methoxy-phenyl)-3-[4-(2,2,2-trifluor-ethoxy)-benzo[d]isoxazol-3-yl]-thioharnstoff
3-{3-[4-(2,2,2-trifluor-ethoxy)-benzo[d]isoxazol-3-yl]-thioureido}-propionsäureethylester
1-[4-(4-Methyl-benzyloxy)-benzo[d]isoxazol-3-yl]-3-octyl-thioharnstoff
1-[4-(4-Methyl-benzyloxy)-benzo[d]isoxazol-3-yl]-3-nonyl-thioharnstoff
1-Cyclopropyl-3-[4-(4-methyl-benzyloxy)-benzo[d]isoxazol-3-yl]-thiohamstoff
1-Cyclopentyl-3-[4-(4-methyl-benzyloxy)-benzo[d]isoxazol-3-yl]-thioharnstoff
1-Cyclohexyl-3-[4-(4-methyl-benzyloxy)-benzo[d]isoxazol-3-yl]-thioharnstoff
1-Cyclohexylmethyl-3-[4-(4-methyl-benzyloxy)-benzo[d]isoxazol-3-yl]-thioharnstoff
1-(4-Dimethylamino-phenyl)-3-[4-(2,2,2-trifluor-ethoxy)-benzo[d]isoxazol-3-yl]-thioharnstoff
1-Allyl-3-(5-methyl-benzo[d]isoxazol-3-yl)-thioharnstoff
[3-(5-Methyl-benzo[d]isoxazol-3-yl)-thioureido]-essigsäuremethylester
1-(2-Isopropyl-phenyl)-3-(5-methyl-benzo[d]isoxazol-3-yl)-thioharnstoff
1-(5-Methyl-benzo[d]isoxazol-3-yl)-3-(4-trifluormethyl-phenyl)-thioharnstoff
2-[3-(5-Fluor-benzo[d]isoxazol-3-yl)-thioureido]-propionsäuremethylester
1-(5-Fluor-benzo[d]isoxazol-3-yl)-3-(tetrahydro-furan-2-ylmethyl)-thioharnstoff
1-(5-Brom-benzo[d]isoxazol-3-yl)-3-(2-Fluor-phenyl)-thioharnstoff
1-(5-Brom-benzo[d]isoxazol-3-yl)-3-(2-ethyl-phenyl)-thioharnstoff
1-(6-Chlor-benzo[d]isoxazol-3-yl)-3-(4-Fluor-phenyl)-thioharnstoff
1-(6-Chlor-benzo[d]isoxazol-3-yl)-3-(2-Fluor-phenyl)-thioharnstoff
1-(6-Chlor-benzo[d]isoxazol-3-yl)-3-cyclopentyl-thioharnstoff
1-(6-Chlor-benzo[d]isoxazol-3-yl)-3-(4-cyano-phenyl)-thioharnstoff
3-[3-(6-Chlor-benzo[d]isoxazol-3-yl)-thioureido]-benzoesäure
1-(6-Chlor-benzo[d]isoxazol-3-yl)-3-(4-methoxy-phenyl)-thioharnstoff
1-(6-Chlor-benzo[d]isoxazol-3-yl)-3-(3-methoxy-phenyl)-thioharnstoff
1-(6-Brom-benzo[d]isoxazol-3-yl)-3-(3,4-dimethoxy-phenyl)-thioharnstoff
1-(6-Brom-benzo[d]isoxazol-3-yl)-3-naphthalen-1-yl-thioharnstoff
1-Benzo[1,3]dioxol-5-ylmethyl-3-(6-brom-benzo[d]isoxazol-3-yl)-thioharnstoff
1-(6-Fluor-benzo[d]isoxazol-3-yl)-3-(2-methylsulfanyl-phenyl)-thioharnstoff
1-(3-Cyano-phenyl)-3-(6-fluor-benzo[d]isoxazol-3-yl)-thioharnstoff
1-(2-Chlor-6-methyl-phenyl)-3-(6-fluor-benzo[d]isoxazol-3-yl)-thioharnstoff
1-(2,6-Diisopropyl-phenyl)-3-(6-fluor-benzo[d]isoxazol-3-yl)-thioharnstoff
1-(7-Fluor-benzo[d]isoxazol-3-yl)-3-methyl-thioharnstoff
1-Ethyl-3-(7-fluor-benzo[d]isoxazol-3-yl)-thioharnstoff
1-(7-Fluor-benzo[d]isoxazol-3-yl)-3-propyl-thioharnstoff
1-(7-Fluor-benzo[d]isoxazol-3-yl)-3-pentyl-thioharnstoff
1-(7-Fluor-benzo[d]isoxazol-3-yl)-3-hexyl-thioharnstoff
1-(7-Fluor-benzo[d]isoxazol-3-yl)-3-octyl-thioharnstoff
1-(7-Fluor-benzo[d]isoxazol-3-yl)-3-nonyl-thioharnstoff
1-(7-Fluor-benzo[d]isoxazol-3-yl)-3-isobutyl-thioharnstoff
1-Allyl-3-(7-fluor-benzo[d]isoxazol-3-yl)-thioharnstoff
1-(7-Fluor-benzo[d]isoxazol-3-yl)-3-p-tolyl-thiohamstoff
1-(5-Brom-benzo[d]isoxazol-3-yl)-3-(4-dimethylamino-phenyl)-thioharnstoff
1-(7-Fluor-benzo[d]isoxazol-3-yl)-3-(2-morpholin-4-yl-ethyl)-thioharnstoff
1-(7-Fluor-benzo[d]isoxazol-3-yl)-3-(3-morpholin-4-yl-propyl)-thioharnstoff
1-(4-Methoxy-benzo[d]isoxazol-3-yl)-3-(1-phenyl-ethyl)-thioharnstoff
1-(4-Chlor-benzyl)-3-(4-methoxy-benzo[d]isoxazol-3-yl)-thicharnstoff
1-(4-Methoxy-benzo[d]isoxazol-3-yl)-3-(2-methoxy-phenyl)-thioharnstoff
1-(5-Brom-benzo[d]isoxazol-3-yl)-3-(4-dimethylamino-phenyl)-thioharnstoff
1-(5-Chlor-2-methoxy-phenyl)-3-(4-methoxy-benzo[d]isoxazol-3-yl)-thioharnstoff
1-(4-Chlor-3-trifluormethyl-phenyl)-3-(4-methoxy-benzo[d]isoxazol-3-yl)-thioharnstoff
1-(2,4-Dimethoxy-phenyl)-3-(4-methoxy-benzo[d]isoxazol-3-yl)-thioharnstoff
1-[4-(2,2,2-Trifluor-ethoxy)-benzo[d]isoxazol-3-yl]-3-(3,4,5-trimethoxy-phenyl)-thioharnstoff
Benzo[d]isoxazol-3-yl-(3-methyl-butyl)-amin
(5-Fluor-benzo[d]isoxazol-3-yl)-(2-methyl-benzyl)-amin
N4,N4-Dimethyl-N3-(3-phenyl-propyl)-benzo[d]isoxazol-3,4-diamin
N3-Butyl-N4,N4-dimethyl-benzo[d]isoxazol-3,4-diamin
Anthracen-9-ylmethyl-(4-methoxy-benzo[d]isoxazol-3-yl)-amin
(4-Chlor-benzyl)-(4-methoxy-benzo[d]isoxazol-3-yl)-amin
(6-Fluor-benzo[d]isoxazol-3-yl)-(3-nitro-benzyl)-amin
Essigsäure-4-[(6-chlor-benzo[d]isoxazol-3-ylamino)-methyl]-2-methoxyphenylester
Essigsäure-4-[(6-brom-benzo[d]isoxazol-3-ylamino)-methyl]-2-methoxyphenylester
Benzo[d]isoxazol-3-yl-(3,4-dichlor-benzyl)-amin
Benzo[d]isoxazol-3-yl-(2,4,5-trimethoxy-benzyl)-amin
Benzo[d]isoxazol-3-yl-(4-ethyl-benzyl)-amin
(6-Chlor-benzo[d]isoxazol-3-yl)-(3,4-dichlor-benzyl)-amin
Benzo[d]isoxazol-3-yl-(2,3,5-trifluor-benzyl)-amin
(6-Chlor-benzo[d]isoxazol-3-yl)-(4-phenoxy-benzyl)-amin
(3-Chlor-4-Fluor-benzyl)-(7-fluor-benzo[d]isoxazol-3-yl)-amin
Benzo[d]isoxazol-3-yl-(4-trifluormethyl-benzyl)-amin
(7-Fluor-benzo[d]isoxazol-3-yl)-(2-methyl-pentyl)-amin
N4,N4-Dimethyl-N3-(2,3,4-trifluor-benzyl)-benzo[d]isoxazol-3,4-diamin
N3-(2-Fluor-5-trifluormethyl-benzyl)-N4,N4-dimethyl-benzo[d]isoxazol-3,4-diamin
N3-(4-Methoxy-3-methyl-benzyl)-benzo[d]isoxazol-3,4-diamin
N3-(4-Methoxy-3-methyl-benzyl)-benzo[d]isoxazol-3,4-diamin
Benzo[d]isoxazol-3-yl-(4-trifluormethoxy-benzyl)-amin
(5-Fluor-benzo[d]isoxazol-3-yl)-(4-trifluormethoxy-benzyl)-amin
Benzo[d]isoxazol-3-yl-(4-trifluormethylsulfanyl-benzyl)-amin
(4-Butyl-benzyl)-(6-chlor-benzo[d]isoxazol-3-yl)-amin
(5-Fluor-benzo[d]isoxazol-3-yl)-(4-trifluormethylsulfanyl-benzyl)-amin
Benzo[d]isoxazol-3-yl-(2-fluor-4-trifluormethyl-benzyl)-amin
(7-Fluor-benzo[d]isoxazol-3-yl)-(4-trifluormethoxy-benzyl)-amin
(7-Fluor-benzo[d]isoxazol-3-yl)-[3-(3-trifluormethyl-phenoxy)-benzyl]-amin
(4-Difluormethoxy-benzyl)-(4-methoxy-benzo[d]isoxazol-3-yl)-amin
(3,5-Dimethyl-benzyl)-(7-fluor-benzo[d]isoxazol-3-yl)-amin
(3-Brom-4-methoxy-benzyl)-(6-fluor-benzo[d]isoxazol-3-yl)-amin
(3,5-Dimethyl-benzyl)-(6-fluor-benzo[d]isoxazol-3-yl)-amin
(4-Benzyloxy-3,5-dimethyl-benzyl)-(6-fluor-benzo[d]isoxazol-3-yl)-amin
(4-Butyl-benzyl)-(6-fluor-benzo[d]isoxazol-3-yl)-amin
(6-Fluor-benzo[d]isoxazol-3-yl)-(4-trifluormethylsulfanyl-benzyl)-amin
(3-Benzyloxy-benzyl)-(6-fluor-benzo[d]isoxazol-3-yl)-amin
N3-(3,5-Dimethyl-benzyl)-benzo[d]isoxazol-3,4-diamin
N3-(4-Butyl-benzyl)-benzo[d]isoxazol-3,4-diamin
(5-Brom-benzo[d]isoxazol-3-yl)-(4-trifluormethylsulfanyl-benzyl)-amin
(3-Brom-4,5-dimethoxy-benzyl)-(7-fluor-benzo[d]isoxazol-3-yl)-amin
(7-Fluor-benzo[d]isoxazol-3-yl)-(2-fluor-4-trifluormethyl-benzyl)-amin
N3-(3-Fluor-2-methyl-benzyl)-N4,N4-dimethyl-benzo[d]isoxazol-3,4-diamin
N3-(2-Chlor-3-trifluormethyl-benzyl)-N4,N4-dimethyl-benzo[d]isoxazol-3,4-diamin
N3-(3-Chlor-2-fluor-5-trifluormethyl-benzyl)-N4,N4-dimethyl-benzo[d]isoxazol-3,4-diamin
(6-Fluor-benzo[d]isoxazol-3-yl)-(2-fluor-4-trifluormethyl-benzyl)-amin
(4-Allyloxy-benzyl)-(6-fluor-benzo[d]isoxazol-3-yl)-amin
Benzo[d]isoxazol-3-yl-(2-benzyloxy-4,5-dimethoxy-benzyl)-amin
(2-Benzyloxy-4,5-dimethoxy-benzyl)-(6-chlor-benzo[d]isoxazol-3-yl)-amin
N3-(2-Benzyloxy-4,5-dimethoxy-benzyl)-N4,N4-dimethyl-benzo[d]isoxazol-3,4-diamin
N3-Biphenyl-2-ylmethyl-N4,N4-dimethyl-benzo[d]isoxazol-3,4-diamin
(6-Fluor-benzo[d]isoxazol-3-yl)-(3-iodo-benzyl)-amin
(2-Benzyloxy-4,5-dimethoxy-benzyl)-(4-methoxy-benzo[d]isoxazol-3-yl)-amin
(4-Fluor-benzo[d]isoxazol-3-yl)-(3-iodo-4,5-dimethoxy-benzyl)-amin
2-[(5-Methyl-benzo[d]isoxazol-3-ylamino)-methyl]-benzonitril
Butyl-[4-(2,2,2-trifluor-ethoxy)-benzo[d]isoxazol-3-yl]-amin
(3-Brom-4,5-dimethoxy-benzyl)-(5-methyl-benzo[d]isoxazol-3-yl)-amin
4-[(4-Chlor-benzo[d]isoxazol-3-ylamino)-methyl]-phenol
(6-Chlor-benzo[d]isoxazol-3-yl)-(3,4-dimethyl-benzyl)-amin
(4-Chlor-benzo[d]isoxazol-3-yl)-(3-chlor-2-fluor-benzyl)-amin
(3,4-Difluor-benzyl)-(5-fluor-benzo[d]isoxazol-3-yl)-amin
(6-Brom-benzo[d]isoxazol-3-yl)-(2,6-dichlor-benzyl)-amin
(7-Fluor-benzo[d]isoxazol-3-yl)-(3-trifluormethoxy-benzyl)-amin
sowie jeweils deren entsprechende Salze, insbesondere deren Hydrochlorid-Additionssalze, und ggf. jeweils deren entsprechende Solvate.

Ein weiterer Gegenstand der vorliegenden Erfindung ist ein Verfahren zur Herstellung der erfindungsgemäßen substituierten Benzo[d]isoxazol-3-yl-amin-Verbindungen, gemäß dem eine ggf. substituierte 2-Fluor-benzonitril-verbindung der allgemeinen Formel II, 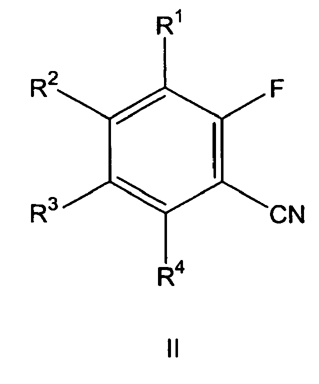 worin die Reste R¹, R², R³ und R⁴ die vorstehend angegebene Bedeutung haben, in einem Reaktionsmedium, vorzugsweise ausgewählt aus der Gruppe bestehend aus Diethylether, Tetrahydrofuran, Acetonitril, Dimethylsulfoxid, Dimethylformamid und Dichlormethan, in Gegenwart einer Base, vorzugsweise in Gegenwart wenigstens eines Alkalimetallalkoholatsalzes, besonders bevorzugt in Gegenwart eines Alkalimetallalkoholatsalzes ausgewählt aus der Gruppe bestehend aus Kaliummethanolat, Natriummethanolat, Kalium-tert-butylat und Natrium-tert-butylat mit Acethydroxamsäure der Formel III 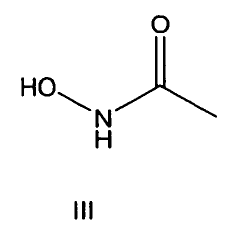 vorzugsweise bei Temperaturen von 20 °C bis 100 °C zu einer Verbindung der allgemeinen Formel I, 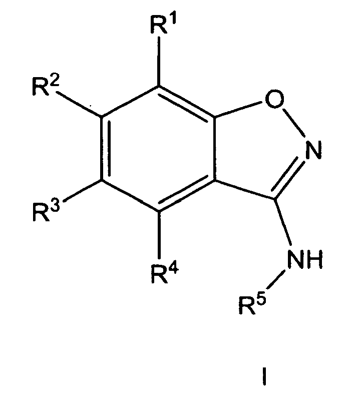 worin die Reste R¹-R⁴ die vorstehend genannte Bedeutung haben sowie der Rest R⁵ für einen Wasserstoff-Rest steht, umgesetzt wird, diese ggf. gereinigt und/oder ggf. isoliert wird,
diese ggf. anschließend in einem Reaktionsmedium, vorzugsweise ausgewählt aus der Gruppe bestehend aus Acetonitril, Toluol, Dimethylformamid, Benzol, Ethanol, Methanol und entsprechenden Mischungen, mit wenigstens einem Isothiocyanat der allgemeinen Formel S=C=N-R²², worin R²² die vorstehend angegebene Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹, R², R³, R⁴ die vorstehend genannte Bedeutung haben und R⁵ für -C(=S)-NR²¹R²² steht, wobei R²² die vorstehend genannte Bedeutung hat und R²¹ für einen Wasserstoff-Rest steht, und diese ggf. gereinigt und/oder ggf. isoliert wird,
und ggf. wenigstens eine Verbindung der allgemeinen Formel I, worin R¹, R², R³, R⁴ die vorstehend genannte Bedeutung haben, R⁵ für für -C(=S)-NR²¹R²² steht, wobei R²² die vorstehend genannte Bedeutung hat und R²¹ für einen Wasserstoff-Rest steht, in einem Reaktionsmedium, vorzugsweise ausgewählt aus der Gruppe bestehend aus Acetonitril, Toluol, Dimethylformamid, Benzol, Ethanol, Methanol und entsprechenden Mischungen, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R²¹, worin LG für eine Abgangsgruppe, bevorzugt für ein Halogen-Atom, besonders bevorzugt für ein Chloratom steht, und R²¹ die vorstehend genannte Bedeutung mit Ausnahme von Wasserstoff hat, zu wenigstens einer Verbindung der allgemeinen Formel I umgesetzt wird, und diese ggf. gereinigt und/oder ggf. isoliert wird,
oder
ggf. wenigstens eine Verbindung der allgemeinen Formel I, worin R¹, R², R³, R⁴ die vorstehend genannte Bedeutung haben und R⁵ für H steht, in einem Reaktionsmedium, vorzugsweise ausgewählt aus der Gruppe bestehend aus Acetonitril, Toluol, Dimethylformamid, Benzol, Ethanol, Methanol, DCM, Trifluoressigsäure und entsprechenden Mischungen, in Gegenwart wenigstens einer Base, bevorzugt in Gegenwart wenigstens eines Metallhydridsalzes oder eines Metallalkoholatsalzes oder Triethylsilan, besonders bevorzugt in Gegenwart des Triethylsilans, eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel R³⁰C(=O)H oder R⁶C(O)R²⁵, wobei R⁶ und R²⁵ die vorstehend genannte Bedeutung haben und R³⁰ für einen linearen oder verzweigten, gesättigten oder ungesättigten, unsubstituierten oder wenigstens einfach substituierten aliphatischen Rest; für einen unsubstituierten oder wenigstens einfach substituierten Aryl- oder Heteroaryl-Rest steht, der mit einem mono- oder polyzyklischen Ringsystem kondensiert sein kann; oder für einen unsubstituierten oder wenigstens einfach substituierten Aryl- oder Heteroaryl-Rest steht, der mit einem mono- oder polyzyklischen Ringsystem kondensiert sein kann und über eine lineare oder verzweigte Alkylen-Gruppe gebunden ist, zu wenigstens einer Verbindung der allgemeinen Formel I umgesetzt und diese ggf. gereinigt und/oder ggf. isoliert wird.

Wenn der Rest R³⁰ in einer bestimmten Verbindung definiert ist, bedeutet dies, dass für diesen Fall in Strukturen der allgemeinen Formel I R⁵ für CH₂R³⁰ steht.

Die in den vorstehend beschriebenen Umsetzungen zum Einsatz kommenden Chemikalien und Reaktionskomponenten sind käuflich erhältlich oder können jeweils nach üblichen, dem Fachmann bekannten Methoden hergestellt werden.

Die vorstehend beschriebenen Umsetzungen können ferner jeweils unter üblichen, dem Fachmann geläufigen Bedingungen, beispielsweise in Hinblick auf Druck, Temperatur, Schutzgasatmosphäre oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie sowie HPLC.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre oder Argonatmosphäre, durchgeführt werden.

Die erfindungsgemäßen substituierten Benzo[d]isoxazol-3-yl-amin-Verbindungen können sowohl in Form ihrer freien Basen, ihrer freien Säuren wie auch jeweils in Form entsprechender Salze, insbesondere physiologisch verträglicher Salze, isoliert werden.

Die freien Basen der jeweiligen erfindungsgemäßen substituierten Benzo[d]isoxazol-3-yl-amin-Verbindungen können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Maleinsäure, Äpfelsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden.

Die freien Basen der jeweiligen erfindungsgemäßen substituierten Benzo[d]isoxazol-3-yl-amin-Verbindungen können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der erfindungsgemäßen substituierten Benzo[d]isoxazol-3-yl-amin-Verbindungen durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten Benzo[d]isoxazol-3-yl-amin-Verbindungen können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Benzo[d]isoxazol-3-yl-amin-Verbindungen nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Benzo[d]isoxazol-3-yl-amin-Verbindungen sowie jeweils die entsprechenden Säuren, Basen, Salze und Solvate eignen sich als pharmazeutische Wirkstoffe in Arzneimitteln.

Ein weiterer Gegenstand der vorliegenden Erfindung ist daher ein Arzneimittel enthaltend wenigstens eine erfindungsgemäße substituierte Benzo[d]isoxazol-3-yl-amin-Verbindung sowie ggf. einen oder mehrere pharmazeutisch verträgliche Hilfsstoffe.

Diese erfindungsgemäßen Arzneimittel eignen sich zur Beeinflussung von KCNQ2/3-Kanälen und üben insbesondere eine agonistische Wirkung aus.

Bevorzugt eignen sich die erfindungsgemäßen Arzneimittel zur Behandlung von Störungen oder Krankheiten, die zumindest teilweise durch KCNQ2/3-Kanäle vermittelt werden.

Bevorzugt eignet sich das erfindungsgemäße Arzneimittel zur Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz, Migräne; Epilepsie, Angstzuständen und Harninkontinenz. Besonders bevorzugt eignen sich die erfindungsgemäßen Arzneimittel zur Behandlung von Schmerz, ganz besonders bevorzugt von chronischem Schmerz, neuropathischem Schmerz, inflammatorischem Schmerz und muskulärem Schmerz.

Ein weiterer Gegenstand der vorliegenden Erfindung ist die Verwendung wenigstens einer erfindungsgemäßen substituierten Benzo[d]isoxazol-3-yl-amin-Verbindung sowie ggf. eines oder mehrerer pharmazeutisch verträglicher Hilfsstoffe zur Herstellung eines Arzneimittels zur Behandlung von Störungen oder Krankheiten, die zumindest teilweise durch KCNQ2/3-Kanäle vermittelt werden.

Bevorzugt ist die Verwendung wenigstens einer erfindungsgemäßen substituierten Benzo[d]isoxazol-3-yl-amin-Verbindung sowie ggf. eines oder mehrerer pharmazeutisch verträglicher Hilfsstoffe zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Migräne; Epilepsie, Angstzuständen und Harninkontinenz.

Besonders bevorzugt ist die Verwendung wenigstens einer erfindungsgemäßen substituierten Benzo[d]isoxazol-3-yl-amin-Verbindung sowie ggf. eines oder mehrerer pharmazeutisch verträglicher Hilfsstoffe zur Herstellung eines Arzneimittels zur Behandlung von Schmerz, ganz besonders bevorzugt von chronischem Schmerz, neuropathischem Schmerz, inflammatorischem Schmerz und muskulärem Schmerz.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden. Neben wenigstens einer erfindungsgemäßen substituierten Benzo[d]isoxazol-3-yl-amin-Verbindung enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten Benzo[d]isoxazol-3-yl-amin-Verbindungen können in einem Depot, in gelöster Form oder in einem Pflaster, gegebenenfalls unter Zusatz von die Hautpenetration fördernden Mitteln, als geeignete perkutane Applikationszubereitungen vorliegen. Oral oder perkutan anwendbare Zubereitungsformen können die jeweilige erfindungsgemäße substituierte Benzo[d]isoxazol-3-yl-amin-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remingtons Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind.

Die an den Patienten zu verabreichende Menge der jeweiligen erfindungsgemäßen substituierten Benzo[d]isoxazol-3-yl-amin-Verbindung kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

Im Folgenden wird die Erfindung mit Hilfe einiger Beispiele erläutert. Diese Erläuterungen sind als beispielhaft zu verstehen.

### Beispiele:

Die Ausbeuten der hergestellten Verbindungen sind nicht optimiert.

Alle Temperaturen sind unkorrigiert.

### Abkürzungen

| | |
|---|---|
| aq. | wäßrig |
| APCI | atmospheric pressure chemical ionisation |
| Äquiv. | Stoffmengenäquivalente |
| DCM | Dichlormethan |
| DMF | Dimethylformamid |
| DMSO | Dimethylsulfoxid |
| EtOAc | Ethylacetat |
| ges. | gesättigt |
| h | Stunden |
| min | Minuten |
| NMR | Kernresonanzspektroskopie |
| RT | Raumtemperatur |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch HPLC-MS oder NMR. In den meisten Fällen wurde eine Reinheit von >80% erzielt.

### Allgemeine Vorschrift zur Herstellung des Benzisoxazol-Grundgerüstes:

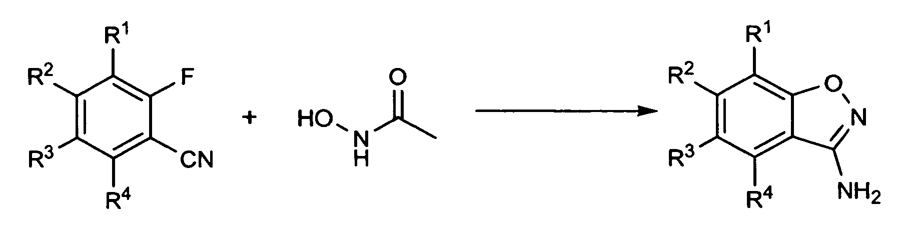

Das Grundgerüst der erfindungsgemäßen Benzisoxazole wurde analog der Vorschrift von Palermo (M. G. Palermo, Tetrahedron Lett. 1996; 37; 17; 2885-2886) hergestellt. Die entsprechende Beschreibung wird hiermit als Referenz eingeführt und gilt somit als Teil der vorliegenden Offenbarung. Abweichend von der genannten Vorschrift erfolgt die Aufreinigung der Benzisoxazol-Verbindungen zum Teil durch Fällen des entsprechenden HCl-Salzes.

### Durchführung:

In einem Dreihalskolben wurde Acethydroxamsäure (1,1 Äquiv.) in DMF (1,45 ml / mmol Acethydroxamsäure) suspendiert. Unter Inertgas wurde Kalium-*t*-Butylat (1,1 Äquiv.) zugegeben. Die Mischung wurde 30 min. bei Raumtemperatur gerührt und anschließend mit dem ggf. substituierten 2-Fluor-benzonitril (1 Äquiv.) versetzt. Der Reaktionsansatz wurde auf 50°C erwärmt und 1 h bei dieser Temperatur gerührt. Nach dem Abkühlen wurde die Reaktionsmischung auf eine Mischung (1,8 ml / mmol Acethydroxamsäure) aus gleichen Volumenanteilen gesättigter NaCl-Lösung und Essigsäureethylester gegeben und 30 min gut gerührt. Die Phasen wurden getrennt und die wässrige Phase 3mal mit Essigester (je 0,8 ml/mmol Acethydroxamsäure) extrahiert. Die organischen Phasen wurden vereinigt und 3mal mit gesättigter NaCl-Lösung (je 0,8 ml / mmol Acethydroxamsäure) gewaschen und anschließend über Magnesiumsulfat getrocknet. Das Magnesiumsulfat wurde abfiltriert und das Filtrat zunächst am Rotationsverdampfer und anschließend an der Ölpumpe aufkonzentriert.

Zur weiteren Aufreinigung war es in einigen Fällen vorteilhaft das erhaltene Hydrochlorid zu fällen.

Hierzu wurde der Rückstand in Methylethylketon (8,7 ml/g Rückstand) gelöst. Nach Zugabe von Wasser (0,1 ml/g Rückstand) wurde unter langsamen Rühren und EisWasser- Kühlung Trimethylchlorsilan (0,7 ml/g Rückstand) zugetropft.
Der Kolben wurde über Nacht in den Kühlschrank gestellt, der entstandene Niederschlag abfiltriert und im Exikkator mit Hilfe von Phosphorpentoxid als Trockenmittel getrocknet.

Nach dieser Vorschrift wurden die folgenden Zwischenprodukte hergestellt:

| | | | |
|---|---|---|---|
| A | 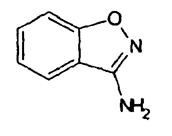 | I | 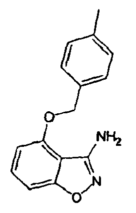 |
| B | 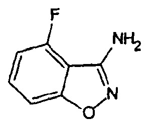 | J | 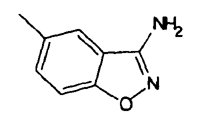 |
| C | 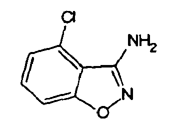 | K | 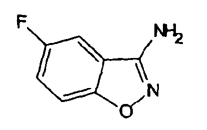 |
| D | 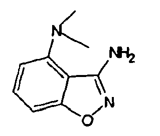 | L | 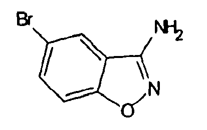 |
| E | 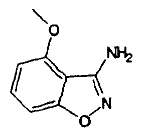 | M | 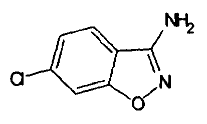 |
| F | 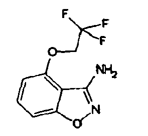 | N | 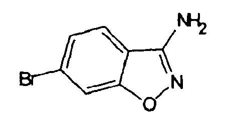 |
| G | 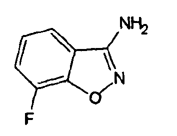 | O | 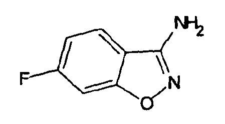 |
| H | 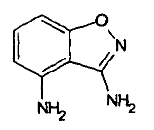 | P | 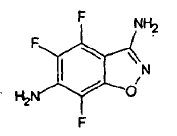 |

**Allgemeine Vorschrift zur reduktiven Aminierung des Amino-substituierten Benzisoxazol-Grundgerüstes zur Darstellung von** α**-alkyl-substituierten Benzisoxazolaminen:**

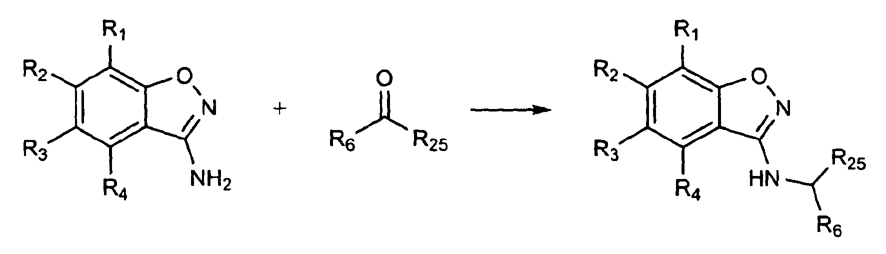

### Durchführung

Das jeweilige Benzisoxazol (4,55 mmol, 1 Äquiv.) wurde in DCM (11 ml) gelöst, mit dem entsprechenden Alkylphenylketon (4,55 mmol, 1 Äquiv.) versetzt und 1 h unter Inertgas bei Raumtemperatur ge rührt. Anschließend wurden unter inerten Bedingungen Triethylsilan (4,5 mmol, 1 Äquiv.) und Trifluoressigsäure (13,65 mmol, 3 Äquiv.) zugetropft und der Reaktionsansatz 4 - 12 h unter Rückfluss gerührt. Nach dem Abkühlen wurde der Ansatz mit ges. NaHCO₃-Lsg. auf pH 8 - 9 gestellt und die wässrige Phase 4mal mit DCM extrahiert. Die vereinigten organischen Phasen wurden über MgSO4 getrocknet und eingeengt. Das erhaltene Rohprodukt wurde durch Flash-Chromatographie (Diethylether/Hexan) aufgereinigt.

Nach dieser Vorschrift wurden die folgenden Alkylphenylketone umgesetzt:

| Nr. | Alkylphenylketon | Nr. | Alkylphenylketon |
|---|---|---|---|
| Z1 | 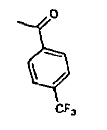 | Z2 | 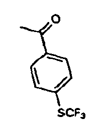 |

Die folgenden Beispiele wurden nach der vorstehenden Vorschrift synthetisiert.
Die Nummern der eingesetzten Edukte sind in der Tabelle angegeben.

**Eingesetzte Edukte**

| **Beispiel** | **Benzisoxazol** | **Alkylphenylketon** | **Molmasse** | **Identifiziert durch: (Reinheit)** | **Name** |
|---|---|---|---|---|---|
| 1 | A | Z1 | 306,286992 | NMR | Benzo[d]isoxazol-3-yl-[1-(4-trifluormethyl-phenyl)-ethyl]-amin |
| 2 | A | Z2 | 338,350991 | NMR | Benzo[d]isoxazol-3-yl-[1-(4-trifluormethylsulfanyl-phenyl)-ethyl]-amin |

**Allgemeine Vorschrift zur reduktiven Aminierung des Amino-substituierten Benzisoxazol-Grundgerüstes:** 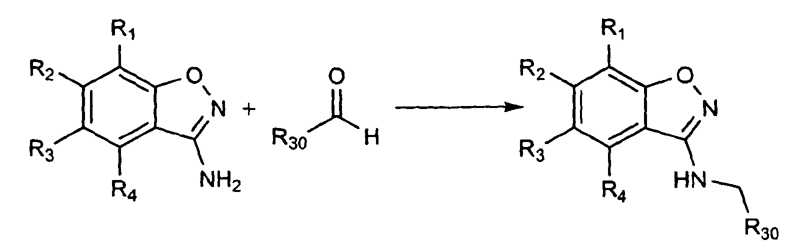 wobei R³⁰ für einen linearen oder verzweigten, gesättigten oder ungesättigten, unsubstituierten oder wenigstens einfach substituierten aliphatischen Rest;
für einen unsubstituierten oder wenigstens einfach substituierten Aryl- oder Heteroaryl-Rest steht, der mit einem mono- oder polyzyklischen Ringsystem kondensiert sein kann
oder für einen unsubstituierten oder wenigstens einfach substituierten Aryl- oder Heteroaryl-Rest steht, der mit einem mono- oder polyzyklischen Ringsystem kondensiert sein kann und über eine lineare oder verzweigte Alkylen-Gruppe gebunden ist.

### Durchführung

### Automatisierte Synthese

In ein trockenes Gewindeglas wurde bei RT 100 µmol Benzisoxazol-Lösung (Lösung I, 0,05 M in DCM, 2 ml) vorgelegt und mit 100 µmol des entsprechenden Aldehyds (Lösung II, 0,15 M in DCM, 0,66 ml) versetzt. Zu der Reaktionslösung wurde eine Lösung des Gemisches aus 120µmol Triethylsilan und 300 µmol Trifluoressigsäure gegeben (Lösung III, 0,1 M Triethylsilan in DCM, 0,25 M Trifluoressigsäure in DCM, 1,2 ml) hinzupipettiert. Das Gewindeglas wurde mit einer Septumkappe verschlossen, und die Reaktionslösung 16h unter Rückfluß bei 60°C im gerührt. Anschließend wurde die Reaktionslösung auf RT gebracht und mit 1,5 ml 7%iger NaCO₃-Lösung alkalisch eingestellt und 30 min. durchmischt. Der Rührfisch wurde abfiltriert, und das Gefäß mit 1,5 ml DCM ausgespült.
Die organische Phase wurde abgenommen und gesammelt. Die wäßrige Phase wurde mit 2ml DCM versetzt, gevortext und 15 min. intensiv durchmischt. Nach dem Zentrifugieren wurde die organische Phasen abgetrennt und mit der ersten Fraktion vereinigt. Die wäßrige Phase wurde analog ein zweites Mal mit DCM extrahiert. Anschließend wurden die vereinigten, organischen Phasen über eine MgSO₄-Kartusche getrocknet und in der GeneVac eingeengt.
Die Aufreinigung erfolgte mittels HPLC.

Nach dieser Vorschrift wurden die folgenden Aldehyde umgesetzt

| Nr. | Aldehyd | Nr. | Aldehyd |
|---|---|---|---|
| Z25 | 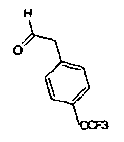 | Z26 | 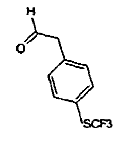 |
| Z3 | 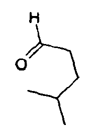 | Z27 | 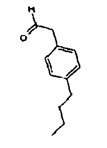 |
| Z4 | 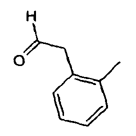 | Z28 | 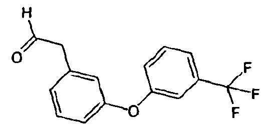 |
| Z5 | 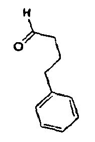 | Z29 | 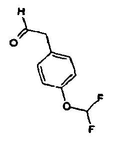 |
| Z6 | 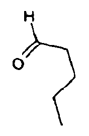 | Z30 | 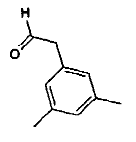 |
| Z7 | 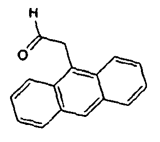 | Z31 | 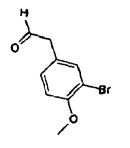 |
| Z8 | 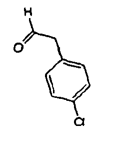 | Z32 | 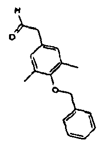 |
| Z9 | 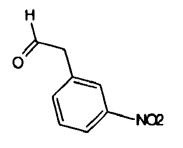 | Z33 | 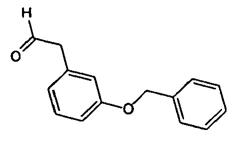 |
| Z10 | 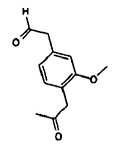 | Z34 | 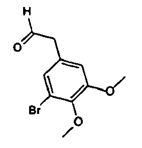 |
| Z11 | 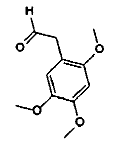 | Z35 | 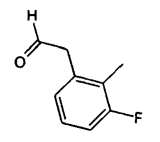 |
| Z12 | 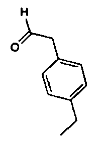 | Z36 | 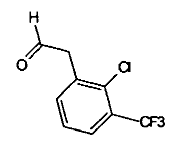 |
| Z13 | 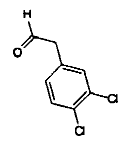 | Z37 | 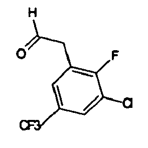 |
| Z14 | 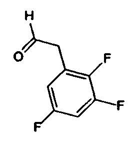 | Z38 | 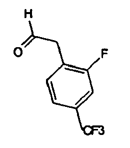 |
| Z15 | 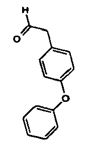 | Z39 | 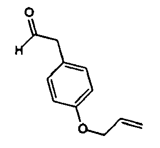 |
| Z16 | 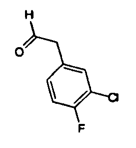 | Z40 | 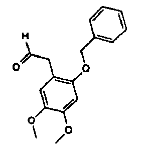 |
| Z17 | 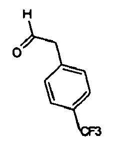 | Z41 | 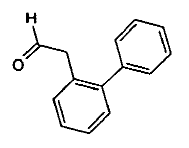 |
| Z18 | 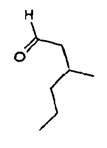 | Z42 | 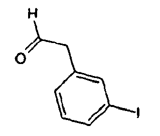 |
| Z19 | 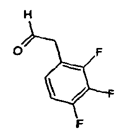 | Z43 | 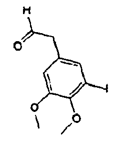 |
| Z20 | 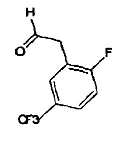 | Z44 | 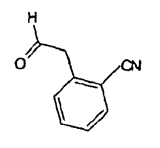 |
| Z21 | 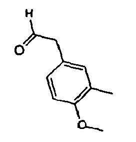 | Z45 | 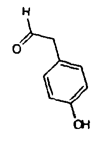 |
| Z22 | 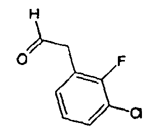 | Z46 | 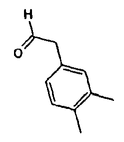 |
| Z23 | 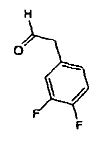 | Z47 | 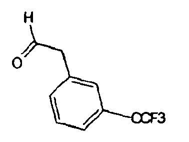 |
| Z24 | 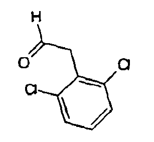 | | |

Die folgenden Beispiele wurden nach der vorstehenden Vorschrift synthetisiert.
Die Nummern der eingesetzten Edukte sind in der Tabelle angegeben.

**Eingesetzte Edukte**

| **Beispiel** | **Benzisoxazol** | **Aldehyd** | **Molmasse** | **Identifiziert durch: (Reinheit)** | **Name** |
|---|---|---|---|---|---|
| **136** | **A** | **Z3** | 204,272996 | MS | Benzo[d]isoxazol-3-yl-(3-methyl-butyl)-amin |
| **137** | **K** | **Z4** | 256,279994 | MS (>80%) | (5-Fluor-benzo[d]isoxazol- 3-yl)-(2-methyl-benzyl)-amin |
| **138** | **D** | **Z5** | 295,385994 | MS (>80%) | N4,N4-Dimethyl-N3-(3-phenyl-propyl)-benzo[d]isoxazol-3,4-diamin |
| **139** | **D** | **Z6** | 233,314996 | MS | N3-Butyl-N4,N4-dimethyl-benzo[d]isoxazol-3,4-diamin |
| **140** | **E** | **Z7** | 354,408992 | MS | Anthracen-9-ylmethyl-(4-methoxy-benzo[d]isoxazol-3-yl)-amin |
| **141** | **E** | **Z8** | 288,733994 | MS (>80%) | (4-Chlor-benzyl)-(4-methoxy-benzo[d]isoxazol-3-yl)-amin |
| **142** | **O** | **Z9** | 287,249994 | MS (>80%) | (6-Fluor-benzo[d]isoxazol-3-yl)-(3-nitro-benzyl)-amin |
| **143** | **M** | **Z10** | 346,769992 | MS (>80%) | Essigsäure-4-[(6-chlor-benzo[d]isoxazol-3-ylamino)-methyl]-2-methoxyphenylester |
| **144** | **N** | **Z10** | 391,22599 | MS (>80%) | Essigsäure-4-[(6-brom-benzo[d]isoxazol-3-ylamino)-methyl]-2-methoxyphenylester |
| **145** | **A** | **Z13** | 293,152993 | MS (>80%) | Benzo[d]isoxazol-3-yl-(3,4-dichlor-benzyl)-amin |
| **146** | **A** | **Z11** | 314,340993 | MS | Benzo[d]isoxazol-3-yl-(2,4,5-trimethoxy-benzyl)-amin |
| **147** | **A** | **Z12** | 252,316995 | MS (>80%) | Benzo[d]isoxazol-3-yl-(4-ethyl-benzyl)-amin |
| **148** | **M** | **Z13** | 327,597993 | MS (>80%) | (6-Chlor-benzo[d]isoxazol-3-yl)-(3,4-dichlor-benzyl)-amin |
| **149** | **A** | **Z14** | 278,232993 | MS (>80%) | Benzo[d]isoxazol-3-yl-(2,3,5-trifluor-benzyl)-amin |
| **150** | **M** | **Z15** | 350,804992 | MS (>80%) | (6-Chlor-benzo[d] isoxazol-3-yl)-(4-phenoxy-benzyl)-amin |
| **151** | **G** | **Z16** | 294,687993 | MS (>80%) | (3-Chlor-4-Fluor-benzyl)-(7-fluor-benzo[d]isoxazol-3-yl)-amin |
| **152** | **A** | **Z17** | 292,259992 | MS (>80%) | Benzo[d]isoxazol-3-yl-(4-trifluormethyl-benzyl)-amin |
| **153** | **G** | **Z18** | 236,289995 | MS (>80%) | (7-Fluor-benzo[d]isoxazol-3-yl)-(2-methyl-pentyl)-amin |
| **154** | **D** | **Z19** | 321,301992 | MS (>80%) | N4,N4-Dimethyl-N3-(2,3,4-trifluor-benzyl)-benzo[d]isoxazol-3,4-diamin |
| **155** | **D** | **Z20** | 353,318991 | MS (>80%) | N3-(2-Fluor-5-trifluormethyl-benzyl)-N4,N4-dimethyl-benzo[d]isoxazol-3,4-diamin |
| **156** | **H** | **Z21** | 283,330994 | MS (>80%) | N3-(4-Methoxy-3-methyl-benzyl)-benzo[d]isoxazol-3,4-diamin |
| **157** | **H** | **Z21** | 283,330994 | MS | N3-(4-Methoxy-3-methyl-benzyl)-benzo[d]isoxazol-3,4-diamin |
| **158** | **A** | **Z25** | 308,258992 | MS (>80%) | Benzo[d]isoxazol-3-yl-(4-trifluormethoxy-benzyl)-amin |
| **159** | **K** | **Z25** | 326,248991 | MS (>80%) | (5-Fluor-benzo[d]isoxazol-3-yl)-(4-trifluormethoxy-benzyl)-amin |
| **160** | **A** | **Z26** | 324,323992 | MS (>80%) | Benzo[d]isoxazol-3-yl-(4-trifluormethylsulfanyl-benzyl)-amin |
| **161** | **M** | **Z27** | 314,815993 | MS (>80%) | (4-Butyl-benzyl)-(6-chlor-benzo[d]isoxazol-3-yl)-amin |
| **162** | **K** | **Z26** | 342,313991 | MS (>80%) | (5-Fluor-benzo[d]isoxazol-3-yl)-(4-trifluormethylsulfanyl-benzyl)-amin |
| **163** | **A** | **Z38** | 310,249992 | MS (>80%) | Benzo[d]isoxazol-3-yl-(2-fluor-4-trifluormethyl-benzyl)-amin |
| **164** | **G** | **Z25** | 326,248991 | MS (>80%) | (7-Fluor-benzo[d]isoxazol-3-yl)-(4-trifluormethoxy-benzyl)-amin |
| **165** | **G** | **Z28** | 402,346989 | MS (>80%) | (7-Fluor-benzo[d]isoxazol-3-yl)-[3-(3-trifluormethyl-phenoxy)-benzyl]-amin |
| **166** | **E** | **Z29** | 320,294992 | MS (>80%) | (4-Difluormethoxy-benzyl)-(4-methoxy-benzo[d]isoxazol-3-yl)-amin |
| **167** | **G** | **Z30** | 270,306994 | MS (>80%) | (3,5-Dimethyl-benzyl)-(7-fluor-benzo[d]isoxazol-3-yl)-amin |
| **168** | **O** | **Z31** | 351,17999 | MS (>80%) | (3-Brom-4-methoxy-benzyl)-(6-fluor-benzo[d]isoxazol-3-yl)-amin |
| **169** | **O** | **Z30** | 270,306994 | MS (>80%) | (3,5-Dimethyl-benzyl)-(6-fluor-benzo[d]isoxazol-3-yl)-amin |
| **170** | **O** | **Z32** | 376,430991 | MS | (4-Benzyloxy-3,5-dimethyl-benzyl)-(6-fluor-benzo[d]isoxazol-3-yl)-amin |
| **171** | **O** | **Z27** | 298,360993 | MS (>80%) | (4-Butyl-benzyl)-(6-fluor-benzo[d]isoxazol-3-yl)-amin |
| **172** | **O** | **Z26** | 342,313991 | MS (>80%) | (6-Fluor-benzo[d]isoxazol-3-yl)-(4-trifluormethylsulfanyl-benzyl)-amin |
| **173** | **O** | **Z33** | 348,376992 | MS (>80%) | (3-Benzyloxy-benzyl)-(6-fluor-benzo[d]isoxazol-3-yl)-amin |
| **174** | **H** | **Z30** | 267,331995 | MS | N3-(3,5-Dimethyl-benzyl)-benzo[d]isoxazol-3,4-diamin |
| **175** | **H** | **Z27** | 295,385994 | MS | N3-(4-Butyl-benzyl)-benzo[d]isoxazol-3,4-diamin |
| **176** | **L** | **Z26** | 403,224988 | MS (>80%) | (5-Brom-benzo[d]isoxazol-3-yl)-(4-trifluormethylsulfanyl-benzyl)-amin |
| **177** | **G** | **Z34** | 381,20599 | MS (>80%) | (3-Brom-4,5-dimethoxy-benzyl)-(7-fluor-benzo[d]isoxazol-3-yl)-amin |
| **178** | **G** | **Z38** | 328,239991 | MS (>80%) | (7-Fluor-benzo[d]isoxazol-3-yl)-(2-fluor-4-trifluormethyl-benzyl)-amin |
| **179** | **D** | **Z35** | 299,348994 | MS (>80%) | N3-(3-Fluor-2-methyl-benzyl)-N4,N4-dimethyl-benzo[d]isoxazol-3,4-diamin |
| **180** | **D** | **Z36** | 369,773991 | MS (>80%) | N3-(2-Chlor-3-trifluormethyl-benzyl)-N4,N4-dimethyl-benzo[d]isoxazol-3,4-diamin |
| **181** | **D** | **Z37** | 387,76399 | MS | N3-(3-Chlor-2-fluor-5-trifluormethyl-benzyl)-N4,N4-dimethyl-benzo[d]isoxazol-3,4-diamin |
| **182** | **O** | **Z38** | 328,239991 | MS (>80%) | (6-Fluor-benzo[d]isoxazol-3-yl)-(2-fluor-4-trifluormethyl-benzyl)-amin |
| **183** | **O** | **Z39** | 298,316993 | MS (>80%) | (4-Allyloxy-benzyl)-(6-fluor-benzo[d]isoxazol-3-yl)-amin |
| **184** | **A** | **Z40** | 390,438991 | MS (>80%) | Benzo[d]isoxazol-3-yl-(2-benzyloxy-4,5-dimethoxy-benzyl)-amin |
| **185** | **M** | **Z40** | 424,88399 | MS | (2-Benzyloxy-4,5-dimethoxy-benzyl)-(6-chlor-benzo[d]isoxazol-3-yl)-amin |
| **186** | **D** | **Z40** | 433,507991 | MS (>80%) | N3-(2-Benzyloxy-4,5-dimethoxy-benzyl)-N4,N4-dimethyl-benzo[d]isoxazol-3,4-diamin |
| **187** | **D** | **Z41** | 343,429993 | MS | N3-Biphenyl-2-ylmethyl-N4,N4-dimethyl-benzo[d]isoxazol-3,4-diamin |
| **188** | **O** | **Z42** | 368,148994 | MS (>80%) | (6-Fluor-benzo[d]isoxazol-3-yl)-(3-iod-benzyl)-amin |
| **189** | **E** | **Z40** | 420,464991 | MS (>80%) | (2-Benzyloxy-4,5-dimethoxy-benzyl)-(4-methoxy-benzo[d]isoxazol-3-yl)-amin |
| **190** | **B** | **Z43** | 428,200992 | MS (>80%) | (4-Fluor-benzo[d]isoxazol-3-yl)-(3-iodo-4,5-dimethoxy-benzyl)-amin |
| **191** | **J** | **Z44** | 263,299995 | MS | 2-[(5-Methyl-benzo[d]isoxazol-3-ylamino)-methyl]-benzonitril |
| **192** | **F** | **Z6** | 288,268993 | MS (>80%) | Butyl-[4-(2,2,2-trifluor-ethoxy)-benzo[d]isoxazol-3-yl]-amin |
| **193** | **J** | **Z34** | 377,24299 | MS (>80%) | (3-Brom-4,5-dimethoxy-benzyl)-(5-methyl-benzo[d]isoxazol-3-yl)-amin |
| **198** | **C** | **Z45** | 274,706994 | MS (>80%) | 4-[(4-Chlor-benzo[d]isoxazol-3-ylamino)-methyl]-phenol |
| **199** | **M** | **Z46** | 286,761994 | MS (>80%) | (6-Chlor-benzo[d]isoxazol-3-yl)-(3,4-dimethyl-benzyl)-amin |
| **200** | **C** | **Z22** | 311,142993 | MS (>80%) | (4-Chlor-benzo[d]isoxazol-3-yl)-(3-chlor-2-fluor-benzyl)-amin |
| **201** | **K** | **Z23** | 278,232993 | MS (>80%) | (3,4-Difluor-benzyl)-(5-fluor-benzo[d]isoxazol-3-yl)-amin |
| **202** | **N** | **Z24** | 372,05399 | MS (>80%) | (6-Brom-benzo[d]isoxazol-3-yl)-(2,6-dichlor-benzyl)-amin |
| **203** | **G** | **Z47** | 326,248991 | MS (>80%) | (7-Fluor-benzo[d]isoxazol-3-yl)-(3-trifluormethoxy-benzyl)-amin |

### Synthese der Thioharnstoffe

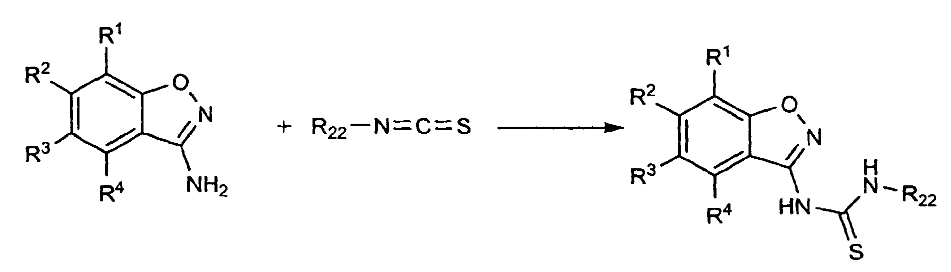

### Automatisierte Synthese

In ein trockenes Gewindeglas mit Septumkappe wurden bei RT 100 µmol Benzisoxazol-Derivat-Lösung (Lösung I; 0,1 M in Pyridin, 1 ml)) vorgelegt und mit 100 µmol lsothiocyanat-Derivat-Lösung (Lösung II; 0,1 M in Pyridin, 1 ml) versetzt. Die Reaktionslösung wurde 24h unter Rückfluß gerührt. Der Rührfisch wurde abfiltriert, und das Gefäß mit 2ml CH₂Cl₂ ausgespült.
Die Suspension wurde kurz aufgeschwänkt und direkt über einen 0,45 µm GHP Filter Funnel gegossen. Das Reagenzglas wurde mit 7,5 ml DCM ausgespült und anschließend wurde die Suspension im Filter-Funnel mit Hilfe von Druckluft abfiltriert.
Die klare organische Phase wurde in der Gene-Vac eingeengt.
Die Aufreinigung erfolgte durch HPLC.

Die folgenden Isothiocyanate wurden eingesetzt:

| | | | | | |
|---|---|---|---|---|---|
| I1 | 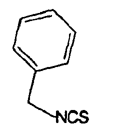 | I28 | 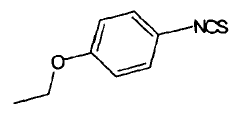 | I55 | 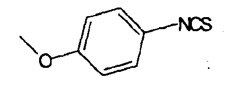 |
| I2 | 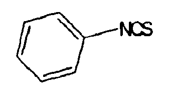 | I29 | 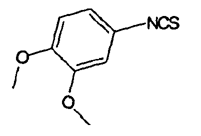 | I56 | 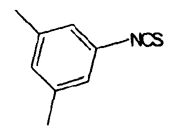 |
| I3 | 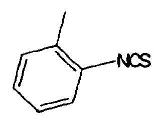 | I30 | 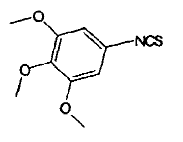 | I57 | 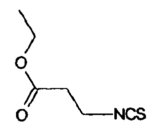 |
| I4 | 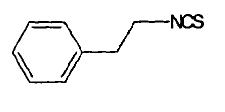 | I31 | 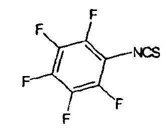 | I58 | 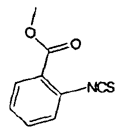 |
| I5 | 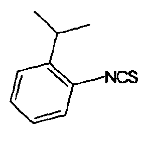 | I32 | 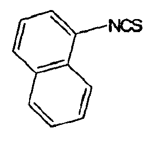 | I59 | 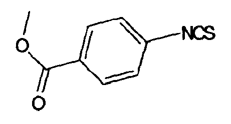 |
| I6 | 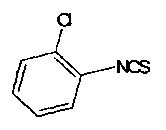 | I33 | 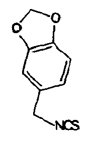 | I60 | 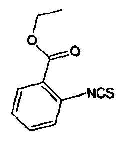 |
| I7 | 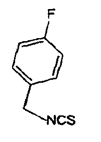 | I34 | 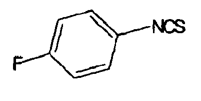 | I61 | 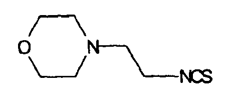 |
| I8 | 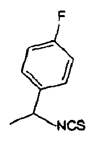 | I35 | 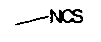 | I62 | 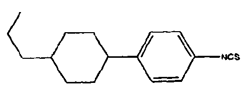 |
| I9 | 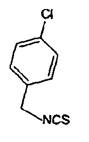 | I36 | 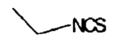 | I63 | 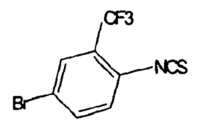 |
| I10 | 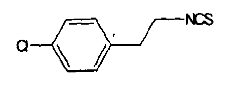 | I37 | 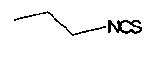 | I64 | 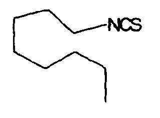 |
| I11 | 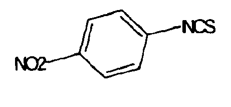 | I38 | 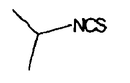 | I65 | 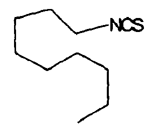 |
| I12 | 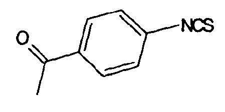 | I39 | 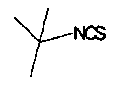 | I66 | 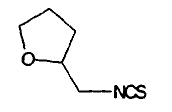 |
| I13 | 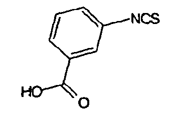 | I40 | 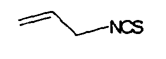 | I67 | 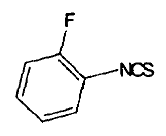 |
| I14 | 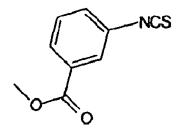 | I41 | 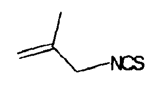 | I68 | 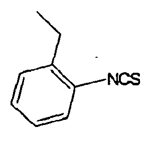 |
| I15 | 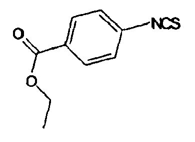 | I42 | 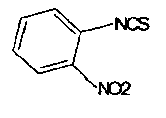 | I69 | 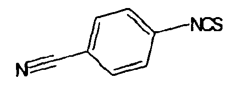 |
| I16 | 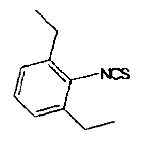 | I43 | 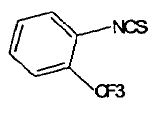 | I70 | 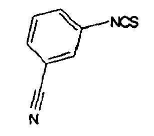 |
| I17 | 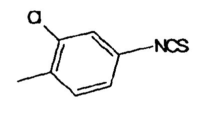 | I44 | 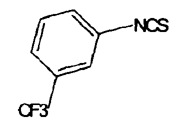 | I71 | 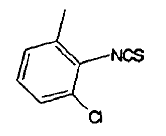 |
| I18 | 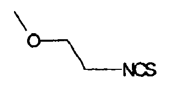 | I45 | 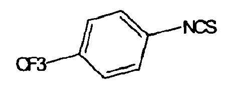 | I72 | 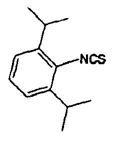 |
| I19 | 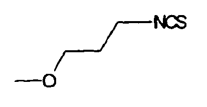 | I46 | 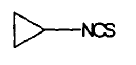 | I73 | 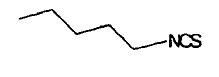 |
| I20 | 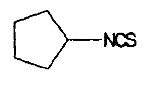 | I47 | 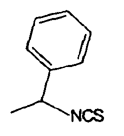 | I74 | 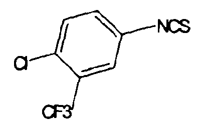 |
| I21 | 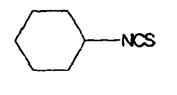 | I48 | 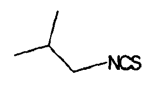 | I75 | 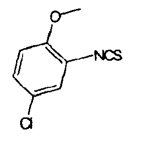 |
| I22 | 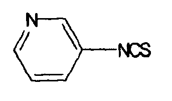 | I49 | 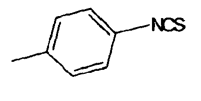 | I76 | 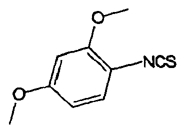 |
| I23 | 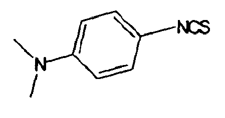 | I50 | 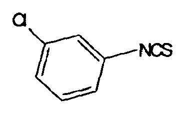 | I77 | 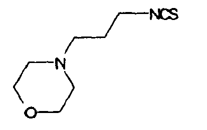 |
| I24 | 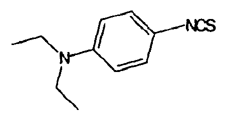 | I51 | 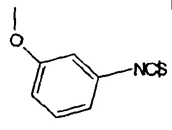 | I78 | 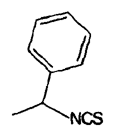 |
| I25 | 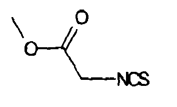 | I52 | 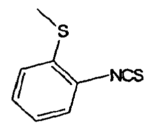 | I79 | 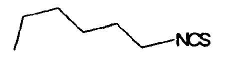 |
| I26 | 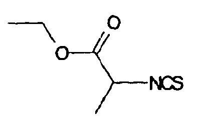 | I53 | 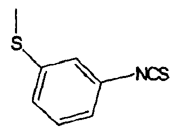 | I80 | 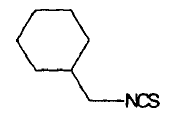 |
| I27 | 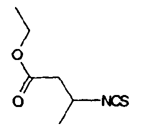 | I54 | 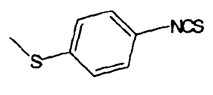 | I81 | 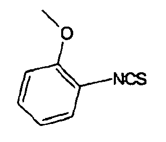 |
| | | | | I82 | 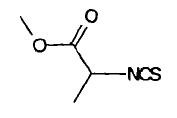 |

Die folgenden Beispiele wurden nach der vorstehenden Vorschrift synthetisiert.

**Eingesetzte Edukte**

| **Beispiel** | **Benzisoxazol** | **Isothiocyanat** | **Exakte Masse** | **Identifiziert durch: (Reinheit)** | **Name** |
|---|---|---|---|---|---|
| **3** | **A** | **I1** | 283,352994 | MS (>80%) | 1-Benzo[d]isoxazol-3-yl-3-benzyl-thioharnstoff |
| **4** | **A** | **I2** | 269,325994 | MS (>80%) | 1-Benzo[d]isoxazol-3-yl-3-phenyl-thioharnstoff |
| **5** | **A** | **I3** | 283,352994 | MS (>80%) | 1-Benzo[d]isoxazol-3-yl-3-o-tolyl-thioharnstoff |
| **6** | **A** | **I4** | 297,379994 | MS (>80%) | 1-Benzo[d]isoxazol-3-yl-3-phenethyl-thioharnstoff |
| **7** | **A** | **I5** | 311,406994 | MS (>80%) | 1-Benzo[d]isoxazol-3-yl-3-(2-isopropyl-phenyl)-thioharnstoff |
| **8** | **A** | **I6** | 303,770993 | MS (>80%) | 1-Benzo[d]isoxazol-3-yl-3-(2-chlor-phenyl)-thioharnstoff |
| **9** | **A** | **I7** | 301,342993 | MS (>80%) | 1-Benzo[d]isoxazol-3-yl-3-(4-fluor-benzyl)-thioharnstoff |
| **10** | **A** | **I8** | 315,369993 | MS (>80%) | 1-Benzo[d]isoxazol-3-yl-3-[1-(4-fluor-phenyl)-ethyl]-thioharnstoff |
| **11** | **A** | **I9** | 317,797993 | MS (>80%) | 1-Benzo[d]isoxazol-3-yl-3-(4-chlor-benzyl)-thioharnstoff |
| **12** | **A** | **I10** | 331,824993 | MS (>80%) | 1-Benzo[d]isoxazol-3-yl-3-[2-(4-chlor-phenyl)-ethyl]-thioharnstoff |
| **13** | **A** | **I11** | 314,322994 | MS (>80%) | 1-Benzo[d]isoxazol-3-yl-3-(4-nitro-phenyl)-thioharnstoff |
| **14** | **A** | **I12** | 311,362993 | MS (>80%) | 1-(4-Acetyl-phenyl)-3-benzo[d]isoxazol-3-yl-thioharnstoff |
| **15** | **A** | **I13** | 313,334993 | MS (>80%) | 3-(3-Benzo[d]isoxazol-3-yl-thioureido)-benzoesäure |
| **16** | **A** | **I14** | 327,361993 | MS (>80%) | 3-(3-Benzo[d]isoxazol-3-yl-thioureido)-benzoesäuremethylester |
| **17** | **A** | **I15** | 341,388993 | MS (>80%) | 4-(3-Benzo[d]isoxazol-3-yl-thioureido)-benzoesäureethylester |
| **18** | **A** | **I16** | 325,433993 | MS (>80%) | 1-Benzo[d]isoxazol-3-yl-3-(2,6-diethyl-phenyl)-thioharnstoff |
| **19** | **A** | **I17** | 317,797993 | MS (>80%) | 1-Benzo[d]isoxazol-3-yl-3-(3-chlor-4-methyl-phenyl)-thioharnstoff |
| **20** | **A** | **I18** | 251,307995 | MS (>80%) | 1-Benzo[d]isoxazol-3-yl-3-(2-methoxy-ethyl)-thioharnstoff |
| **21** | **A** | **I19** | 265,334995 | MS (>80%) | 1-Benzo[d]isoxazol-3-yl-3-(3-methoxy-propyl)-thioharnstoff |
| **22** | **A** | **I20** | 261,346995 | MS (>80%) | 1-Benzo[d]isoxazol-3-yl-3-cyclopentyl-thioharnstoff |
| **23** | **A** | **I21** | 275,373994 | MS (>80%) | 1-Benzo[d]isoxazol-3-yl-3-cyclohexyl-thioharnstoff |
| **24** | **A** | **I22** | 270,313995 | MS (>80%) | 1-Benzo[d]isoxazol-3-yl-3-pyridin-3-yl-thioharnstoff |
| **25** | **A** | **I23** | 312,394994 | MS (>80%) | 1-Benzo[d]isoxazol-3-yl-3-(4-dimethylamino-phenyl)-thioharnstoff |
| **26** | **A** | **I24** | 340,448993 | MS (>80%) | 1-Benzo[d]isoxazol-3-yl-3-(4-diethylamino-phenyl)-thioharnstoff |
| **27** | **A** | **I25** | 265,290995 | MS (>80%) | (3-Benzo[d]isoxazol-3-yl-thioureido)-essigsäuremethylester |
| **28** | **A** | **I26** | 293,344994 | MS (>80%) | 2-(3-Benzo[d]isoxazol-3-yl-thioureido)-propionsäureethylester |
| **29** | **A** | **I27** | 307,371994 | MS (>80%) | 3-(3-Benzo[d]isoxazol-3-yl-thioureido)-buttersäureethylester |
| **30** | **A** | **I80** | 289,400994 | MS (>80%) | 1-Benzo[d]isoxazol-3-yl-3-cyclohexylmethyl-thioharnstoff |
| **31** | **A** | **I28** | 313,378993 | MS (>80%) | 1-Benzo[d]isoxazol-3-yl-3-(4-ethoxy-phenyl)-hioharnstoff |
| **32** | **A** | **I29** | 329,377993 | MS (>80%) | 1-Benzo[d]isoxazol-3-yl-3-(3,4-dimethoxy-phenyl)-thioharnstoff |
| **33** | **A** | **I30** | 359,403992 | MS (>80%) | 1-Benzo[d]isoxazol-3-yl-3-(3,4,5-trimethoxy-phenyl)-thioharnstoff |
| **34** | **A** | **I31** | 359,27599 | MS (>80%) | 1-Benzo[d]isoxazol-3-yl-3-pentafluorphenyl-thioharnstoff |
| **35** | **A** | **I32** | 319,385993 | MS (>80%) | 1-Benzo[d]isoxazol-3-yl-3-naphthalen-1-yl-thioharnstoff |
| **36** | **A** | **I33** | 327,361993 | MS (>80%) | 1-Benzo[1,3]dioxol-5-ylmethyl-3-benzo[d]isoxazol-3-yl-thioharnstoff |
| **37** | **A** | **I34** | 287,315994 | MS (>80%) | 1-Benzo[d]isoxazol-3-yl-3-(4-fluor-phenyl)-thioharnstoff |
| **38** | **A** | **I35** | 207,254996 | MS (>80%) | 1-Benzo[d]isoxazol-3-yl-3-methyl-thioharnstoff |
| **39** | **A** | **I36** | 221,281996 | MS (>80%) | 1-Benzo[d]isoxazol-3-yl-3-ethyl-thioharnstoff |
| **40** | **A** | **I37** | 235,308995 | MS (>80%) | 1-Benzo[d]isoxazol-3-yl-3-propyl-thioharnstoff |
| **41** | **A** | **I38** | 235,308995 | MS (>80%) | 1-Benzo[d]isoxazol-3-yl-3-isopropyl-thioharnstoff |
| **42** | **A** | **I39** | 249,335995 | MS (>80%) | 1-Benzo[d]isoxazol-3-yl-3-tert-butyl-thioharnstoff |
| **43** | **A** | **I40** | 233,292995 | MS | 1-Allyl-3-benzo[d]isoxazol-3-yl-thioharnstoff |
| **44** | **A** | **I41** | 247,319995 | MS (>80%) | 1-Benzo[d]isoxazol-3-yl-3-(2-methyl-allyl)-thioharnstoff |
| **45** | **A** | **I42** | 314,322994 | NMR | 1-Benzo[d]isoxazol-3-yl-3-(2-nitro-phenyl)-thioharnstoff |
| **46** | **A** | **I43** | 337,322992 | MS (>80%) | 1-Benzo[d]isoxazol-3-yl-3-(2-trifluormethyl-phenyl)-thioharnstoff |
| **47** | **A** | **I44** | 337,322992 | MS (>80%) | 1-Benzo[d]isoxazol-3-yl-3-(3-trifluormethyl-phenyl)-thioharnstoff |
| **48** | **A** | **I45** | 337,322992 | MS (>80%) | 1-Benzo[d]isoxazol-3-yl-3-(4-trifluormethyl-phenyl)-thioharnstoff |
| **49** | **A** | **I46** | 233,292995 | MS (>80%) | 1-Benzo[d]isoxazol-3-yl-3-cyclopropyl-thioharnstoff |
| **50** | **B** | **I82** | 297,307993 | MS (>80%) | 2-[3-(4-Fluor-benzo[d]isoxazol-3-yl)-thioureidol-propionsäuremethylester |
| **51** | **C** | **I3** | 317,797993 | MS | 1-(4-Chlor-benzo[d]isoxazol-3-yl)-3-o-tolyl-thioharnstoff |
| **52** | **C** | **I1** | 317,797993 | MS (>80%) | 1-Benzyl-3-(4-chlor-benzo[d]isoxazol-3-yl)-thioharnstoff |
| **53** | **C** | **I47** | 331,824993 | MS (>80%) | 1-(4-Chlor-benzo[d]isoxazol-3-yl)-3-(1-phenyl-ethyl)-thioharnstoff |
| **54** | **D** | **I48** | 292,404994 | MS | 1-(4-Dimethylamino-benzo[d]isoxazol-3-yl)-3-isobutyl-thioharnstoff |
| **55** | **D** | **I49** | 326,421993 | MS | 1-(4-Dimethylamino-benzo[d]isoxazol--yl)-3-p-tolyl-thioharnstoff |
| **56** | **D** | **I50** | 346,839993 | MS (>80%) | 1-(3-Chlor-phenyl)-3-(4-dimethylamino-benzo[d]isoxazol-3-yl)-thioharnstoff |
| **57** | **D** | **I51** | 342,420993 | MS (>80%) | 1-(4-Dimethylamino-benzo[d]isoxazol-3-yl)-3-(3-methoxy-phenyl)-thioharnstoff |
| **58** | **D** | **I52** | 358,485993 | MS (>80%) | 1-(4-Dimethylamino-benzo[d]isoxazol-3-yl)-3-(2-methylsulfanyl-phenyl)-thioharnstoff |
| **59** | **D** | **I53** | 358,485993 | MS (>80%) | 1-(4-Dimethylamino-benzo[d]isoxazol-3-yl)-3-(3-methylsulfanyl-phenyl)-thioharnstoff |
| **60** | **D** | **I54** | 358,485993 | MS (>80%) | 1-(4-Dimethylamino-benzo[d]isoxazol-3-yl)-3-(4-methylsulfanyl-phenyl)-thioharnstoff |
| **61** | **D** | **I81** | 342,420993 | MS (>80%) | 1-(4-Dimethylamino-benzo[d]isoxazol-3-yl)-3-(2-methoxy-phenyl)-thioharnstoff |
| **62** | **D** | **I55** | 342,420993 | MS (>80%) | 1-(4-Dimethylamino-benzo[d]isoxazol-3-yl)-3-(4-methoxy-phenyl)-thioharnstoff |
| **63** | **D** | **I56** | 340,448993 | MS (>80%) | 1-(4-Dimethylamino-benzo[d]isoxazol-3-yl)-3-(3,5-dimethyl-phenyl)-thioharnstoff |
| **64** | **D** | **I1** | 326,421993 | MS (>80%) | 1-Benzyl-3-(4-dimethylamino-benzo[d]isoxazol-3-yl)-thioharnstoff |
| **65** | **D** | **I19** | 308,403994 | MS (>80%) | 1-(4-Dimethylamino-benzo[d]isoxazol-3-yl)-3-(3-methoxy-propyl)-thioharnstoff |
| **66** | **D** | **I57** | 336,413993 | MS (>80%) | 3-[3-(4-Dimethylamino-benzo[d]isoxazol-3-yl)-thioureido]-propionsäurethylester |
| **67** | **D** | **I26** | 336,413993 | MS (>80%) | 2-[3-(4-Dimethylamino-benzo[d]isoxazol-3-yl)-thioureido]-propionsäurethylester |
| **68** | **D** | **I27** | 350,440993 | MS (>80%) | 3-[3-(4-Dimethylamino-benzo[d]isoxazol-3-yl)-thioureido]-buttersäureethylester |
| **69** | **D** | **I13** | 356,403993 | MS (>80%) | 3-[3-(4-Dimethylamino-benzo[d]isoxazol-3-yl)-thioureido]-benzoesäure |
| **70** | **D** | **I28** | 356,447993 | MS (>80%) | 1-(4-Dimethylamino-benzo[d]isoxazol-3-yl)-3-(4-ethoxy-phenyl)-thioharnstoff |
| **71** | **D** | **I58** | 370,430992 | MS (>80%) | 2-[3-(4-Dimethylamino-benzo[d]isoxazol-3-yl)-thioureido]-benzoesäuremethylester |
| **72** | **D** | **I14** | 370,430992 | MS | 3-[3-(4-Dimethylamino-benzo[d]isoxazol-3-yl)-thioureido]-benzoesäuremethylester |
| **73** | **D** | **I59** | 370,430992 | MS (>80%) | 4-[3-(4-Dimethylamino-benzo[d]isoxazol-3-yl)-thioureido]-benzoesäuremethylester |
| **74** | **D** | **I60** | 384,457992 | MS (>80%) | 2-[3-(4-Dimethylamino-benzo[d]isoxazol-3-yl)-thioureido]-benzoesäureethylester |
| **75** | **D** | **I15** | 384,457992 | MS (>80%) | 4-[3-(4-Dimethylamino-benzo[d]isoxazol-3-yl)-thioureido]-benzoesäureethylester |
| **76** | **D** | **I12** | 354,431993 | MS | 1-(4-Acetyl-phenyl)-3-(4-dimethylamino-benzo[d]isoxazol-3-yl)-thioharnstoff |
| **77** | **E** | **I6** | 333,796993 | MS (>80%) | 1-(2-Chlor-phenyl)-3-(4-methoxy-benzo[d]isoxazol-3-yl)-thioharnstoff |
| **78** | **E** | **I24** | 370,474993 | MS (>80%) | 1-(4-Diethylamino-phenyl)-3-(4-methoxy-benzo[d]isoxazol-3-yl)-thioharnstoff |
| **79** | **E** | **I61** | 336,413993 | MS (>80%) | 1-(4-Methoxy-benzo[d]isoxazol-3-yl)-3-(2-morpholin-4-yl-ethyl)-thioharnstoff |
| **80** | **F** | **I82** | 377,340991 | MS (>80%) | 2-{3-[4-(2,2,2-trifluor-ethoxy)-benzo[d]isoxazol-3-yl]-thioureido}-propionsäuremethylester |
| **81** | **F** | **I33** | 425,38499 | MS (>80%) | 1-Benzo[1,3]dioxol-5-ylmethyl-3 -[4-(2,2,2-trifluor-ethoxy)-benzo[d]isoxazol-3-yl]-thioharnstoff |
| **82** | **F** | **I62** | 491,575988 | MS (>80%) | 1-[4-(4-Propyl-cyclohexyl)-phenyl]-3-[4-(2,2,2-trifluor-ethoxy)-benzo[d]isoxazol-3-yl]-thioharnstoff |
| **83** | **F** | **I63** | 514,246985 | MS (>80%) | 1-(4-Brom-2-trifluormethyl-phenyl)-3-[4-(2,2,2-trifluor-ethoxy)-benzo[d]isoxazol-3-yl]-thioharnstoff |
| **84** | **F** | **I55** | 397,37499 | MS (>80%) | 1-(4-Methoxy-phenyl)-3-[4-(2,2,2-trifluor-ethoxy)-benzo[d]isoxazol-3-yl]-thioharnstoff |
| **85** | **F** | **I57** | 391,36799 | MS (>80%) | 3-{3-[4-(2,2,2-trifluor-ethoxy)-benzo[d]isoxazol-3-yl]-thioureido}-propionsäureethylester |
| **86** | **I** | **I64** | 425,594991 | MS (>80%) | 1-[4-(4-Methyl-benzyloxy)-benzo[d]isoxazol-3-yl]-3-octyl-thioharnstoff |
| **87** | **I** | **I65** | 439,621991 | MS (>80%) | 1-[4-(4-Methyl-benzyloxy)-benzo[d]isoxazol-3-yl]-3-nonyl-thioharnstoff |
| **88** | **I** | **I46** | 353,443992 | MS (>80%) | 1-Cyclopropyl-3-[4-(4-methyl-benzyloxy)-benzo[d]isoxazol-3-yl]-thioharnstoff |
| **89** | **I** | **I20** | 381,497992 | MS (>80%) | 1-Cyclopentyl-3-[4-(4-methyl-benzyloxy)-benzo[d]isoxazol-3-yl]-thioharnstoff |
| **90** | **I** | **I21** | 395,524992 | MS (>80%) | 1-Cyclohexyl-3-[4-(4-methyl-benzyloxy)-benzo[d]isoxazol-3-yl]-thioharnstoff |
| **91** | **I** | **I80** | 409,551991 | MS (>80%) | 1-Cyclohexylmethyl-3-[4-(4-methyl-benzyloxy)-benzo[d]isoxazol-3-yl]-thioharnstoff |
| **92** | **F** | **I23** | 410,41799 | MS (>80%) | 1-(4-Dimethylamino-phenyl)-3-[4-(2,2,2-trifluor-ethoxy)-benzo[d]isoxazol-3-yl]-thioharnstoff |
| **93** | **J** | **I40** | 247,319995 | MS | 1-Allyl-3-(5-methyl-benzo[d]isoxazol-3-yl)-thioharnstoff |
| **94** | **J** | **I25** | 279,317994 | MS (>80%) | [3-(5-Methyl-benzo[d]isoxazol-3-yl)-thioureido]-essigsäuremethylester |
| **95** | **J** | **I5** | 325,433993 | MS (>80%) | 1-(2-Isopropyl-phenyl)-3-(5-methyl-benzo[d]isoxazol-3-yl)-thioharnstoff |
| **96** | **J** | **I45** | 351,349991 | MS (>80%) | 1-(5-Methyl-benzo[d]isoxazol-3-yl)-3-(4-trifluormethyl-phenyl)-thioharnstoff |
| **97** | **K** | **I82** | 297,307993 | MS (>80%) | 2-[3-(5-Fluor-benzo[d]isoxazol-3-yl)-thioureido]-propionsäuremethylester |
| **98** | **K** | **I66** | 295,335994 | MS (>80%) | 1-(5-Fluor-benzo[d]isoxazol-3-yl)-3-(tetrahydro-furan-2-ylmethyl)-thioharnstoff |
| **99** | **L** | **I67** | 366,21699 | MS (>80%) | 1-(5-Brom-benzo[d]isoxazol-3-yl)-3-(2-fluor-phenyl)-thioharnstoff |
| **100** | **L** | **I68** | 376,28099 | MS (>80%) | 1-(5-Brom-benzo[d]isoxazol-3-yl)-3-(2-ethyl-phenyl)-thioharnstoff |
| **101** | **M** | **I34** | 321,760993 | MS (>80%) | 1-(6-Chlor-benzo[d]isoxazol-3-yl)-3-(4-fluor-phenyl)-thioharnstoff |
| **102** | **M** | **I67** | 321,760993 | MS (>80%) | 1-(6-Chlor-benzo[d]isoxazol-3-yl)-3-(2-fluor-phenyl)-thioharnstoff |
| **103** | **M** | **I20** | 295,791994 | MS (>80%) | 1-(6-Chlor-benzo[d]isoxazol-3-yl)-3-cyclopentyl-thioharnstoff |
| **104** | **M** | **I69** | 328,780993 | MS (>80%) | 1-(6-Chlor-benzo[d]isoxazol-3-yl)-3-(4-cyano-phenyl)-thioharnstoff |
| **105** | **M** | **I13** | 347,779992 | MS (>80%) | 3-[3-(6-Chlor-benzo[d]isoxazol-3-yl)-thioureido]-benzoesäure |
| **106** | **M** | **I55** | 333,796993 | MS (>80%) | 1-(6-Chlor-benzo[d]isoxazol-3-yl)-3-(4-methoxy-phenyl)-thioharnstoff |
| **107** | **M** | **I51** | 333,796993 | MS (>80%) | 1-(6-Chlor-benzo[d]isoxazol-3-yl)-3-(3-methoxy-phenyl)-thioharnstoff |
| **108** | **N** | **I29** | 408,27899 | MS (>80%) | 1-(6-Brom-benzo[d]isoxazol-3-yl)-3-(3,4-dimethoxy-phenyl)-thioharnstoff |
| **109** | **N** | **I32** | 398,28699 | MS | 1-(6-Brom-benzo[d]isoxazol-3-yl)-3-naphthalen-1-yl-thioharnstoff |
| **110** | **N** | **I33** | 406,26299 | MS | 1-Benzo[1,3]dioxol-5-ylmethyl-3-(6-brom-benzo[d]isoxazol-3-yl)-thioharnstoff |
| **111** | **O** | **I52** | 333,406992 | MS (>80%) | 1-(6-Fluor-benzo[d]isoxazol-3-yl)-3-(2-methylsulfanyl-phenyl)-thioharnstoff |
| **112** | **O** | **I70** | 312,325993 | MS (>80%) | 1-(3-Cyano-phenyl)-3-(6-fluor-benzo[d]isoxazol-3-yl)-thioharnstoff |
| **113** | **O** | **I71** | 335,787992 | MS (>80%) | 1-(2-Chlor-6-methyl-phenyl)-3-(6-fluor-benzo[d]isoxazol-3-yl)-thioharnstoff |
| **114** | **O** | **I72** | 371,477992 | MS (>80%) | 1-(2,6-Diisopropyl-phenyl)-3-(6-fluor-benzo[d]isoxazol-3-yl)-thioharnstoff |
| **115** | **G** | **I35** | 225,244995 | MS (>80%) | 1-(7-Fluor-benzo[d]isoxazol-3-yl)-3-methyl-thioharnstoff |
| **116** | **G** | **I36** | 239,271995 | MS (>80%) | 1-Ethyl-3-(7-fluor-benzo[d]isoxazol-3-yl)-thioharnstoff |
| **117** | **G** | **I37** | 253,298995 | MS (>80%) | 1-(7-Fluor-benzo[d]isoxazol-3-yl)-3-propyl-thioharnstoff |
| **118** | **G** | **I73** | 281,352994 | MS (>80%) | 1-(7-Fluor-benzo[d]isoxazol-3-yl)-3-pentyl-thioharnstoff |
| **119** | **G** | **I79** | 295,379994 | MS (>80%) | 1-(7-Fluor-benzo[d]isoxazol-3-yl)-3-hexyl-thioharnstoff |
| **120** | **G** | **I64** | 323,433993 | MS (>80%) | 1-(7-Fluor-benzo[d]isoxazol-3-yl)-3-octyl-thioharnstoff |
| **121** | **G** | **I65** | 337,460993 | MS | 1-(7-Fluor-benzo[d]isoxazol-3-yl)-3-nonyl-thioharnstoff |
| **122** | **G** | **I48** | 267,325994 | MS (>80%) | 1-(7-Fluor-benzo[d]isoxazol-3-yl)-3-isobutyl-thioharnstoff |
| **123** | **G** | **I40** | 251,282995 | MS | 1-Allyl-3-(7-fluor-benzo[d]isoxazol-3-yl)-thioharnstoff |
| **124** | **G** | **I49** | 301,342993 | MS | 1-(7-Fluor-benzo[d]isoxazol-3-yl)-3-p-tolyl-thioharnstoff |
| **125** | **L** | **I23** | 391,29599 | MS (>80%) | 1-(5-Brom-benzo[d]isoxazol-3-yl)-3-(4-dimethylamino-phenyl)-thioharnstoff |
| **126** | **G** | **I61** | 324,377993 | MS (>80%) | 1-(7-Fluor-benzo[d]isoxazol-3-yl)-3-(2-morpholin-4-yl-ethyl)-thioharnstoff |
| **127** | **G** | **I77** | 338,404993 | MS (>80%) | 1-(7-Fluor-benzo[d]isoxazol-3-yl)-3-(3-morpholin-4-yl-propyl)-thioharnstoff |
| **128** | **E** | **I78** | 327,405993 | MS (>80%) | 1-(4-Methoxy-benzo[d]isoxazol-3-yl)-3-(1-phenyl-ethyl)-thioharnstoff |
| **129** | **E** | **I9** | 347,823992 | MS (>80%) | 1-(4-Chlor-benzyl)-3-(4-methoxy-benzo[d]isoxazol-3-yl)-thioharnstoff |
| **130** | **E** | **I81** | 329,377993 | MS (>80%) | 1-(4-Methoxy-benzo[d]isoxazol-3-yl)-3-(2-methoxy-phenyl)-thioharnstoff |
| **131** | **L** | **I23** | 391,29599 | MS | 1-(5-Brom-benzo[d]isoxazol-3-yl)-3-(4-dimethylamino-phenyl)-thioharnstoff |
| **132** | **E** | **I75** | 363,822992 | MS (>80%) | 1-(5-Chlor-2-methoxy-phenyl)-3-(4-methoxy-benzo[d]isoxazol-3-yl)-thioharnstoff |
| **133** | **E** | **I74** | 401,79399 | MS (>80%) | 1-(4-Chlor-3-trifluormethyl-phenyl)-3-(4-methoxy-benzo[d]isoxazol-3-yl)-thioharnstoff |
| **134** | **E** | **I76** | 359,403992 | MS (>80%) | 1-(2,4-Dimethoxy-phenyl)-3-(4-methoxy-benzo[d]isoxazol-3-yl)-thioharnstoff |
| **135** | **F** | **I30** | 457,426989 | MS (>80%) | 1-[4-(2,2,2-Trifluor-ethoxy)-benzo[d]isoxazol-3-yl]-3-(3,4,5-trimethoxy-phenyl)-thioharnstoff |

### Pharmakologische Methoden:

### I. Fluoreszenzassay (FLIPR^{™}-Instrument) zur Identifizierung von agonistisch wirkenden Substanzen am Ionenkanal KCNQ2/3

Am Vortag des Versuchs erfolgt die Aussaat von KCNQ2/3-exprimierenden CHO-K1-Zellen bzw. CHO-K1-Leerstamm-Zellen auf beschichtete Poly-D-Lysin-Platten black/clear (Firma Falcon/BD; Bestellnr.: 356640) in einer Konzentration von 20000 Zellen/100µl MEM alpha, 50ml FCS, 5,5ml P/S/G Solution (100x) und anschließende Inkubation für 20 - 24 h im Brutschrank (37°C, 5% CO₂).

Am Versuchstag werden zunächst die HBSS/Hepes-Arbeitslösung und das FMP/HBSS/Hepes-Gemisch (Membranpotential Assay Kit Red (FMP), Bulk Format Cat Nr. R8123) nach folgendem Schema hergestellt:
1. HBSS/Hepes:
   1000 ml 1xHBSS-Puffer (Hank's Balanced Salt Solution (1X)(Gibco; Nr.14025) +10 ml HEPES (1M Stammlösung, pH 7.4; Hepes-Na- Salt (Firma Sigma; Nr. H7006-500g; Lagerung bei 4°C im Kühlschrank)
2. 1x FMP/HBSS/Hepes-Arbeitslösung: 5 ml der bei -20°C gelagerten FMP-Lösung (Herstellung s. unten; Auftauprozess bei Raumtemperatur) werden mit 6 ml HBSS/Hepes-Puffer versetzt.

Danach erfolgt eine Verdünnung der sich in Drugplates (Costar 96 well-Platten, Katnr. 3795) befindlichen Testsubstanzen (Stammlösung 2mM in 100% DMSO) durch Zugabe von HBSS/Hepes-Puffer auf eine Konzentration von 30 µM (3x konzentriert).

Die mit den Zellen bewachsenen Assayplatten werden einmalig mit 200 µl HBSS/Hepes gewaschen (Cellwash Washer, Firma Labsystems, Katnr. 5161550) und Restvolumina durch zweimaliges Ausklopfen der invertierten Platte entfernt. Anschließend werden die Zellplatten jeweils mit 100 µl der FMP/HBSS/Hepes Arbeitslösung versetzt und 1h im CO₂-Brutschrank bei 37°C und 5% CO₂ inkubiert.

Danach erfolgt die Vermessung am Instrument FLIPR III der Fa. Molecular Devices (96 Format; Benutzung des 540-590 nm Bandpass-Filters in der #2-Lokalisation). Im experimentellen Setup der FLIPR-Software ist Filter #2 zu wählen. Die Zellplatten werden alternierend mit den Substanzplatten in den Inputstacker gestellt. Von den Zellplatten wird jeweils vor Substanzzugabe ein Signaltest durchgeführt. Danach erfolgt durch die Pipettiereinheit des FLIPRs die Zugabe der Testsubstanzen (Additionsvolumen 50µl, 3x konzentriert, Endkonzentration 10 µM) in die Cavitäten der Zellplatten zu einem Gesamtvolumen von 150 µl/well.

Die Eckdaten des FLIPR Programmes sind:
Pipettiervolumina: 50 µl
Pipettierhöhe: 225 µl
Pipettiergeschwindigkeit : 50 µl/sec
Kamerakonfiguration: Exposure = 0.4; Gain = 50
Die Pipettenspitzen werden nach jedem Pipettiervorgang 3x mit 1% DMSO in Wasser gewaschen.

Die Zeiten der Messintervalle nach Zugabe der Testsubstanzen sehen folgendermaßen aus:
Sequenz 1: 60 x 1 sec
Sequenz 2: 15 x 20 sec
Gesamte Messzeit: 6 min/Platte

Die Auswertung erfolgt durch Differenzbildung zwischen Maximal- und Minimalwert. EC₅₀/IC₅₀ -Werte werden kalkuliert unter Benutzung der Graph Pad Prism-Software.

### Lösungen:

Herstellung der Membranpotential - Loading-Puffers (Membranpotential Assay Kit Red (FMP), Bulk Format Cat Nr. R8123) nach Angaben des Herstellers:
10 ml des 10x Assay-Puffers (Komponente B) werden mit 90 ml sterilem Wasser verdünnt und anschließend pH 7.4 mit 1.0N NaOH eingestellt (1 x Assay-Puffer).
1 Gefäß mit der FLIPR Membranpotentialassay-Komponente A wird in 100 ml 1x Assay-Puffer vollständig gelöst und für 10 min auf einem Magnetrührer durchmischt. Anschließend werden 5 ml-Aliquots bei -20°C gelagert (Haltbarkeit ca. 2 Wochen).

Ab einem Wert von -40 (und niedriger) ist eine agonistische Wirkung zu erkennen.

| **Beispiel** | **FLIPR** **ΔF** **%Hemmung** |
|---|---|
| **55** | -84 |
| **107** | -80 |
| **65** | -79 |
| **200** | -74 |
| **172** | -68 |
| **52** | -67,5 |
| **87** | -66,5 |
| **54** | -64 |
| **95** | -64 |
| **169** | -63,25 |
| **167** | -61 |
| **50** | -59,5 |
| **106** | -59 |
| **97** | -58,5 |
| **103** | -58 |
| **160** | -58 |
| **175** | -57,7 |
| **96** | -57 |
| **63** | -56 |
| **163** | -56 |
| **113** | -55 |
| **123** | -55 |
| **173** | -55 |
| **56** | -55 |
| **114** | -54 |
| **108** | -53,5 |
| **158** | -53 |
| **171** | -53 |
| **64** | -52 |
| **92** | -51 |
| **51** | -50 |
| **124** | -50 |
| **152** | -49 |
| **31** | -49 |
| **203** | -49 |
| **135** | -47,5 |
| **94** | -47 |
| **128** | -47 |
| **93** | -45,5 |
| **174** | -45,5 |
| **132** | -45 |
| **98** | -43,5 |
| **53** | -42,5 |
| **100** | -42 |
| **134** | -42 |
| **129** | -42 |
| **90** | -41,5 |
| **159** | -41 |
| **23** | -40 |
| **84** | -40 |
| **161** | -40 |

### II. Voltage-clamp Messungen

Um eine KCNQ2/3 agonistische Wirkung der Substanzen elektrophysiologisch zu konfirmieren wurden patch-clamp Messungen (Hamill et al., 1981) im voltage-clamp Modus an einer stabil transfizierten hKCNQ2/3 CHO-K1 Zelllinie durchgeführt. Nach Ausbildung des Gigaseals wurden die Zellen zunächst auf ein Haltepotential von -60 mV geklemmt. Im Anschluss wurden depolarisierende Spannungssprünge bis zu einem Potential von +20 mV appliziert (Inkrement: 20 mV, Dauer: 1 Sekunde), um die funktionelle Expression von KCNQ2/3 typischen Strömen zu bestätigen. Die Substanztestung erfolgte bei einem Potential von -40 mV. An jeder Zelle wurde zunächst die durch Retigabin (10 µM) induzierte Stromzunahme bei -40 mV als Positivkontrolle registriert. Nach komplettem Auswaschen des Retigabineffektes (Dauer: 80 s) wurde die Testsubstanz (10 µM) appliziert. Die durch die Testsubstanz induzierte Stromzunahme wurde auf den Retigabineffekt normiert und als relative Efficacy angegeben (s. u.).

Hamill OP, Marty A, Neher E, Sakmann B, Sigworth FJ.: Improved patch-clamp techniques for high-resolution current recording from cells and cell-free membrane patches. Pflugers Arch. 1981 Aug; 391(2):85-100.

| **Beispiel.** | **voltage clamp: relative efficacy** **gegenüber Retigabin [10**µ**M]** |
|---|---|
| **163** | 1,29 |
| **173** | 0,84 |
| **174** | 0,8 |
| **162** | 0,79 |
| **170** | 0,72 |
| **171** | 0,68 |
| **176** | 0,62 |
| **161** | 0,61 |
| **175** | 0,59 |
| **160** | 0,45 |
| **159** | 0,42 |
| **1** | 0,42 |
| **2** | 0,37 |
| **168** | 0,35 |
| **112** | 0,3 |
| **167** | 0,25 |
| **152** | 0,22 |
| **130** | 0,2 |

## Patentansprüche

1. Substituierte Benzo[d]isoxazol-3-yl-amin-Verbindungen der allgemeinen Formel I, 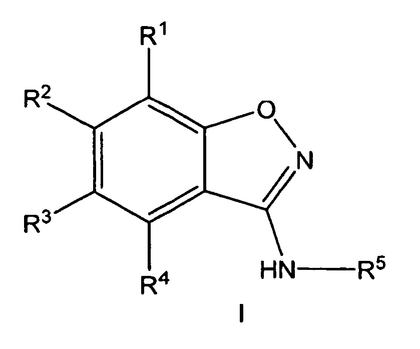 worin
R¹, R², R³ und R⁴, unabhängig voneinander, jeweils für
H; F; Cl; Br; I; -CN; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; C₁₋₁₀-Alkyl, C₂₋₁₀-Alkenyl, C₂₋₁₀-Alkinyl;
R⁵ für
-C(=S)NR²¹R²² oder (CHR⁶)ₙ-R²⁵
mit n = 1, 2 oder 3
wobei R⁶ für H, C₃₋₈-Cycloalkyl oder C₁₋₆-Alkyl steht
und R²⁵ Aryl oder Heteroaryl bedeutet
R⁷ und R⁸, unabhängig voneinander, jeweils für
H, -C(=O)R¹⁴ oder C₁₋₁₀-Alkyl stehen, oder
R⁷ und R⁸ zusammen mit dem sie verbindenden Stickstoffatom als Ringglied einen Rest bilden, der ausgewählt ist aus Morpholin, Piperidin oder Pyrrolidin;
R⁹, R¹⁰, R¹¹ und R¹⁶ unabhängig voneinander, jeweils für
H; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
R¹² und R¹³, unabhängig voneinander, jeweils für
H oder für einen C₁₋₁₀-Alkyl-Rest; oder
R¹² und R¹³ zusammen mit dem sie verbindenden Stickstoffatom als Ringglied einen Rest bilden, der ausgewählt ist aus Morpholin, Piperidin oder Pyrrolidin;
R¹⁴ für
-NR⁷R⁸; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
R¹⁵ und R¹⁷, unabhängig voneinander, jeweils für
C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
R²¹ und R²², unabhängig voneinander, jeweils für H, C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
wobei
die vorstehend genannten C₁₋₁₀-Alkyl-, C₂₋₁₀-Alkenyl- und C₂₋₁₀-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; COOH; COOC₁₋₄-Alkyl; -CN; -OH; -SH; -O-C₁₋₂-Alkyl; -S-C₁₋₂-Alkyl und -NH₂ substituiert sein können,
die vorstehend genannten C₃₋₈-Cycloalkyl-Reste jeweils ggf. mit 1, 2, 3, 4 oder 5 Substituenten unabhängig voneinander ausgewählt aus der Gruppe bestehend aus F; Cl; Br; -CN; -OH; -SH; -C₁₋₅-Alkyl; -O-C₁₋₂-Alkyl; -S-C₁₋₂-Alkyl und -NH₂ substituiert sein können,
die vorstehend genannten Heterocycloalkyl-Reste jeweils 4-, 5-, 6- oder 7-gliedrig sind, 1 oder 2 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; -CN; -OH; -SH; -C₁₋₅-Alkyl; -O-C₁₋₂-Alkyl; -S-C₁₋₂-Alkyl und -NH₂ substituiert sein können,
die vorstehend genannten Aryl-Reste jeweils für einen Phenyl-, Anthracenyl- oder Naphthyl-Rest stehen, der mit 1, 2, 3, 4 oder 5 Substituenten unabhängig voneinander ausgewählt aus der Gruppe bestehend aus F; Cl; Br; I; -CF₃;
-OCF₃; -SCF₃; C(=O)C₁₋₅-Alkyl; 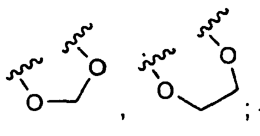 -NO₂; Cyclohexyl; -SF₅; -CN; -OH; -SH; -C₁₋₅-Alkyl; -O-C₁₋₅-Alkyl; -S-C₁₋₅-Alkyl; -C(=O)-OH; -O(C=O)C₁₋₂-Alkyl; -C(=O)-OC₁₋₅-Alkyl; -NH₂; -N(H)(C₁₋₅-Alkyl); -N(C₁₋₅-Alkyl)(C₁₋₅-Alkyl); -C(=O)NH₂; -C(=O)N(H)(C₁₋₅-Alkyl); -C(=O)N(C₁₋₅-Alkyl)(C₁₋₅-Alkyl); -S(=O)₂NH₂; -S(=O)₂N(H)(C₁₋₅-Alkyl); -S(=O)₂N(C₁₋₅-Alkyl)(C₁₋₅-Alkyl); -S(=O)₂-Phenyl; -S(=O)₂-C₁₋₅-Alkyl; Phenyl; Phenoxy, Benzyl; Benzyloxy; Thiophenyl (Thienyl), Furanyl und Pyridinyl substituiert sein können, die vorstehend genannten Heteroaryl-Reste jeweils 5- oder 6-gliedrig sind, 1, 2 oder 3 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; I; -CF₃; -OCF₃; -SCF₃; -SF₅; -CN; -OH; -SH; -C₁₋₅-Alkyl; -O-C₁₋₅-Alkyl; -S-C₁₋₅-Alkyl; -C(=O)-OH; -C(=O)-OC₁₋₅-Alkyl; -NH₂; -N(H)(C₁₋₅-Alkyl); -N(C₁₋₅-Alkyl)(C₁₋₅-Alkyl); -C(=O)NH₂; -C(=O)N(H)(C₁₋₅-Alkyl); -C(=O)N(C₁₋₅-Alkyl)(C₁₋₅-Alkyl); -S(=O)₂NH₂; -S(=O)₂N(H)(C₁₋₅-Alkyl); -S(=O)₂N(C₁₋₅-Alkyl)(C₁₋₅-Alkyl); -S(=O)₂-Phenyl; -S(=O)₂-C₁₋₅-Alkyl; Phenyl; Phenoxy, Benzyl; Thiophenyl (Thienyl), Furanyl und Pyridinyl substituiert sein können,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

2. Verbindungen gemäß Anspruch 1, worin
R¹, R², R³ und R⁴, unabhängig voneinander, jeweils für
H; F; Cl; Br; I; -CN; -NR⁷R⁸; -OR⁹; -SR¹⁰; C₁₋₄-Alkyl, C₂₋₄-Alkenyl oder C₂₋₄-Alkinyl;
R⁵ für
-C(=S)NR²¹R²²
oder
-(CHR⁶)ₙ-R²⁵
mit n = 1, 2 oder 3
wobei R⁶ für H oder C₁₋₆-Alkyl steht
und R²⁵ Aryl oder Heteroaryl bedeutet
R⁷ und R⁸, unabhängig voneinander, jeweils für
H, -C(=O)R¹⁵ oder C₁₋₄-Alkyl stehen, oder
R⁷ und R⁸ zusammen mit dem sie verbindenden Stickstoffatom als Ringglied einen Rest bilden, der ausgewählt ist aus Morpholin, Piperidin oder Pyrrolidin;
R⁹, R¹⁰ unabhängig voneinander, jeweils für
H; C₁₋₄-Alkyl, C₂₋₄-Alkenyl, C₂₋₄-Alkinyl; C₃₋₈-Cycloalkyl; -(C_{1,2 oder 3}-Alkylen)-C₃₋₈-Cycloalkyl; Heterocycloalkyl; -(C_{1,2 oder 3}-Alkylen)-Heterocycloalkyl; Aryl; Heteroaryl; -(C_{1,2 oder 3}-Alkylen)-Aryl; -(C_{1,2 oder 3}-Alkylen)-Heteroaryl;
R²¹ und R²², unabhängig voneinander, jeweils für
H; C₁₋₁₀-Alkyl, C₂₋₁₀-Alkenyl, C₂₋₁₀-Alkinyl; C₃₋₈-Cycloalkyl; -(C_{1, 2 oder 3}--Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl; -(C_{1, 2 oder 3}-Alkylen)-Heteroaryl;
wobei
die vorstehend genannten C₁₋₁₀-Alkyl-, C₂₋₁₀-Alkenyl- und C₂₋₁₀-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; -CN; COOH; COOC₁₋₄-Alkyl; -OH; -SH; -O-C₁₋₂-Alkyl; -S-C₁₋₂-Alkyl und -NH₂ substituiert sein können,
die vorstehend genannten C₁₋₄-Alkyl-, C₂₋₄-Alkenyl- und C₂₋₄-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; -CN; -OH; -OCH₃ und -NH₂ substituiert sein können,
die vorstehend genannten C₃₋₈-Cycloalkyl-Reste jeweils ggf. mit 1, 2, 3, 4 oder 5 Substituenten unabhängig voneinander ausgewählt aus der Gruppe bestehend aus F; Cl; Br; -CN; -OH; -CH₃; -C₂H₅; -OCH₃; und -NH₂ substituiert sein können,
die vorstehend genannten Heterocycloalkyl-Reste jeweils 4-, 5-, 6- oder 7-gliedrig sind, 1 oder 2 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; -CN; -OH; -CH₃; -C₂H₅; -OCH₃; und -NH₂ substituiert sein können,
die vorstehend genannten Aryl-Reste jeweils für einen Phenyl-, Anthracenyl- oder Naphthyl-Rest stehen, der mit 1, 2, 3, 4 oder 5 Substituenten unabhängig voneinander ausgewählt aus der Gruppe bestehend aus F; Cl; Br; -CF₃; -OCF₃; -SCF₃; 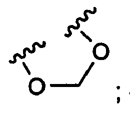 -NO₂, -C(=O)C₁₋₂-Alkyl; Cyclohexyl; -O(C=O)C₁₋₂-Alkyl;
-SF₅; -CN; -OH; Methyl; Ethyl; n-Propyl; iso-Propyl; n-Butyl; iso-Butyl; sec-Butyl; tert.-Butyl; Methoxy; Ethoxy; -NH₂; -N(CH₃)₂; -N(C₂H₅)₂; Phenyl; Benzyloxy; Phenoxy, Benzyl; Thiophenyl (Thienyl), Furanyl und Pyridinyl substituiert sein können;
die vorstehend genannten Heteroaryl-Reste jeweils für einen Furanyl-, Thienyl- (Thiophenyl-) oder Pyridinyl-Rest stehen und ggf. mit 1, 2, 3, 4 oder 5 Substituenten unabhängig voneinander ausgewählt aus der Gruppe bestehend F; Cl; Br; -CF₃; -OCF₃; -SCF₃; -SF₅; -CN; -OH; Methyl; Ethyl; n-Propyl; iso-Propyl; n-Butyl; iso-Butyl; sec-Butyl; tert.-Butyl; Methoxy; Ethoxy; - NH₂; -N(CH₃)₂; -N(C₂H₅)₂; Phenyl; Phenoxy, Benzyl; Thiophenyl (Thienyl), Furanyl und Pyridinyl substituiert sein können,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

3. Verbindungen gemäß einem der Ansprüche 1-2, worin R⁵ -C(=S)NR²¹R²² bedeutet.

4. Verbindungen gemäß einem der Ansprüche 1-2, worin R⁵ -(CHR⁶)ₙ-R²⁵ bedeutet.

5. Verbindungen gemäß Anspruch 3, worin R²¹ für H und R²² für Benzyl, Phenyl, Pyridyl, Naphthyl oder Phenethyl, unsubstituiert oder einfach oder mehrfach substituiert mit Methyl, Ethyl, Isopropyl, Cl, F, Br, NO₂, Acetyl, CN, COOH, COOC₁₋₄-Alkyl, Methoxy, Ethoxy, N(CH₃)₂, N(C₂H₅)₂, 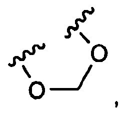 CF₃, SCH₃;
C₁₋₁₀-Alkyl, verzweigt oder unverzweigt, gesättigt oder ungesättigt, unsubstituiert oder substituiert mit OCH₃, COOCH₃ oder COOC₂H₅; C₃₋₆-Cycloalkyl, wobei die Cycloalkylgruppe über eine CH2-Gruppe verknüpft sein kann; C₅₋₆-Heterocycloalkyl wobei die Heterocycloalkylgruppe über eine CH₂-Gruppe verknüpft sein kann; steht

6. Verbindungen gemäß Anspruch 5, worin R²¹ für H und R²² für Benzyl, Phenyl, 2-Methylphenyl, Phenethyl, 2-lsoropylphenyl, 2-Chlorphenyl, 4-Fluorbenzyl, 1-(4-Fluorphenyl)ethyl, 4-Chlorbenzyl, 4-Chlor-phenethyl, 4-Nitrophenyl, 4-Acetyl-phenyl, 3-Carboxyphenyl, 3-Methylbenzoat, 4-Ethylbenzoat, 2,6-Diethylphenyl, 3-Chlor-4-methyl-phenyl, 2-Methoxyethyl, 3-Methoxypropyl, Cyclopentyl, Cyclohexyl, 3-Pyridyl, 4-Dimethylaminophenyl, 4-Diethylaminophenyl, CH₂COOCH₃, CH(CH₃)COOC₂H₅, CH(CH₃)CH₂COOC₂H₅, Cyclohexylmethyl, 4-Ethoxyphenyl, 3,4-Dimethoxyphenyl, 1-Naphthyl, 3,4,5-Trimethoxyphenyl, 2,3,4,5,6-Pentafluorphenyl, Benzodioxol, 4-Fluorphenyl, Methyl, Ethyl, Propyl, Isopropyl, tert.-Butyl, Allyl, 2-Methylprop-2-enyl" 2-Nitrophenyl, 3-Trifluormethylphenyl, 2-Trifluormethylphenyl, 4-Trifluormethylphenyl, Cyclopropyl, 2-Methylsulfanylphenyl, 3-Methylsulfanylphenyl, 4-Methylsulfanylphenyl, 3,5-Dimethylphenyl, Ethylmorpholin, ((4-Propyl)-cyclohexyl)phenyl, 4-Brom-2-trifluormethyl-phenyl, n-Octyl, n-Nonanyl, Tetrahydrofurylmethyl, 2-Ethylphenyl, 4-Cyanophenyl, 3-Cyanophenyl, 2,6-Diisopropylphenyl, n-Pentyl, n-Hexyl, sec-Butyl, Propylmorpholin, 5-Chlor-2-methoxyphenyl, 4-Chlor-3-trifluormethylphenyl, 3-Chlorphenyl, 1-Phenylethyl, CH(CH₃)COOCH₃, 2-Methoxyphenyl, 3-Methoxyphenyl, 4-Methoxyphenyl, CH₂CH₂COOC₂H₅, 2-Methylbenzoat, 4-Methylbenzoat, 2-Ethylbenzoat, 2-Fluorphenyl, 2-Methoxy-5-chlorphenyl oder 2,4-Dimethoxyphenyl steht.

7. Verbindungen gemäß Anspruch 4, worin R⁶ H bedeutet.

8. Verbindungen gemäß Anspruch 4, worin R⁶ CH₃ bedeutet.

9. Verbindungen gemäß einem der Ansprüche 4, 7 oder 8, worin R²⁵ Phenyl, Pyridyl, Thienyl oder Furyl bedeutet, jeweils unsubstituiert oder einfach oder mehrfach substituiert mit CF₃, SCF₃, C₁₋₄-Alkyl, Cl, NO₂, O-Acetyl, OCH₃, F, O-Phenyl, OCF₃, Br, O-Benzyl, O-Allyl, Phenyl, I, CN oder OH.

10. Verbindungen nach Anspruch 9, worin R²⁵ für 4-Trifluormethylphenyl, 4-SCF₃-Phenyl, 2-Methylphenyl, Phenyl, Anthracenyl, 4-Cl-Phenyl, 4-OCF₃-Phenyl, 4-n-Butylphenyl, 3(3-CF₃-Phenoxy)-phenyl, 4-OCHF₂-Phenyl, 3,5-Dimethylphenyl, 3-Brom-4-methoxyphenyl, 4-Benzyloxy-3,5-dimethylphenyl, 3-Nitrophenyl, 3-Methoxy-4-(acetylmethyl)-phenyl, 2,4,5-Trimethoxyphenyl, 4-Ethylphenyl, 3,4-Dichlorphenyl, 2,3,5-Trifluorphenyl, 4-Phenoxyphenyl, 3-Chlor-4-fluorphenyl, 3-Benzyloxy-phenyl, 3-Brom-4,5-dimethoxyphenyl, 3-Fluor-2-methylphenyl, 2-Chlor-3-trifluormethylphenyl, 3-Chlor-2-fluor-5-trifluormethylphenyl, 2-Fluor-4-trifluormethylphenyl, 4-(Allyloxy)-phenyl, 2-(Benzyloxy)-4,5-dimethoxyphenyl, 2-Phenyl-phenyl, 2,3,4-Trifluorphenyl, 2-Fluor-5-trifluorphenyl, 4-Methoxy-3-methylphenyl, 2-Fluor-3-chlorphenyl, 3,4-Difluorphenyl, 2,6-Dichlorphenyl, 3-lodphenyl, 3-lod-4,5-dimethoxyphenyl, 2-Cyanophenyll, 4-Hydroxyphenyl, 3,4-Dimethylphenyl oder 3-OCF₃-Phenyl steht.

11. Verbindungen gemäß Anspruch 1 aus der Gruppe bestehend aus
Benzo[d]isoxazol-3-yl-[1-(4-trifluormethyl-phenyl)-ethyl]-amin
Benzo[d]isoxazol-3-yl-[1-(4-trifluormethylsulfanyl-phenyl)-ethyl]-amin
1-Benzo[d]isoxazol-3-yl-3-benzyl-thioharnstoff
1-Benzo[d]isoxazol-3-yl-3-phenyl-thioharnstoff
1-Benzo[d]isoxazol-3-yl-3-o-tolyl-thioharnstoff
1-Benzo[d]isoxazol-3-yl-3-phenethyl-thioharnstoff
1-Benzo[d]isoxazol-3-yl-3-(2-isopropyl-phenyl)-thioharnstoff
1-Benzo[d]isoxazol-3-yl-3-(2-chlor-phenyl)-thioharnstoff
1-Benzo[d]isoxazol-3-yl-3-(4-fluor-benzyl)-thioharnstoff
1-Benzo[d]isoxazol-3-yl-3-[1-(4-fluor-phenyl)-ethyl]-thioharnstoff
1-Benzo[d]isoxazol-3-yl-3-(4-chlor-benzyl)-thioharnstoff
1-Benzo[d]isoxazol-3-yl-3-[2-(4-chlor-phenyl)-ethyl]-thioharnstoff
1-Benzo[d]isoxazol-3-yl-3-(4-nitro-phenyl)-thioharnstoff
1-(4-Acetyl-phenyl)-3-benzo[d]isoxazol-3-yl-thioharnstoff
3-(3-Benzo[d]isoxazol-3-yl-thioureido)-benzoesäure
3-(3-Benzo[d]isoxazol-3-yl-thioureido)-benzoesäuremethylester
4-(3-Benzo[d]isoxazol-3-yl-thioureido)-benzoesäureethylester
1-Benzo[d]isoxazol-3-yl-3-(2,6-d iethyl-phenyl)-thioharnstoff
1-Benzo[d]isoxazol-3-yl-3-(3-chlor-4-methyl-phenyl)-thioharnstoff
1-Benzo[d]isoxazol-3-yl-3-(2-methoxy-ethyl)-thioharnstoff
1-Benzo[d]isoxazol-3-yl-3-(3-methoxy-propyl)-thioharnstoff
1-Benzo[d]isoxazol-3-yl-3-cyclopentyl-thioharnstoff
1-Benzo[d]isoxazol-3-yl-3-cyclohexyl-thioharnstoff
1-Benzo[d]isoxazol-3-yl-3-pyridin-3-yl-thioharnstoff
1-Benzo[d]isoxazol-3-yl-3-(4-dimethylamino-phenyl)-thioharnstoff
1-Benzo[d]isoxazol-3-yl-3-(4-diethylamino-phenyl)-thioharnstoff
(3-Benzo[d]isoxazol-3-yl-thioureido)-essigsäuremethylester
2-(3-Benzo[d]isoxazol-3-yl-thioureido)-propionsäureethylester
3-(3-Benzo[d]isoxazol-3-yl-thioureido)-buttersäureethylester
1-Benzo[d]isoxazol-3-yl-3-cyclohexylmethyl-thioharnstoff
1-Benzo[d]isoxazol-3-yl-3-(4-ethoxy-phenyl)-thioharnstoff
1-Benzo[d]isoxazol-3-yl-3-(3,4-dimethoxy-phenyl)-thioharnstoff
1-Benzo[d]isoxazol-3-yl-3-(3,4,5-trimethoxy-phenyl)-thioharnstoff
1-Benzo[d]isoxazol-3-yl-3-pentafluorphenyl-thioharnstoff
1-Benzo[d]isoxazol-3-yl-3-naphthalen-1-yl-thioharnstoff
1-Benzo[1,3]dioxol-5-ylmethyl-3-benzo[d]isoxazol-3-yl-thioharnstoff
1-Benzo[d]isoxazol-3-yl-3-(4-fluor-phenyl)-thiohamstoff
1-Benzo[d]isoxazol-3-yl-3-methyl-thioharnstoff
1-Benzo[d]isoxazol-3-yl-3-ethyl-thioharnstoff
1-Benzo[d]isoxazol-3-yl-3-propyl-thioharnstoff ,
1-Benzo[d]isoxazol-3-yl-3-isopropyl-thioharnstoff
1-Benzo[d]isoxazol-3-yl-3-tert-butyl-thioharnstoff
1-Allyl-3-benzo[d]isoxazol-3-yl-thioharnstoff
1-Benzo[d]isoxazol-3-yl-3-(2-methyl-allyl)-thioharnstoff
1-Benzo[d]isoxazol-3-yl-3-(2-nitro-phenyl)-thioharnstoff
1-Benzo[d]isoxazol-3-yl-3-(2-trifluormethyl-phenyl)-thioharnstoff
1-Benzo[d]isoxazol-3-yl-3-(3-trifluormethyl-phenyl)-thioharnstoff
1-Benzo[d]isoxazol-3-yl-3-(4-trifluormethyl-phenyl)-thioharnstoff
1-Benzo[d]isoxazol-3-yl-3-cyclopropyl-thioharnstoff
2-[3-(4-Fluor-benzo[d]isoxazol-3-yl)-thioureido]-propionsäuremethylester
1-(4-Chlor-benzo[d]isoxazol-3-yl)-3-o-tolyl-thioharnstoff
1-Benzyl-3-(4-chlor-benzo[d]isoxazol-3-yl)-thioharnstoff
1-(4-Chlor-benzo[d]isoxazol-3-yl)-3-(1-phenyl-ethyl)-thioharnstoff
1-(4-Dimethylamino-benzo[d]isoxazol-3-yl)-3-isobutyl-thioharnstoff
1-(4-Dimethylamino-benzo[d]isoxazol-3-yl)-3-p-tolyl-thioharnstoff
1-(3-Chlor-phenyl)-3-(4-dimethylamino-benzo[d]isoxazol-3-yl)-thioharnstoff
1-(4-Dimethylamino-benzo[d]isoxazol-3-yl)-3-(3-methoxy-phenyl)-thioharnstoff
1-(4-Dimethylamino-benzo[d]isoxazol-3-yl)-3-(2-methylsulfanyl-phenyl)-thioharnstoff
1-(4-Dimethylamino-benzo[d]isoxazol-3-yl)-3-(3-methylsulfanyl-phenyl)-thioharnstoff
1-(4-Dimethylamino-benzo[d]isoxazol-3-yl)-3-(4-methylsulfanyl-phenyl)-thioharnstoff
1-(4-Dimethylamino-benzo[d]isoxazol-3-yl)-3-(2-methoxy-phenyl)-thioharnstoff
1-(4-Dimethylamino-benzo[d]isoxazol-3-yl)-3-(4-methoxy-phenyl)-thioharnstoff
1-(4-Dimethylamino-benzo[d]isoxazol-3-yl)-3-(3,5-dimethyl-phenyl)-thiohamstoff
1-Benzyl-3-(4-dimethylamino-benzo[d]isoxazol-3-yl)-thioharnstoff
1-(4-Dimethylamino-benzo[d]isoxazol-3-yl)-3-(3-methoxy-propyl)-thioharnstoff
3-[3-(4-Dimethylamino-benzo[d]isoxazol-3-yl)-thioureido]-propionsäurethylester
2-[3-(4-Dimethylamino-benzo[d]isoxazol-3-yl)-thioureido]-propionsäurethylester
3-[3-(4-Dimethylamino-benzo[d]isoxazol-3-yl)-thioureido]-buttersäureethylester
3-[3-(4-Dimethylamino-benzo[d]isoxazol-3-yl)-thioureido]-benzoesäure
1-(4-Dimethylamino-benzo[d]isoxazol-3-yl)-3-(4-ethoxy-phenyl)-thioharnstoff
2-[3-(4-Dimethylamino-benzo[d]isoxazol-3-yl)-thioureido]-benzoesäuremethylester
3-[3-(4-Dimethylamino-benzo[d]isoxazol-3-yl)-thioureido]-benzoesäuremethylester
4-[3-(4-Dimethylamino-benzo[d]isoxazol-3-yl)-thioureido]-benzoesäuremethylester
2-[3-(4-Dimethylamino-benzo[d]isoxazol-3-yl)-thioureido]-benzoesäureethylester
4-[3-(4-Dimethylamino-benzo[d]isoxazol-3-yL)-thioureido]-benzoesäureethylester
1-(4-Acetyl-phenyl)-3-(4-dimethylamino-benzo[d]isoxazol-3-yl)-thioharnstoff
1-(2-Chlor-phenyl)-3-(4-methoxy-benzo[d]isoxazol-3-yl)-thioharnstoff
1-(4-Diethylamino-phenyl)-3-(4-methoxy-benzo[d]isoxazol-3-yl)-thioharnstoff
1-(4-Methoxy-benzo[d]isoxazol-3-yl)-3-(2-morpholin-4-yl-ethyl)-thioharnstoff
2-{3-[4-(2,2,2-trifluor-ethoxy)-benzo[d]isoxazol-3-yl]-thioureido}-propionsäuremethylester
1-Benzo[1,3]dioxol-5-ylmethyl-3-[4-(2,2,2-trifluor-ethoxy)-benzo[d]isoxazol-3-yl]-thioharnstoff
1-[4-(4-Propyl-cyclohexyl)-phenyl]-3-[4-(2,2,2-trifluor-ethoxy)-benzo[d]isoxazol-3-yl]-thioharnstoff
1-(4-Brom-2-trifluormethyl-phenyl)-3-[4-(2,2,2-trifluor-ethoxy)-benzo[d]isoxazol-3-yl]-thioharnstoff
1-(4-Methoxy-phenyl)-3-[4-(2,2,2-trifluor-ethoxy)-benzo[d]isoxazol-3-yl]-thioharnstoff
3-{3-[4-(2,2,2-trifluor-ethoxy)-benzo[d]isoxazol-3-yl]-thioureido}-propionsäureethylester
1-[4-(4-Methyl-benzyloxy)-benzo[d]isoxazol-3-yl]-3-octyl-thioharnstoff
1-[4-(4-Methyl-benzyloxy)-benzo[d]isoxazol-3-yl]-3-nonyl-thioharnstoff
1-Cyclopropyl-3-[4-(4-methyl-benzyloxy)-benzo[d]isoxazol-3-yl]-thioharnstoff
1-Cyclopentyl-3-[4-(4-methyl-benzyloxy)-benzo[d]isoxazol-3-yl]-thioharnstoff
1-Cyclohexyl-3-[4-(4-methyl-benzyloxy)-benzo[d]isoxazol-3-yl]-thioharnstoff
1-Cyclohexylmethyl-3-[4-(4-methyl-benzyloxy)-benzo[d]isoxazol-3-yl]-thioharnstoff
1-(4-Dimethylamino-phenyl)-3-[4-(2,2,2-trifluor-ethoxy)-benzo[d]isoxazol-3-yl]-thioharnstoff
1-Allyl-3-(5-methyl-benzo[d]isoxazol-3-yl)-thioharnstoff
[3-(5-Methyl-benzo[d]isoxazol-3-yl)-thioureido]-essigsäuremethylester
1-(2-Isopropyl-phenyl)-3-(5-methyl-benzo[d]isoxazol-3-yl)-thioharnstoff
1-(5-Methyl-benzo[d]isoxazol-3-yl)-3-(4-trifluormethyl-phenyl)-thioharnstoff
2-[3-(5-Fluor-benzo[d]isoxazol-3-yl)-thioureido]-propionsäuremethylester
1-(5-Fluor-benzo[d]isoxazol-3-yl)-3-(tetrahydro-furan-2-ylmethyl)-thioharnstoff
1-(5-Brom-benzo[d]isoxazol-3-yl)-3-(2-Fluor-phenyl)-thioharnstoff
1-(5-Brom-benzo[d]isoxazol-3-yl)-3-(2-ethyl-phenyl)-thioharnstoff
1-(6-Chlor-benzo[d]isoxazol-3-yl)-3-(4-Fluor-phenyl)-thioharnstoff
1-(6-Chlor-benzo[d]isoxazol-3-yl)-3-(2-Fluor-phenyl)-thioharnstotf
1-(6-Chlor-benzo[d]isoxazol-3-yl)-3-cyclopentyl-thioharnstoff
1-(6-Chlor-benzo[d]isoxazol-3-yl)-3-(4-cyano-phenyl)-thioharnstoff
3-[3-(6-Chlor-benzo[d]isoxazol-3-yl)-thioureido]-benzoesäure
1-(6-Chlor-benzo[d]isoxazol-3-yl)-3-(4-methoxy-phenyl)-thioharnstoff
1-(6-Chlor-benzo[d]isoxazol-3-yl)-3-(3-methoxy-phenyl)-thioharnstoff
1-(6-Brom-benzo[d]isoxazol-3-yl)-3-(3,4-dimethoxy-phenyl)-thioharnstoff
1-(6-Brom-benzo[d]isoxazol-3-yl)-3-naphthalen-1-yl-thioharnstoff
1-Benzo[1,3]dioxol-5-ylmethyl-3-(6-brom-benzo[d]isoxazol-3-yl)-thioharnstoff
1-(6-Fluor-benzo[d]isoxazol-3-yl)-3-(2-methylsulfanyl-phenyl)-thioharnstoff
1-(3-Cyano-phenyl)-3-(6-fluor-benzo[d]isoxazol-3-yl)-thioharnstoff
1-(2-Chlor-6-methyl-phenyl)-3-(6-fluor-benzo[d]isoxazol-3-yl)-thioharnstoff
1-(2,6-Diisopropyl-phenyl)-3-(6-fluor-benzo[d]isoxazol-3-yl)-thioharnstoff
1-(7-Fluor-benzo[d]isoxazol-3-yl)-3-methyl-thiohamstoff
1-Ethyl-3-(7-fluor-benzo[d]isoxazol-3-yl)-thioharnstoff
1-(7-Fluor-benzo[d]isoxazol-3-yl)-3-propyl-thioharnstoff
1-(7-Fluor-benzo[d]isoxazol-3-yl)-3-pentyl-thioharnstoff
1-(7-Fluor-benzo[d]isoxazol-3-yl)-3-hexyl-thioharnstoff
1-(7-Fluor-benzo[d]isoxazol-3-yl)-3-octyl-thioharnstoff
1-(7-Fluor-benzo[d]isoxazol-3-yl)-3-nonyl-thioharnstoff
1-(7-Fluor-benzo[d]isoxazol-3-yl)-3-isobutyl-thioharnstofl
1-Allyl-3-(7-fluor-benzo[d]isoxazol-3-yl)-thioharnstoff
1-(7-Fluor-benzo[d]isoxazol-3-yl)-3-p-tolyl-thioharnstoff
1-(5-Brom-benzo[d]isoxazol-3-yl)-3-(4-dimethylamino-phenyl)-thioharnstoff
1-(7-Fluor-benzo[d]isoxazol-3-yl)-3-(2-morpholin-4-yl-ethyl)-thioharnstoff
1-(7-Fluor-benzo[d]isoxazol-3-yl)-3-(3-morpholin-4-yl-propyl)-thioharnstoff
1-(4-Methoxy-benzo[d]isoxazol-3-yl)-3-(1-phenyl-ethyl)-thioharnstoff
1-(4-Chlor-benzyl)-3-(4-methoxy-benzo[d]isoxazol-3-yl)-thioharnstoff
1-(4-Methoxy-benzo[d]isoxazol-3-yl)-3-(2-methoxy-phenyl)-thioharnstoff
1-(5-Brorn-benzo[d]isoxazo!-3-yl)-3-(4-dimethylarnino-phenyl)-thioharnstoff
1-(5-Chlor-2-methoxy-phenyl)-3-(4-methoxy-benzo[d]isoxazol-3-yl)-thioharnstoff
1-(4-Chlor-3-trifluormethyl-phenyl)-3-(4-methoxy-benzo[d]isoxazol-3-yl)-thioharnstoff
1-(2,4-Dimethoxy-phenyl)-3-(4-methoxy-benzo[d]isoxazol-3-yl)-thioharnstoff
1-[4-(2,2,2-Trifluor-ethoxy)-benzo[d]isoxazol-3-yl]-3-(3,4,5-trimethoxy-phenyl)-thioharnstoff
Benzo[d]isoxazol-3-yl-(3-methyl-butyl)-amin
(5-Fluor-benzo[d]isoxazol-3-yl)-(2-methyl-benzyl)-amin
N4,N4-Dimethyl-N3-(3-phenyl-propyl)-benzo[d]isoxazol-3,4-diamin
N3-Butyl-N4,N4-dimethyl-benzo[d]isoxazol-3,4-diamin
Anthracen-9-ylmethyl-(4-methoxy-benzo[d]isoxazol-3-yl)-amin
(4-Chlor-benzyl)-(4-methoxy-benzo[d]isoxazol-3-yl)-amin
(6-Fluor-benzo[d]isoxazol-3-yl)-(3-nitro-benzyl)-amin
Essigsäure-4-[(6-chlor-benzo[d]isoxazol-3-ylamino)-methyl]-2-methoxyphenylester
Essigsäure-4-[(6-brom-benzo[d]isoxazol-3-ylamino)-methyl]-2-methoxyphenylester
Benzo[d]isoxazol-3-yl-(3,4-dichlor-benzyl)-amin
Benzo[d]isoxazol-3-yl-(2,4,5-trimethoxy-benzyl)-amin
Benzo[d]isoxazol-3-yl-(4-ethyl-benzyl)-amin
(6-Chlor-benzo[d]isoxazol-3-yl)-(3,4-dichlor-benzyl)-amin
Benzo[d]isoxazol-3-yl-(2,3,5-trifluor-benzyl)-amin
(6-Chlor-benzo[d]isoxazol-3-yl)-(4-phenoxy-benzyl)-amin
(3-Chlor-4-Fluor-benzyl)-(7-fluor-benzo[d]isoxazol-3-yl)-amin
Benzo[d]isoxazol-3-yl-(4-trifluormethyl-benzyl)-amin
(7-Fluor-benzo[d]isoxazol-3-yl)-(2-methyl-pentyl)-amin
N4,N4-Dimethyl-N3-(2,3,4-trifluor-benzyl)-benzo[d]isoxazol-3,4-diamin
N3-(2-Fluor-5-trifluormethyl-benzyl)-N4,N4-dimethyl-benzo[d]isoxazol-3,4-diamin
N3-(4-Methoxy-3-methyl-benzyl)-benzo[d]isoxazol-3,4-diamin
N3-(4-Methoxy-3-methyl-benzyl)-benzo[d]isoxazol-3,4-diamin
Benzo[d]isoxazol-3-yl-(4-trifluormethoxy-benzyl)-amin
(5-Fluor-benzo[d]isoxazol-3-yl)-(4-trifluormethoxy-benzyl)-amin
Benzo[d]isoxazol-3-yl-(4-trifluormethylsulfanyl-benzyl)-amin
(4-Butyl-benzyl)-(6-chlor-benzo[d]isoxazol-3-yl)-amin
(5-Fluor-benzo[d]isoxazol-3-yl)-(4-trifluormethylsulfanyl-benzyl)-amin
Benzo[d]isoxazol-3-yl-(2-fluor-4-trifluormethyl-benzyl)-amin
(7-Fluor-benzo[d]isoxazol-3-yl)-(4-trifluormethoxy-benzyl)-amin
(7-Fluor-benzo[d]isoxazol-3-yl)-[3-(3-trifluormethyl-phenoxy)-benzyl]-amin
(4-Difluormethoxy-benzyl)-(4-methoxy-benzo[d]isoxazol-3-yl)-amin
(3,5-Dimethyl-benzyl)-(7-fluor-benzo[d]isoxazol-3-yl)-amin
(3-Brom-4-methoxy-benzyl)-(6-fluor-benzo[d]isoxazol-3-yl)-amin
(3,5-Dimethyl-benzyl)-(6-fluor-benzo[d]isoxazol-3-yl)-amin
(4-Benzyloxy-3,5-dimethyl-benzyl)-(6-fluor-benzo[d]isoxazol-3-yl)-amin
(4-Butyl-benzyl)-(6-fluor-benzo[d]isoxazol-3-yl)-amin
(6-Fluor-benzo[d]isoxazol-3-yl)-(4-trifluormethylsulfanyl-benzyl)-amin
(3-Benzyloxy-benzyl)-(6-fluor-benzo[d]isoxazol-3-yl)-amin
N3-(3,5-Dimethyl-benzyl)-benzo[d]isoxazol-3,4-diamin
N3-(4-Butyl-benzyl)-benzo[d]isoxazol-3,4-diamin
(5-Brom-benzo[d]isoxazol-3-yl)-(4-trifluormethylsulfanyl-benzyl)-amin
(3-Brom-4,5-dimethoxy-benzyl)-(7-fluor-benzo[d]isoxazol-3-yl)-amin
(7-Fluor-benzo[d]isoxazol-3-yl)-(2-fluor-4-trifluormethyl-benzyl)-amin
N3-(3-Fluor-2-methyl-benzyl)-N4,N4-dimethyl-benzo[d]isoxazol-3,4-diamin
N3-(2-Chlor-3-trifluormethyl-benzyl)-N4,N4-dimethyl-benzo[d]isoxazol-3,4-diamin
N3-(3-Chlor-2-fluor-5-trifluormethyl-benzyl)-N4,N4-dimethyl-benzo[d]isoxazol-3,4-diamin
(6-Fluor-benzo[d]isoxazol-3-yl)-(2-fluor-4-trifluormethyl-benzyl)-amin
(4-Allyloxy-benzyl)-(6-fluor-benzo[d]isoxazol-3-yl)-amin
Benzo[d]isoxazol-3-yl-(2-benzyloxy-4,5-dimethoxy-benzyl)-amin
(2-Benzyloxy-4,5-dimethoxy-benzyl)-(6-chlor-benzo[d]isoxazol-3-yl)-amin
N3-(2-Benzyloxy-4,5-dimethoxy-benzyl)-N4,N4-dimethyl-benzo[d]isoxazol-3,4-diamin
N3-Biphenyl-2-ylmethyl-N4,N4-dimethyl-benzo[d]isoxazol-3,4-diamin
(6-Fluor-benzo[d]isoxazol-3-yl)-(3-iodo-benzyl)-amin
(2-Benzyloxy-4,5-dimethoxy-benzyl)-(4-methoxy-benzo[d]isoxazol-3-yl)-amin
(4-Fluor-benzo[d]isoxazol-3-yl)-(3-iodo-4,5-dimethoxy-benzyl)-amin
2-[(5-Methyl-benzo[d]isoxazol-3-ylamino)-methyl]-benzonitril
Butyl-[4-(2,2,2-trifluor-ethoxy)-benzo[d]isoxazol-3-yl]-amin
(3-Brom-4,5-dimethoxy-benzyl)-(5-methyl-benzo[d]isoxazol-3-yl)-amin
4-[(4-Chlor-benzo[d]isoxazol-3-ylamino)-methyl]-phenol
(6-Chlor-benzo[d]isoxazol-3-yt)-(3,4-dimethyl-benzyl)-amin
(4-Chlor-benzo[d]isoxazol-3-yl)-(3-chlor-2-fluor-benzyl)-amin
(3,4-Difluor-benzyl)-(5-fluor-benzo[d]isoxazol-3-yl)-amin
(6-Brom-benzo[d]isoxazol-3-yl)-(2,6-dichlor-benzyl)-amin
(7-Fluor-benzo[d]isoxazol-3-yl)-(3-trifluormethoxy-benzyl)-amin
sowie jeweils deren entsprechende Salze, insbesondere deren Hydrochlorid-Additionssalze, und ggf. jeweils deren entsprechende Solvate.

12. Verfahren zur Herstellung der erfindungsgemäßen substituierten Benzo[d]isoxazol-3-yl-amin-Verbindungen, gemäß dem eine ggf. substituierte 2-Fluor-benzonitril-verbindung der allgemeinen Formel II, 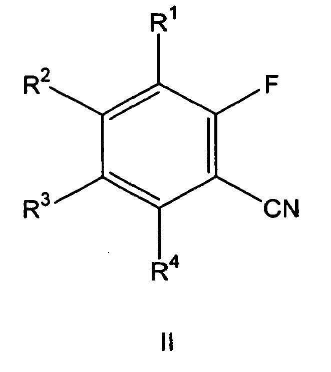 worin die Reste R¹, R², R³ und R⁴ die vorstehend angegebene Bedeutung haben, in einem Reaktionsmedium, vorzugsweise ausgewählt aus der Gruppe bestehend aus Diethylether, Tetrahydrofuran, Acetonitril, Dimethylsulfoxid, Dimethylformamid und Dichlormethan, in Gegenwart einer Base, vorzugsweise in Gegenwart wenigstens eines Alkalimetallalkoholatsalzes, besonders bevorzugt in Gegenwart eines Alkalimetallalkoholatsalzes ausgewählt aus der Gruppe bestehend aus Kaliummethanolat, Natriummethanolat, Kalium-tert-butylat und Natrium-tert-butylat mit Acethydroxamsäure der Formel III 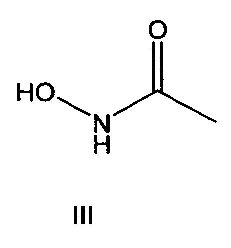 vorzugsweise bei Temperaturen von 20 °C bis 100 °C zu einer Verbindung der allgemeinen Formel I, 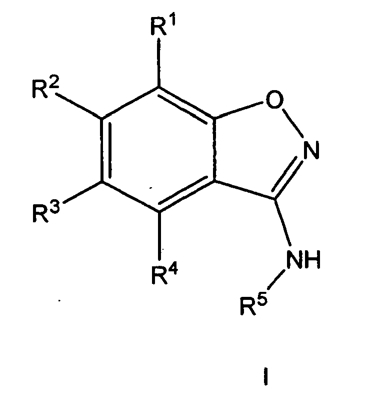 worin die Reste R¹-R⁴ die vorstehend genannte Bedeutung haben sowie der Rest R⁵ für einen Wasserstoff-Rest steht, umgesetzt wird, diese ggf. gereinigt und/oder ggf. isoliert wird,
diese ggf. anschließend in einem Reaktionsmedium, vorzugsweise ausgewählt aus der Gruppe bestehend aus Acetonitril, Toluol, Dimethylformamid, Benzol, Ethanol, Methanol und entsprechenden Mischungen, mit wenigstens einem Isothiocyanat der allgemeinen Formel S=C=N-R²², worin R²² die vorstehend angegebene Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹, R², R³, R⁴ die vorstehend genannte Bedeutung haben und R⁵ für -C(=S)-NR²¹R²² steht, wobei R²² die vorstehend genannte Bedeutung hat und R²¹ für einen Wasserstoff-Rest steht, und diese ggf. gereinigt und/oder ggf. isoliert wird,
und ggf. wenigstens eine Verbindung der allgemeinen Formel I, worin R¹, R², R³, R⁴ die vorstehend genannte Bedeutung haben, R⁵ für für -C(=S)-NR²¹R²² steht, wobei R²² die vorstehend genannte Bedeutung hat und R²¹ für einen Wasserstoff-Rest steht, in einem Reaktionsmedium, vorzugsweise ausgewählt aus der Gruppe bestehend aus Acetonitril, Toluol, Dimethylformamid, Benzol, Ethanol, Methanol und entsprechenden Mischungen, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R²¹, worin LG für eine Abgangsgruppe, bevorzugt für ein Halogen-Atom, besonders bevorzugt für ein Chloratom steht, und R²¹ die vorstehend genannte Bedeutung mit Ausnahme von Wasserstoff hat, zu wenigstens einer Verbindung der allgemeinen Formel I ungesetzt wird, und diese ggf. gereinigt und/oder ggf. isoliert wird,
oder
ggf. wenigstens eine Verbindung der allgemeinen Formel I, worin R¹, R², R³, R⁴ die vorstehend genannte Bedeutung haben und R⁵ für H steht, in einem Reaktionsmedium, vorzugsweise ausgewählt aus der Gruppe bestehend aus Acetonitril, Toluol, Dimethylformamid, Benzol, Ethanol, Methanol, DCM, Trifluoressigsäure und entsprechenden Mischungen, in Gegenwart wenigstens einer Base, bevorzugt in Gegenwart wenigstens eines Metallhydridsalzes oder eines Metallalkoholatsalzes oder Triethylsilan, besonders bevorzugt in Gegenwart des Triethylsilans, eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel R³⁰C(=O)H oder R⁶C(O)R²⁵, wobei R⁶ und R²⁵ die vorstehend genannte Bedeutung haben und R³⁰ für einen linearen oder verzweigten, gesättigten oder ungesättigten, unsubstituierten oder wenigstens einfach substituierten aliphatischen Rest; für einen unsubstituierten oder wenigstens einfach substituierten Aryl- oder Heteroaryl-Rest steht, der mit einem mono- oder polyzyklischen Ringsystem kondensiert sein kann; oder für einen unsubstituierten oder wenigstens einfach substituierten Aryl- oder -Heteroaryl-Rest steht, der mit einem mono- oder polyzyklischen Ringsystem kondensiert sein kann und über eine lineare oder verzweigte Alkylen-Gruppe gebunden ist, zu wenigstens einer Verbindung der allgemeinen Formel I umgesetzt und diese ggf. gereinigt und/oder ggf. isoliert wird.

13. Arzneimittel enthaltend wenigstens eine Verbindung gemäß Anspruch 1 und ggf. einen oder mehrere physiologisch verträgliche Hilfsstoffe.

14. Verwendung wenigstens einer Verbindung gemäß Anspruch 1 zur Herstellung eines Arzneimittels zur Behandlung von Schmerz, Migräne, Angstzuständen, Harninkontinenz oder Epilepsie.

15. Verwendung wenigstens einer Verbindung gemäß Anspruch 1 zur Herstellung eines Arzneimittels zur Behandlung von Schmerz ausgewählt aus der Gruppe bestehend aus akutem Schmerz, chronischem Schmerz, neuropathischem Schmerz, muskulärem Schmerz und inflammatorischem Schmerz.

## Claims

1. Substituted benzo[d]isoxazol-3-yl-amine compounds of the general formula I, 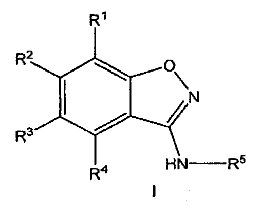 wherein
R¹, R², R³ and R⁴, independently of one another, each denote
H; F; Cl; Br; I; -CN; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; C₁₋₁₀ alkyl, C₂₋₁₀ alkenyl, C₂₋₁₀ alkynyl;
R⁵ denotes
-C(=S)NR²¹R²² or (CHR⁶)ₙ-R²⁵
where n = 1, 2 or 3
wherein R⁶ denotes H, C₃₋₈ cycloalkyl or C₁₋₆ alkyl
and R²⁵ denotes aryl or heteroaryl
R⁷ and R⁸, independently of each other, each denote
H, -C(=O)R¹⁴ or C₁₋₁₀ alkyl, or
R⁷ and R⁸ together with the nitrogen atom binding them as ring member form a radical selected from morpholine, piperidine or pyrrolidine;
R⁹, R¹⁰, R¹¹ and R¹⁶, independently of one another, each denote
H; C₁₋₁₀ alkyl, C₂₋₁₀ alkenyl, C₂₋₁₀ alkynyl; C₃₋₈ cycloalkyl; -(C₁₋₅ alkylene)-C₃₋₈ cycloalkyl; heterocycloalkyl; -(C₁₋₅ alkylene)-heterocycloalkyl; aryl; heteroaryl; -(C₁₋₅ alkylene)-aryl; -(C₁₋₅ alkylene)-heteroaryl;
R¹² and R¹³, independently of each other, each denote
H or a C₁₋₁₀ alkyl radical; or
R¹² and R¹³ together with the nitrogen atom binding them as ring member form a radical selected from morpholine, piperidine or pyrrolidine;
R¹⁴ denotes
-NR⁷R⁸; C₁₋₁₀ alkyl, C₂₋₁₀ alkenyl, C₂₋₁₀ alkynyl; C₃₋₈ cycloalkyl; -(C₁₋₅ alkylene)-C₃₋₈ cycloalkyl; heterocycloalkyl; -(C₁₋₅ alkylene)-heterocycloalkyl; aryl; heteroaryl; -(C₁₋₅ alkylene)-aryl; -(C₁₋₅ alkylene)-heteroaryl;
R¹⁵ and R¹⁷, independently of each other, each denote
C₁₋₁₀ alkyl, C₂₋₁₀ alkenyl, C₂₋₁₀ alkynyl; C₃₋₈ cycloalkyl; -(C₁₋₅ alkylene)-C₃₋₈ cycloalkyl; heterocycloalkyl; -(C₁₋₅ alkylene)-heterocycloalkyl; aryl; heteroaryl; -(C₁₋₅ alkylene)-aryl; -(C₁₋₅ alkylene)-heteroaryl;
R²¹ and R²², independently of each other, each denote H, C₁₋₁₀ alkyl, C₂₋₁₀ alkenyl, C₂₋₁₀ alkynyl; C₃₋₈ cycloalkyl; -(C₁₋₅ alkylene)-C₃₋₈ cycloalkyl; heterocycloalkyl; -(C₁₋₅ alkylene)-heterocycloalkyl; aryl; heteroaryl; -(C₁₋₅ alkylene)-aryl; -(C₁₋₅ alkylene)-heteroaryl;
wherein
the aforementioned C₁₋₁₀ alkyl, C₂₋₁₀ alkenyl and C₂₋₁₀ alkynyl radicals are each linear or branched and can optionally be substituted with 1, 2, 3, 4 or 5 substituents selected independently of one another from the group consisting of F; Cl; Br; COOH; COOC₁₋₄ alkyl; -CN; -OH; -SH; -O-C₁₋₂ alkyl; -S-C₁₋₂ alkyl and -NH₂,
the aforementioned C₃₋₈ cycloalkyl radicals can each optionally be substituted with 1, 2, 3, 4 or 5 substituents selected independently of one another from the group consisting of F; Cl; Br; -CN; -OH; -SH; -C₁₋₅ alkyl; -O-C₁₋₂ alkyl; -S-C₁₋₂ alkyl and -NH₂,
the aforementioned heterocycloalkyl radicals are each 4-, 5-, 6- or 7-membered, have 1 or 2 heteroatoms selected independently of one another from the group consisting of oxygen, sulfur and nitrogen as ring member(s) and can optionally be substituted with 1, 2, 3, 4 or 5 substituents selected independently of one another from the group consisting of F; Cl; Br; -CN; -OH; -SH; -C₁₋₅ alkyl; -O-C₁₋₂ alkyl; -S-C₁₋₂ alkyl and -NH₂,
the aforementioned aryl radicals can each denote a phenyl, anthracenyl or naphthyl radical, which can be substituted with 1, 2, 3, 4 or 5 substituents selected independently of one another from the group consisting of F; Cl; Br; I; -CF₃; -OCF₃; -SCF₃; C(=O)C₁₋₅ alkyl; 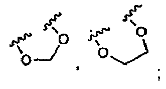 -NO₂; cyclohexyl; -SF₅; -CN; -OH; -SH; -C₁₋₅ alkyl; -O-C₁₋₅ alkyl; -S-C₁₋₅ alkyl; -C(=O)-OH; -O(C=O)C₁₋₂ alkyl; -C(=O)-OC₁₋₅ alkyl; -NH₂; -N(H)(C₁₋₅ alkyl); -N(C₁₋₅ alkyl)(C₁₋₅ alkyl); -C(=O)NH₂; -C(=O)N(H)(C₁₋₅ alkyl); -C(=O)N(C₁₋₅ alkyl)(C₁₋₅ alkyl); -S(=O)₂NH₂; -S(=O)₂N(H)(C₁₋₅ alkyl); -S(=O)₂N(C₁₋₅ alkyl)(C₁₋₅ alkyl); -S(=O)₂ phenyl; -S(=O)₂-C₁₋₅ alkyl; phenyl; phenoxy, benzyl; benzyloxy; thiophenyl (thienyl), furanyl and pyridinyl,
the aforementioned heteroaryl radicals are each 5- or 6-membered, have 1, 2 or 3 heteroatoms selected independently of one another from the group consisting of oxygen, sulfur and nitrogen as ring member(s) and can optionally be substituted with 1, 2, 3, 4 or 5 substituents selected independently of one another from the group consisting of F; Cl; Br; I; -CF₃; -OCF₃; -SCF₃; -SF₅; -CN; -OH; -SH; -C₁₋₅ alkyl; -O-C₁₋₅ alkyl; -S-C₁₋₅ alkyl; -C(=O)-OH; -C(=O)-OC₁₋₅ alkyl; -NH₂; -N(H)(C₁₋₅ alkyl); -N(C₁₋₅ alkyl)(C₁₋₅ alkyl); -C(=O)NH₂; -C(=O)N(H)(C₁₋₅ alkyl); -C(=O)N(C₁₋₅ alkyl)(C₁₋₅ alkyl); -S(=O)₂NH₂; -S(=O)₂N(H)(C₁₋₅ alkyl); -S(=O)₂N(C₁₋₅ alkyl)(C₁₋₅ alkyl); -S(=O)₂ phenyl; -S(=O)₂-C₁₋₅ alkyl; phenyl; phenoxy, benzyl; thiophenyl (thienyl), furanyl and pyridinyl,
in the form of the racemate; the enantiomers, diastereomers, mixtures of enantiomers or diastereomers or a single enantiomer or diastereomer; the bases and/or salts of physiologically compatible acids.

2. Compounds according to claim 1, wherein
R¹, R², R³ and R⁴, independently of one another, each denote
H; F; Cl; Br; I; -CN; -NR⁷R⁸; -OR⁹; -SR¹⁰; C₁₋₄ alkyl, C₂₋₄ alkenyl or C₂₋₄ alkynyl;
R⁵ denotes
-C(=S)NR²¹R²²
or
-(CHR⁶)ₙ-R²⁵
where n = 1, 2 or 3
wherein R⁶ denotes H or C₁₋₆ alkyl
and R²⁵ denotes aryl or heteroaryl
R⁷ and R⁸, independently of each other, each denote
H, -C(=O)R¹⁵ or C₁₋₄ alkyl, or
R⁷ and R⁸ together with the nitrogen atom binding them as ring member form a radical selected from morpholine, piperidine or pyrrolidine;
R⁹, R¹⁰, independently of each other, each denote
H; C₁₋₄ alkyl, C₂₋₄ alkenyl, C₂₋₄ alkynyl; C₃₋₈ cycloalkyl; -(C_{1, 2 or 3} alkylene)-C₃₋₈ cycloalkyl; heterocycloalkyl; -(C_{1, 2 or 3} alkylene)-heterocycloalkyl; aryl; heteroaryl; -(C_{1, 2 or 3} alkylene)-aryl; -(C_{1, 2 or 3} alkylene)-heteroaryl;
R²¹ and R²², independently of each other, each denote
H; C₁₋₁₀ alkyl, C₂₋₁₀ alkenyl, C₂₋₁₀ alkynyl; C₃₋₈ cycloalkyl; -(C_{1, 2 or 3} alkylene)-C₃₋₈ cycloalkyl; heterocycloalkyl; -(C_{1 2 or 3} alkylene)-heterocycloalkyl; aryl; heteroaryl; -(C_{1, 2 or 3} alkylene)-aryl; -(C_{1, 2 or 3} alkylene)-heteroaryl;
wherein
the aforementioned C₁₋₁₀ alkyl, C₂₋₁₀ alkenyl and C₂₋₁₀ alkynyl radicals are each linear or branched and can optionally be substituted with 1, 2, 3, 4 or 5 substituents selected independently of one another from the group consisting of F; Cl; Br; -CN; COOH; COOC₁₋₄ alkyl; -OH; -SH; -O-C₁₋₂ alkyl; -S-C₁₋₂ alkyl and -NH₂,
the aforementioned C₁₋₄ alkyl, C₂₋₄ alkenyl and C₂₋₄ alkynyl radicals are each linear or branched and can optionally be substituted with 1, 2, 3, 4 or 5 substituents selected independently of one another from the group consisting of F; Cl; Br; -CN; -OH; -OCH₃ and -NH₂,
the aforementioned C₃₋₈ cycloalkyl radicals can optionally be substituted with 1, 2, 3, 4 or 5 substituents selected independently of one another from the group consisting of F; Cl; Br, -CN; -OH; -CH₃; -C₂H₅; -OCH₃ and -NH₂,
the aforementioned heterocycloalkyl radicals are each 4-, 5-, 6- or 7-membered, have 1 or 2 heteroatoms selected independently of one another from the group consisting of oxygen, sulfur and nitrogen as ring member(s) and can optionally be substituted with 1, 2, 3, 4 or 5 substituents selected independently of one another from the group consisting of F; Cl; Br; -CN; -OH; -CH₃; -C₂H₅; -OCH₃ and -NH₂,
the aforementioned aryl radicals can each denote a phenyl, anthracenyl or naphthyl radical, which can be substituted with 1, 2, 3, 4 or 5 substituents selected independently of one another from the group consisting of F; Cl; Br; -CF₃; -OCF₃; -SCF₃; 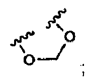 -NO₂, -C(=O)C₁₋₂ alkyl; cyclohexyl; - O(C=O)C₁₋₂ alkyl; -SF₅; -CN; -OH; methyl; ethyl; n-propyl; isopropyl; n-butyl; isobutyl; sec-butyl; tert-butyl; methoxy; ethoxy; -NH₂; -N(CH₃)₂; -N(C₂H₅)₂; phenyl; benzyloxy; phenoxy, benzyl; thiophenyl (thienyl), furanyl and pyridinyl;
the aforementioned heteroaryl radicals each denote a furanyl, thienyl (thiophenyl) or pyridinyl radical and can optionally be substituted with 1, 2, 3, 4 or 5 substituents selected independently of one another from the group consisting of F; Cl; Br; -CF₃; -OCF₃; -SCF₃; -SF₅; -CN; -OH; methyl; ethyl; n-propyl; isopropyl; n-butyl; isobutyl; sec-butyl; tert-butyl; methoxy; ethoxy; -NH₂; -N(CH₃)₂; -N(C₂H₅)₂; phenyl; phenoxy, benzyl; thiophenyl (thienyl), furanyl and pyridinyl,
in the form of the racemate; the enantiomers, diastereomers, mixtures of enantiomers or diastereomers or a single enantiomer or diastereomer; the bases and/or salts of physiologically compatible acids.

3. Compounds according to one of claims 1 to 2, wherein R⁵ denotes -C(=S)NR²¹R²².

4. Compounds according to one of claims 1 to 2, wherein R⁵ denotes -(CHR⁶)ₙ-R²⁵.

5. Compounds according to claim 3, wherein R²¹ denotes H and R²² denotes benzyl, phenyl, pyridyl, naphthyl or phenethyl, unsubstituted or mono- or polysubstituted with methyl, ethyl, isopropyl, Cl, F, Br, NO₂, acetyl, CN, COOH, COOC₁₋₄ alkyl, methoxy, ethoxy, N(CH₃)₂, N(C₂H₅)₂, 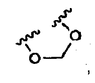 CF₃, SCH₃; C₁₋₁₀ alkyl, branched or unbranched, saturated or unsaturated, unsubstituted or substituted with OCH₃, COOCH₃ or COOC₂H₅; C₃-₆ cycloalkyl, wherein the cycloalkyl group can be linked via a CH₂ group; C₅₋₆ heterocycloalkyl, wherein the heterocycloalkyl group can be linked via a CH₂ group.

6. Compounds according to claim 5, wherein R²¹ denotes H and R²² denotes benzyl, phenyl, 2-methylphenyl, phenethyl, 2-isopropylphenyl, 2-chlorophenyl, 4-fluorobenzyl, 1-(4-fluorophenyl)ethyl, 4-chlorobenzyl, 4-chlorophenethyl, 4-nitrophenyl, 4-acetyl-phenyl, 3-carboxyphenyl, 3-methylbenzoate, 4-ethylbenzoate, 2,6-diethylphenyl, 3-chloro-4-methyl-phenyl, 2-methoxyethyl, 3-methoxypropyl, cyclopentyl, cyclohexyl, 3-pyridyl, 4-dimethylaminophenyl, 4-diethylaminophenyl, CH₂COOCH₃, CH(CH₃)COOC₂H₅, CH(CH₃)CH₂COOC₂H₅, cyclohexylmethyl, 4-ethoxyphenyl, 3,4-dimethoxyphenyl, 1-naphthyl, 3,4,5-trimethoxyphenyl, 2,3,4,5,6-pentafluorophenyl, benzodioxole, 4-fluorophenyl, methyl, ethyl, propyl, isopropyl, tert-butyl, allyl, 2-methylprop-2-enyl, 2-nitrophenyl, 3-trifluoromethylphenyl, 2-trifluoromethylphenyl, 4-trifluoromethylphenyl, cyclopropyl, 2-methylsulfanylphenyl, 3-methylsulfanylphenyl, 4-methylsulfanylphenyl, 3,5-dimethylphenyl, ethylmorpholine, ((4-propyl)-cyclohexyl)phenyl, 4-bromo-2-trifluoromethylphenyl, n-octyl, n-nonanyl, tetrahydrofurylmethyl, 2-ethylphenyl, 4-cyanophenyl, 3-cyanophenyl, 2,6-diisopropylphenyl, n-pentyl, n-hexyl, sec-butyl, propylmorpholine, 5-chloro-2-methoxyphenyl, 4-chloro-3-trifluoromethylphenyl, 3-chlorophenyl, 1-phenylethyl, CH(CH₃)COOCH₃, 2-methoxyphenyl, 3-methoxyphenyl, 4-methoxyphenyl, CH₂CH₂COOC₂H₅, 2-methylbenzoate, 4-methylbenzoate, 2-ethylbenzoate, 2-fluorophenyl, 2-methoxy-5-chlorophenyl or 2,4-dimethoxyphenyl.

7. Compounds according to claim 4, wherein R⁶ denotes H.

8. Compounds according to claim 4, wherein R⁶ denotes CH₃.

9. Compounds according to one of claims 4, 7 or 8, wherein R²⁵ denotes phenyl, pyridyl, thienyl or furyl, each unsubstituted or mono- or polysubstituted with CF₃, SCF₃, C₁-₄ alkyl, Cl, NO₂, O-acetyl, OCH₃, F, O-phenyl, OCF₃, Br, O-benzyl, O-allyl, phenyl, I, CN or OH.

10. Compounds according to claim 9, wherein R²⁵ denotes 4-trifluoromethylphenyl, 4-SCF₃-phenyl, 2-methylphenyl, phenyl, anthracenyl, 4-Cl-phenyl, 4-OCF₃-phenyl, 4-n-butylphenyl, 3(3-CF₃-phenoxy)-phenyl, 4-OCHF₂-phenyl, 3,5-dimethylphenyl, 3-bromo-4-methoxyphenyl, 4-benzyloxy-3,5-dimethylphenyl, 3-nitrophenyl, 3-methoxy-4-(acetylmethyl)-phenyl, 2,4,5-trimethoxyphenyl, 4-ethylphenyl, 3,4-dichlorophenyl, 2,3,5-trifluorophenyl, 4-phenoxyphenyl, 3-chloro-4-fluorophenyl, 3-benzyloxy-phenyl, 3-bromo-4,5-dimethoxyphenyl, 3-fluoro-2-methylphenyl, 2-chloro-3-trifluoromethylphenyl, 3-chloro-2-fluoro-5-trifluoromethylphenyl, 2-fluoro-4-trifluoromethylphenyl, 4-(allyloxy)-phenyl, 2-(benzyloxy)-4,5-dimethoxyphenyl, 2-phenyl-phenyl, 2,3,4-trifluorophenyl, 2-fluoro-5-trifluorophenyl, 4-methoxy-3-methylphenyl, 2-fluoro-3-chlorophenyl, 3,4-difluorophenyl, 2,6-dichlorophenyl, 3-iodophenyl, 3-iodo-4,5-dimethoxyphenyl, 2-cyanophenyl, 4-hydroxyphenyl, 3,4-dimethylphenyl or 3-OCF₃-phenyl.

11. Compounds according to claim 1 from the group consisting of
Benzo[d]isoxazol-3-yl-[1-(4-trifluoromethyl-phenyl)-ethyl]-amine
Benzo[d]isoxazol-3-yl-[1-(4-trifluoromethylsulfanyl-phenyl)-ethyl]-amine
1-Benzo[d]isoxazol-3-yl-3-benzyl-thiourea
1-Benzo[d]isoxazol-3-yl-3-phenyl-thiourea
1-Benzo[d]isoxazol-3-yl-3-o-tolyl-thiourea
1-Benzo[d]isoxazol-3-yl-3-phenethyl-thiourea
1-Benzo[d]isoxazol-3-yl-3-(2-isopropyl-phenyl)-thiourea
1-Benzo[d]isoxazol-3-yl-3-(2-chlorophenyl)-thiourea
1-Benzo[d]isoxazol-3-yl-3-(4-fluorobenzyl)-thiourea
1-Benzo[d]isoxazol-3-yl-3-[1-(4-fluorophenyl)-ethyl]-thiourea
1-Benzo[d]isoxazol-3-yl-3-(4-chlorobenzyl)-thiourea
1-Benzo[d]isoxazol-3-yl-3-[2-(4-chlorophenyl)-ethyl]-thiourea
1-Benzo[d]isoxazol-3-yl-3-(4-nitrophenyl)-thiourea
1-(4-Acetyl-phenyl)-3-benzo[d]isoxazol-3-yl-thiourea
3-(3-Benzo[d]isoxazol-3-yl-thioureido)-benzoic acid
3-(3-Benzo[d]isoxazol-3-yl-thioureido)-benzoic acid methyl ester
4-(3-Benzo[d]isoxazol-3-yl-thioureido)-benzoic acid ethyl ester
1-Benzo[d]isoxazol-3-yl-3-(2,6-diethyl-phenyl)-thiourea
1-Benzo[d]isoxazol-3-yl-3-(3-chloro-4-methyl-phenyl)-thiourea
1-Benzo[d]isoxazol-3-yl-3-(2-methoxy-ethyl)-thiourea
1-Benzo[d]isoxazol-3-yl-3-(3-methoxy-propyl)-thiourea
1-Benzo[d]isoxazol-3-yl-3-cyclopentyl-thiourea
1-Benzo[d]isoxazol-3-yl-3-cyclohexyl-thiourea
1-Benzo[d]isoxazol-3-yl-3-pyridin-3-yl-thiourea
1-Benzo[d]isoxazol-3-yl-3-(4-dimethylamino-phenyl)-thiourea
1-Benzo[d]isoxazol-3-yl-3-(4-diethylamino-phenyl)-thiourea
(3-Benzo[d]isoxazol-3-yl-thioureido)-acetic acid methyl ester
2-(3-Benzo[d]isoxazol-3-yl-thioureido)-propionic acid ethyl ester
3-(3-Benzo[d]isoxazol-3-yl-thioureido)-butyric acid ethyl ester
1-Benzo[d]isoxazol-3-yl-3-cyclohexylmethyl-thiourea
1-Benzo[d]isoxazol-3-yl-3-(4-ethoxy-phenyl)-thiourea
1-Benzo[d]isoxazol-3-yl-3-(3,4-dimethoxy-phenyl)-thiourea
1-Benzo[d]isoxazol-3-yl-3-(3,4,5-trimethoxy-phenyl)-thiourea
1-Benzo[d]isoxazol-3-yl-3-pentafluorophenyl-thiourea
1-Benzo[d]isoxazol-3-yl-3-naphthalen-1-yl-thiourea
1-Benzo[1,3]dioxol-5-ylmethyl-3-benzo[d]isoxazol-3-yl-thiourea
1-Benzo[d]isoxazol-3-yl-3-(4-fluorophenyl)-thiourea
1-Benzo[d]isoxazol-3-yl-3-methyl-thiourea
1-Benzo[d]isoxazol-3-yl-3-ethyl-thiourea
1-Benzo[d]isoxazol-3-yl-3-propyl-thiourea
1-Benzo[d]isoxazol-3-yl-3-isopropyl-thiourea
1-Benzo[d]isoxazol-3-yl-3-tert-butyl-thiourea
1-Allyl-3-benzo[d]isoxazol-3-yl-thiourea
1-Benzo[d]isoxazol-3-yl-3-(2-methyl-allyl)-thiourea
1-Benzo[d]isoxazol-3-yl-3-(2-nitrophenyl)-thiourea
1-Benzo[d]isoxazol-3-yl-3-(2-trifluoromethyl-phenyl)-thiourea
1-Benzo[d]isoxazol-3-yl-3-(3-trifluoromethyl-phenyl)-thiourea
1-Benzo[d]isoxazol-3-yl-3-(4-trifluoromethyl-phenyl)-thiourea
1-Benzo[d]isoxazol-3-yl-3-cyclopropyl-thiourea
2-[3-(4-Fluorobenzo[d]isoxazol-3-yl)-thioureido]-propionic acid methyl ester
1-(4-Chlorobenzo[d]isoxazol-3-yl)-3-o-tolyl-thiourea
1-Benzyl-3-(4-chlorobenzo[d]isoxazol-3-yl)-thiourea
1-(4-Chlorobenzo[d]isoxazol-3-yl)-3-(1-phenyl-ethyl)-thiourea
1-(4-Dimethylamino-benzo[d]isoxazol-3-yl)-3-isobutyl-thiourea
1-(4-Dimethylamino-benzo[d]isoxazol-3-yl)-3-p-tolyl-thiourea
1-(3-Chlorophenyl)-3-(4-dimethylamino-benzo[d]isoxazol-3-yl)-thiourea
1-(4-Dimethylamino-benzo[d]isoxazol-3-yl)-3-(3-methoxy-phenyl)-thiourea
1-(4-Dimethylamino-benzo[d]isoxazol-3-yl)-3-(2-methylsulfanyl-phenyl)-thiourea
1-(4-Dimethylamino-benzo[d]isoxazol-3-yl)-3-(3-methylsulfanyl-phenyl)-thiourea
1-(4-Dimethylamino-benzo[d]isoxazol-3-yl)-3-(4-methylsulfanyl-phenyl)-thiourea
1-(4-Dimethylamino-benzo[d]isoxazol-3-yl)-3-(2-methoxy-phenyl)-thiourea
1-(4-Dimethylamino-benzo[d]isoxazol-3-yl)-3-(4-methoxy-phenyl)-thiourea
1-(4-Dimethylamino-benzo[d]isoxazol-3-yl)-3-(3,5-dimethyl-phenyl)-thiourea
1-Benzyl-3-(4-dimethylamino-benzo[d]isoxazol-3-yl)-thiourea
1-(4-Dimethylamino-benzo[d]isoxazol-3-yl)-3-(3-methoxy-propyl)-thiourea
3-[3-(4-Dimethylamino-benzo[d]isoxazol-3-yl)-thioureido]-propionic acid ethyl ester
2-[3-(4-Dimethylamino-benzo[d]isoxazol-3-yl)-thioureido]-propionic acid ethyl ester
3-[3-(4-Dimethylamino-benzo[d]isoxazol-3-yl)-thioureido]-butyric acid ethyl ester
3-[3-(4-Dimethylamino-benzo[d]isoxazol-3-yl)-thioureido]-benzoic acid
1-(4-Dimethylamino-benzo[d]isoxazol-3-yl)-3-(4-ethoxy-phenyl)-thiourea
2-[3-(4-Dimethylamino-benzo[d]isoxazol-3-yl)-thioureido]-benzoic acid methyl ester
3-[3-(4-Dimethylamino-benzo[d]isoxazol-3-yl)-thioureido]-benzoic acid methyl ester
4-[3-(4-Dimethylamino-benzo[d]isoxazol-3-yl)-thioureido]-benzoic acid methyl ester
2-[3-(4-Dimethylamino-benzo[d]isoxazol-3-yl)-thioureido]-benzoic acid ethyl ester
4-[3-(4-Dimethylamino-benzo[d]isoxazol-3-yl)-thioureido]-benzoic acid ethyl ester
1-(4-Acetyl-phenyl)-3-(4-dimethylamino-benzo[d]isoxazol-3-yl)-thiourea
1-(2-Chlorophenyl)-3-(4-methoxy-benzo[d]isoxazol-3-yl)-thiourea
1-(4-Diethylamino-phenyl)-3-(4-methoxy-benzo[d]isoxazol-3-yl)-thiourea
1-(4-Methoxy-benzo[d]isoxazol-3-yl)-3-(2-morpholin-4-yl-ethyl)-thiourea
2-{3-[4-(2,2,2-trifluoroethoxy)-benzo[d]isoxazol-3-yl]-thioureido}-propionic acid methyl ester
1-Benzo[1,3]dioxol-5-ylmethyl-3-[4-(2,2,2-trifluoroethoxy)-benzo[d]isoxazol-3-yl]-thiourea
1-[4-(4-Propyl-cyclohexyl)-phenyl]-3-[4-(2,2,2-trifluoroethoxy)-benzo[d]isoxazol-3-yl]-thiourea
1-(4-Bromo-2-trifluoromethyl-phenyl)-3-[4-(2,2,2-trifluoroethoxy)-benzo[d]isoxazol-3-yl]-thiourea
1-(4-Methoxy-phenyl)-3-[4-(2,2,2-trifluoroethoxy)-benzo[d]isoxazol-3-yl]-thiourea
3-{3-[4-(2,2,2-trifluoroethoxy)-benzo[d]isoxazol-3-yl]-thioureido}-propionic acid ethyl ester
1-[4-(4-Methyl-benzyloxy)-benzo[d]isoxazol-3-yl]-3-octyl-thiourea
1-[4-(4-Methyl-benzyloxy)-benzo[d]isoxazol-3-yl]-3-nonyl-thiourea
1-Cyclopropyl-3-[4-(4-methyl-benzyloxy)-benzo[d]isoxazol-3-yl]-thiourea
1-Cyclopentyl3-[4-(4-methyl-benzyloxy)-benzo[d]isoxazol-3-yl]-thiourea
1-Cyclohexyl-3-[4-(4-methyl-benzyloxy)-benzo[d]isoxazol-3-yl]-thiourea
1-Cyclohexylmethyl-3-[4-(4-methyl-benzyloxy)-benzo[d]isoxazol-3-yl]-thiourea
1-(4-Dimethylamino-phenyl)-3-[4-(2,2,2-trifluoroethoxy)-benzo[d]isoxazol-3-yl]-thiourea
1-Allyl-3-(5-methyl-benzo[d]isoxazol-3-yl)-thiourea
[3-(5-Methyl-benzo[d]isoxazol-3-yl)-thioureido]-acetic acid methyl ester
1-(2-Isopropyl-phenyl)-3-(5-methyl-benzo[d]isoxazol-3-yl)-thiourea
1-(5-Methyl-benzo[d]isoxazol-3-yl)-3-(4-trifluoromethyl-phenyl)-thiourea
2-[3-(5-Fluorobenzo[d]isoxazol-3-yl)-thioureido]-propionic acid methyl ester
1-(5-Fluorobenzo[d]isoxazol-3-yl)-3-(tetrahydro-furan-2-ylmethyl)-thiourea
1-(5-Bromobenzo[d]isoxazol-3-yl)-3-(2-fluorophenyl)-thiourea
1-(5-Bromobenzo[d]isoxazol-3-yl)-3-(2-ethyl-phenyl)-thiourea
1-(6-Chlorobenzo[d]isoxazol-3-yl)-3-(4-fluorophenyl)-thiourea
1-(6-Chlorobenzo[d]isoxazol-3-yl)-3-(2-fluorophenyl)-thiourea
1-(6-Chlorobenzo[d]isoxazol-3-yl)-3-cyclopentyl-thiourea
1-(6-Chlorobenzo[d]isoxazol-3-yl)-3-(4-cyano-phenyl)-thiourea
3-[3-(6-Chlorobenzo[d]isoxazol-3-yl)-thioureido]-benzoic acid
1-(6-Chlorobenzo[d]isoxazol-3-yl)-3-(4-methoxy-phenyl)-thiourea
1-(6-Chlorobenzo[d]isoxazol-3-yl)-3-(3-methoxy-phenyl)-thiourea
1-(6-Bromobenzo[d]isoxazol-3-yl)-3-(3,4-dimethoxy-phenyl)-thiourea
1-(6-Bromobenzo[d]isoxazol-3-yl)-3-naphthalen-1-yl-thiourea
1-Benzo[1,3]dioxol-5-ylmethyl-3-(6-bromobenzo[d]isoxazol-3-yl)-thiourea
1-(6-Fluorobenzo[d]isoxazol-3-yl)-3-(2-methylsulfanyl-phenyl)-thiourea
1-(3-Cyano-phenyl)-3-(6-fluorobenzo[d]isoxazol-3-yl)-thiourea
1-(2-Chloro-6-methyl-phenyl)-3-(6-fluorobenzo[d]isoxazol-3-yl)-thiourea
1-(2,6-Diisopropyl-phenyl)-3-(6-fluorobenzo[d]isoxazol-3-yl)-thiourea
1-(7-Fluorobenzo[d]isoxazol-3-yl)-3-methyl-thiourea
1-Ethyl-3-(7-fluorobenzo[d]isoxazol-3-yl)-thiourea
1-(7-Fluorobenzo[d]isoxazol-3-yl)-3-propyl-thiourea
1-(7-Fluorobenzo[d]isoxazol-3-yl)-3-pentyl-thiourea
1-(7-Fluorobenzo[d]isoxazol-3-yl)-3-hexyl-thiourea
1-(7-Fluorobenzo[d]isoxazol-3-yl)-3-octyl-thiourea
1-(7-Fluorobenzo[d]isoxazol-3-yl)-3-nonyl-thiourea
1-(7-Fluorobenzo[d]isoxazol-3-yl)-3-isobutyl-thiourea
1-Allyl-3-(7-fluorobenzo[d]isoxazol-3-yl)-thiourea
1-(7-Fluorobenzo[d]isoxazol-3-yl)-3-p-tolyl-thiourea
1-(5-Bromobenzo[d]isoxazol-3-yl)-3-(4-dimethylamino-phenyl)-thiourea
1-(7-Fluorobenzo[d]isoxazol-3-yl)-3-(2-morpholin-4-yl-ethyl)-thiourea
1-(7-Fluorobenzo[d]isoxazol-3-yl)-3-(3-morpholin-4-yl-propyl)-thiourea
1-(4-Methoxy-benzo[d]isoxazol-3-yl)-3-(1-phenyl-ethyl)-thiourea
1-(4-Chlorobenzyl)-3-(4-methoxy-benzo[d]isoxazol-3-yl)-thiourea
1-(4-Methoxy-benzo[d]isoxazol-3-yl)-3-(2-methoxy-phenyl)-thiourea
1-(5-Bromobenzo[d]isoxazol-3-yl)-3-(4-dimethylamino-phenyl)-thiourea
1-(5-Chloro-2-methoxy-phenyl)-3-(4-methoxy-benzo[d]isoxazol-3-yl)-thiourea
1-(4-Chloro-3-trifluoromethyl-phenyl)-3-(4-methoxy-benzo[d]isoxazol-3-yl)-thiourea
1-(2,4-Dimethoxy-phenyl)-3-(4-methoxy-benzo[d]isoxazol-3-yl)-thiourea
1-[4-(2,2,2-Trifluoroethoxy)-benzo[d]isoxazol-3-yl]-3-(3,4,5-trimethoxy-phenyl)-thiourea
Benzo[d]isoxazol-3-yl-(3-methyl-butyl)-amine
(5-Fluorobenzo[d]isoxazol-3-yl)-(2-methyl-benzyl)-amine
N4,N4-Dimethyl-N3-(3-phenyl-propyl)-benzo[d]isoxazole-3,4-diamine
N3-Butyl-N4,N4-dimethyl-benzo[d]isoxazole-3,4-diamine
Anthracen-9-ylmethyl-(4-methoxy-benzo[d]isoxazol-3-yl)-amine
(4-Chlorobenzyl)-(4-methoxy-benzo[d]isoxazol-3-yl)-amine
(6-Fluorobenzo[d]isoxazol-3-yl)-(3-nitro-benzyl)-amine
Acetic acid-4-[(6-chlorobenzo[d]isoxazol-3-ylamino)-methyl]-2-methoxyphenyl ester
Acetic acid-4-[(6-bromobenzo[d]isoxazol-3-ylamino)-methyl]-2-methoxyphenyl ester
Benzo[d]isoxazol-3-yl-(3,4-dichlorobenzyl)-amine
Benzo[d]isoxazol-3-yl-(2,4,5-trimethoxy-benzyl)-amine
Benzo[d]isoxazol-3-yl-(4-ethyl-benzyl)-amine
(6-Chlorobenzo[d]isoxazol-3-yl)-(3,4-dichlorobenzyl)-amine
Benzo[d]isoxazol-3-yl-(2,3,5-trifluorobenzyl)-amine
(6-Chlorobenzo[d]isoxazol-3-yl)-(4-phenoxy-benzyl)-amine
(3-Chloro-4-fluorobenzyl)-(7-fluorobenzo[d]isoxazol-3-yl)-amine
Benzo[d]isoxazol-3-yl-(4-trifluoromethyl-benzyl)-amine
(7-Fluorobenzo[d]isoxazol-3-yl)-(2-methyl-pentyl)-amine
N4,N4-Dimethyl-N3-(2,3,4-trifluorobenzyl)-benzo[d]isoxazole-3,4-diamine
N3-(2-Fluoro-5-trifluoromethyl-benzyl)-N4,N4-dimethyl-benzo[d]isoxazole-3,4-diamine
N3-(4-Methoxy-3-methyl-benzyl)-benzo[d]isoxazole-3,4-diamine
N3-(4-Methoxy-3-methyl-benzyl)-benzo[d]isoxazole-3,4-diamine
Benzo[d]isoxazol-3-yl-(4-trifluoromethoxy-benzyl)-amine
(5-Fluorobenzo[d]isoxazol-3-yl)-(4-trifluoromethoxy-benzyl)-amine
Benzo[d]isoxazol-3-yl-(4-trifluoromethylsulfanyl-benzyl)-amine
(4-Butyl-benzyl)-(6-chlorobenzo[d]isoxazol-3-yl)-amine
(5-Fluorobenzo[d]isoxazol-3-yl)-(4-trifluoromethylsulfanyl-benzyl)-amine
Benzo[d]isoxazol-3-yl-(2-fluoro-4-trifluoromethyl-benzyl)-amine
(7-Fluorobenzo[d]isoxazol-3-yl)-(4-trifluoromethoxy-benzyl)-amine
(7-Fluorobenzo[d]isoxazol-3-yl)-[3-(3-trifluoromethyl-phenoxy)-benzyl]-amine
(4-Difluoromethoxy-benzyl)-(4-methoxy-benzo[d]isoxazol-3-yl)-amine
(3,5-Dimethyl-benzyl)-(7-fluorobenzo[d]isoxazol-3-yl)-amine
(3-Bromo-4-methoxy-benzyl)-(6-fluorobenzo[d]isoxazol-3-yl)-amine
(3,5-Dimethyl-benzyl)-(6-fluorobenzo[d]isoxazol-3-yl)-amine
(4-Benzyloxy-3,5-dimethyl-benzyl)-(6-fluorobenzo[d]isoxazol-3-yl)-amine
(4-Butyl-benzyl)-(6-fluorobenzo[d]isoxazol-3-yl)-amine
(6-Fluorobenzo[d]isoxazol-3-yl)-(4-trifluoromethylsulfanyl-benzyl)-amine
(3-Benzyloxy-benzyl)-(6-fluorobenzo[d]isoxazol-3-yl)-amine
N3-(3,5-Dimethyl-benzyl)-benzo[d]isoxazole-3,4-diamine
N3-(4-Butyl-benzyl)-benzo[d]isoxazole-3,4-diamine
(5-Bromobenzo[d]isoxazol-3-yl)-(4-trifluoromethylsulfanyl-benzyl)-amine
(3-Bromo-4,5-dimethoxy-benzyl)-(7-fluorobenzo[d]isoxazol-3-yl)-amine
(7-Fluorobenzo[d]isoxazol-3-yl)-(2-fluoro-4-trifluoromethyl-benzyl)-amine
N3-(3-Fluoro-2-methyl-benzyl)-N4,N4-dimethyl-benzo[d]isoxazole-3,4-diamine
N3-(2-Chloro-3-trifluoromethyl-benzyl)-N4,N4-dimethyl-benzo[d]isoxazole-3,4-diamine
N3-(3-Chloro-2-fluoro-5-trifluoromethyl-benzyl)-N4,N4-dimethyl-benzo[d]isoxazole-3,4-diamine
(6-Fluorobenzo[d]isoxazol-3-yl)-(2-fluoro-4-trifluoromethyl-benzyl)-amine
(4-Allyloxy-benzyl)-(6-fluorobenzo[d]isoxazol-3-yl)-amine
Benzo[d]isoxazol-3-yl-(2-benzyloxy-4,5-dimethoxy-benzyl)-amine
(2-Benzyloxy-4,5-dimethoxy-benzyl)-(6-chlorobenzo[d]isoxazol-3-yl)-amine
N3-(2-Benzyloxy-4,5-dimethoxy-benzyl)-N4,N4-dimethyl-benzo[d]isoxazole-3,4-diamine
N3-Biphenyl-2-ylmethyl-N4,N4-dimethyl-benzo[d]isoxazole-3,4-diamine
(6-Fluorobenzo[d]isoxazol-3-yl)-(3-iodo-benzyl)-amine
(2-Benzyloxy-4,5-dimethoxy-benzyl)-(4-methoxy-benzo[d]isoxazol-3-yl)-amine
(4-Fluorobenzo[d]isoxazol-3-yl)-(3-iodo-4,5-dimethoxy-benzyl)-amine
2-[(5-Methyl-benzo[d]isoxazol-3-ylamino)-methyl]-benzonitrile
Butyl-[4-(2,2,2-trifluoroethoxy)-benzo[d]isoxazol-3-yl]-amine
(3-Bromo-4,5-dimethoxy-benzyl)-(5-methyl-benzo[d]isoxazol-3-yl)-amine
4-[(4-Chlorobenzo[d]isoxazol-3-ylamino)-methyl]-phenol
(6-Chlorobenzo[d]isoxazol-3-yl)-(3,4-dimethyl-benzyl)-amine
(4-Chlorobenzo[d]isoxazol-3-yl)-(3-chloro-2-fluorobenzyl)-amine
(3,4-Difluorobenzyl)-(5-fluorobenzo[d]isoxazol-3-yl)-amine
(6-Bromobenzo[d]isoxazol-3-yl)-(2,6-dichlorobenzyl)-amine
(7-Fluorobenzo[d]isoxazol-3-yl)-(3-trifluoromethoxy-benzyl)-amine
and the corresponding salts thereof, in particular the hydrochloride addition salts thereof, and optionally the corresponding solvates thereof.

12. Process for the preparation of the substituted benzo[d]isoxazol-3-yl-amine compounds according to the invention, according to which an optionally substituted 2-fluorobenzonitrile compound of the general formula II, 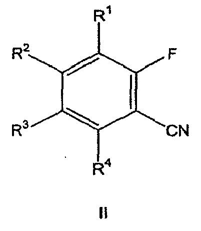 wherein the radicals R¹, R², R³ and R⁴ have the meaning given above, is reacted in a reaction medium, preferably selected from the group consisting of diethyl ether, tetrahydrofuran, acetonitrile, dimethyl sulfoxide, dimethylformamide and dichloromethane, in the presence of a base, preferably in the presence of at least one alkali metal alcoholate salt, particularly preferably in the presence of an alkali metal alcoholate salt selected from the group consisting of potassium methanolate, sodium methanolate, potassium tert-butylate and sodium tert-butylate, with acetohydroxamic acid of the formula III 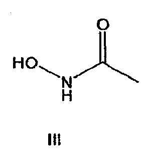 preferably at temperatures from 20°C to 100°C, to form a compound of the general formula I, 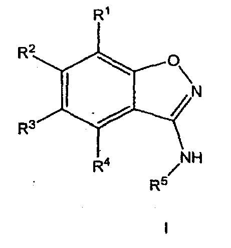 wherein the radicals R¹ to R⁴ have the meaning given above and the radical R⁵ denotes a hydrogen radical, it is optionally purified and/or optionally isolated,
it is then optionally reacted in a reaction medium, preferably selected from the group consisting of acetonitrile, toluene, dimethylformamide, benzene, ethanol, methanol and corresponding mixtures, with at least one isothiocyanate of the general formula S=C=N-R²², wherein R²² has the meaning given above, optionally in the presence of at least one base, preferably in the presence of at least one base selected from the group consisting of triethylamine, 4,4-dimethylaminopyridine and diisopropylethylamine, to form at least one compound of the general formula I, wherein R¹, R², R³, R⁴ have the meaning given above and R⁵ denotes -C(=S)-NR²¹R²², wherein R²² has the meaning given above and R²¹ denotes a hydrogen radical, and this is optionally purified and/or optionally isolated,
and optionally at least one compound of the general formula I, wherein R¹, R², R³, R⁴ have the meaning given above, R⁵ denotes -C(=S)-NR²¹R²², wherein R²² has the meaning given above and R²¹ denotes a hydrogen radical, is reacted in a reaction medium, preferably selected from the group consisting of acetonitrile, toluene, dimethylformamide, benzene, ethanol, methanol and corresponding mixtures, in the presence of at least one base, preferably in the presence of at least one metal hydride salt or metal alcoholate salt, particularly preferably in the presence of a metal hydride salt or a metal alcoholate salt selected from the group consisting of sodium hydride, potassium hydride, potassium tert-butanolate, sodium tert-butanolate, potassium methanolate, sodium methanolate, sodium ethanolate and potassium ethanolate, with at least one compound of the general formula LG-R²¹, wherein LG denotes a leaving group, preferably a halogen atom, particularly preferably a chlorine atom, and R²¹ has the meaning given above with the exception of hydrogen, to form at least one compound of the general formula I, and this is optionally purified and/or optionally isolated,
or
optionally at least one compound of the general formula I, wherein R¹, R², R³, R⁴ have the meaning given above and R⁵ denotes H, is reacted in a reaction medium, preferably selected from the group consisting of acetonitrile, toluene, dimethylformamide, benzene, ethanol, methanol, DCM, trifluoroacetic acid and corresponding mixtures, in the presence of at least one base, preferably in the presence of at least one metal hydride salt or metal alcoholate salt or triethylsilane, particularly preferably in the presence of triethylsilane, a metal hydride salt or a metal alcoholate salt selected from the group consisting of sodium hydride, potassium hydride, potassium tert-butanolate, sodium tert-butanolate, potassium methanolate, sodium methanolate, sodium ethanolate and potassium ethanolate, with at least one compound of the general formula R³⁰C(=O)H or R⁶C(O)R²⁵, wherein R⁶ and R²⁵ have the meaning given above and R³⁰ denotes a linear or branched, saturated or unsaturated, unsubstituted or at least monosubstituted aliphatic radical; an unsubstituted or at least monosubstituted aryl or heteroaryl radical, which can be fused to a monocyclic or polycyclic ring system; or an unsubstituted or at least monosubstituted aryl or heteroaryl radical, which can be fused to a monocyclic or polycyclic ring system and is bonded via a linear or branched alkylene group, to form at least one compound of the general formula I, and this is optionally purified and/or optionally isolated.

13. Medicament containing at least one compound according to claim 1 and optionally one or more physiologically compatible auxiliary substances.

14. Use of at least one compound according to claim 1 to prepare a medicament for the treatment of pain, migraine, anxiety states, urinary incontinence or epilepsy.

15. Use of at least one compound according to claim 1 to prepare a medicament for the treatment of pain selected from the group consisting of acute pain, chronic pain, neuropathic pain, muscular pain and inflammatory pain.

## Revendications

1. Composés substitués de benzo[d]isoxazole-3-yl-amine de formule générale I 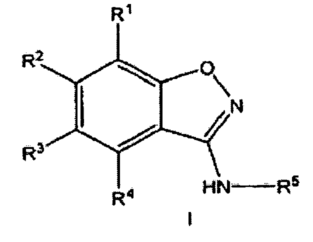 dans laquelle
R¹, R², R³ et R⁴ représentent dans chaque cas, indépendamment les uns des autres
H ; F ; Cl ; Br ; I ; -CN ; -NO₂ ; -SF₅ ; -NR⁷R⁸ ; -OR⁹ ; -SR¹⁰ ; -C(=O)OR¹¹ ; -(C=O)NR¹²R¹³ ; -S(=O)₂R¹⁴ ; -C(=O)R¹⁵ ; -NR¹⁶-S(=O)₂R¹⁷ ; un reste alkyle en C₁ à C₁₀, alcényle en C₂ à C₁₀, alcynyle en C₂ à C₁₀ ; R⁵ représente
un groupe -C(=S)NR²¹R²² ou (CHR⁶)ₙ-R²⁵
avec n = 1, 2 ou 3
R⁶ représentant H, un reste cycloalkyle en C₃ à C₈ ou alkyle en C₁ à C₆,
et R²⁵ représentant un reste aryle ou hétéroaryle, R⁷ et R⁸ représentent, indépendamment l'un de l'autre,
dans chaque cas
H, un groupe -C(=O)R¹⁴ ou un reste alkyle en C₁ à C₁₀, ou bien
R⁷ et R⁸ forment, conjointement avec l'atome d'azote qui les lie en tant que chaînon du noyau, un reste qui est choisi entre morpholine, pipéridine ou pyrrolidine ;
R⁹, R¹⁰, R¹¹ et R¹⁶ représentent dans chaque cas, indépendamment les uns des autres,
H ; un reste alkyle en C₁ à C₁₀, alcényle en C₂ à C₁₀, alcynyle en C₂ à C₁₀ ; cycloalkyle en C₃ à C₈ ; -(alkylène en C₁ à C₅)-(cycloalkyle en C₃ à C₈) ; hétérocycloalkyle ; -(alkylène en C₁ à C₅)-hétérocycloalkyle ; aryle ; hétéroaryle ; -(alkylène en C₁ à C₅)-aryle ; -(alkylène en C₁ à C₅)-hétéroaryle ;
R¹² et R¹³ représentent, indépendamment l'un de l'autre,
dans chaque cas
H ou un reste alkyle en C₁ à C₁₀ ; ou bien
R¹² et R¹³ forment, conjointement avec l'atome d'azote qui les lie en tant que chaînon du noyau, un reste qui est choisi entre morpholine, pipéridine ou pyrrolidine ;
R¹⁴ représente
un groupe -NR⁷R⁸ ; un reste alkyle en C₁ à C₁₀, alcényle en C₂ à C₁₀, alcynyle en C₂ à C₁₀ ; cycloalkyle en C₃ à C₈ ; -(alkylène en C₁ à C₅)-(cycloalkyle en C₃ à C₈) ; hétérocycloalkyle ; -(alkylène en C₁ à C₅)-hétérocycloalkyle ; aryle ; hétéroaryle ; -(alkylène en C₁ à C₅)-aryle ; -(alkylène en C₁ à C₅)-hétéroaryle ; R¹⁵ et R¹⁷ représentent, indépendamment l'un de l'autre
dans chaque cas
un reste alkyle en C₁ à C₁₀, alcényle en C₂ à C₁₀, alcynyle en C₂ à C₁₀ ; cycloalkyle en C₃ à C₈ ; -(alkylène en C₁ à C₅)-(cycloalkyle en C₃ à C₈) ; hétérocycloalkyle ; -(alkylène en C₁ à C₅)-hétérocycloalkyle ; aryle ; hétéroaryle ; -(alkylène en C₁ à C₅)-aryle ; -(alkylène en C₁ à C₅)-hétéroaryle ;
R²¹ et R²² représentent, indépendamment l'un de l'autre dans chaque cas H, un reste alkyle en C₁ à C₁₀, alcényle en C₂ à C₁₀, alcynyle en C₂ à C₁₀ ; cycloalkyle en C₃ à C₈ ; - (alkylène en C₁ à C₅)-(cycloalkyle en C₃ à C₈) ; hétérocycloalkyle ; -(alkylène en C₁ à C₅)-hétérocycloalkyle ; aryle ; hétéroaryle ; -(alkylène en C₁ à C₅)-aryle ; -(alkylène en C₁ à C₅)-hétéroaryle ;
les restes alkyle en C₁ à C₁₀, alcényle en C₂ à C₁₀ et alcynyle en C₂ à C₁₀ mentionnés ci-dessus étant dans chaque cas linéaires ou ramifiés et pouvant éventuellement être substitués avec 1, 2, 3, 4 ou 5 substituants choisis indépendamment les uns des autres dans le groupe constitué de F ; Cl ; Br ; COOH ; COO-(alkyle en C₁ à C₄) ; -CN ; -OH ; -SH ; -O-(alkyle en C₁ ou C₂) ; -S-(alkyle en C₁ ou C₂) et -NH₂,
les restes cycloalkyle en C₃ à C₈ mentionnés ci-dessus pouvant éventuellement être substitués dans chaque avec 1, 2, 3, 4 ou 5 substituants choisis indépendamment les uns des autres dans le groupe constitué de F ; Cl ; Br ; -CN ; -OH ; -SH ; -alkyle en C₁ à C₅ ; -O-(alkyle en C₁ ou C₂) ; -S-(alkyle en C₁ ou C₂) et -NH₂,
les restes hétérocycloalkyle mentionnés ci-dessus ayant chacun 4, 5, 6 ou 7 chaînons, comprenant 1 ou 2 hétéroatomes choisis indépendamment l'un de l'autre dans le groupe constitué d'oxygène, soufre et azote comme chaînon(s) du noyau et pouvant éventuellement être substitués avec 1, 2, 3, 4 ou 5 substituants choisis indépendamment les uns des autres dans le groupe constitué de F ; Cl ; Br ; -CN ; -OH ; -SH ; -alkyle en C₁ à C₅ ; -O-(alkyle en C₁ ou C₂) ; -S-(alkyle en C₁ ou C₂) et -NH₂,
les restes aryle mentionnés ci-dessus représentant dans chaque cas un reste phényle, anthracényle ou naphtyle, ces restes pouvant être substitués indépendamment les uns des autres avec 1, 2, 3, 4 ou 5 substituants choisis dans le groupe constitué de F ; Cl ; Br ; I ; -CF₃ ; -OCF₃ ; -SCF₃ ; C(=O)(alkyle en C₁ à C₅) ; 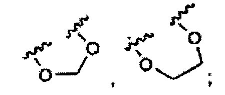 -NO₂ ; cyclohexyle ; -SF₅ ; -CN ; -OH ; -SH ; alkyle en C₁ à C₅ ; -O-(alkyle en C₁ à C₅) ; -S-(alkyle en C₁ à C₅) ; -C(=O)-OH ; -O(C=O)(alkyle en C₁ ou C₂) ; -C(=O)-O-(alkyle en C₁ à C₅) ; -NH₂ ; -N (H) (alkyle en C₁ à C₅) ; -N- (alkyle en C₁ à C₅)(alkyle en C₁ à C₅) ; -C(=O)NH₂ ; -C(=O)N(H)-(alkyle en C₁ à C₅) ; -C(=O)N(alkyle en C₁ à C₅) (alkyle en C₁ à C₅), -S(=O)₂NH₂ ; -S(=O)₂N(H)(alkyle en C₁ à C₅) ; -S(=O)₂-N(alkyle en C₁ à C₅) (alkyle en C₁ à C₅) ; -S(=O)₂-phényle ; -S(=O)₂-(alkyle en C₁ à C₅) ; phényle ; phénoxy ; benzyle ; benzyloxy ; thiophényle (thiényle), furannyle et pyridinyle,
les restes hétéroaryle mentionnés ci-dessus ayant 5 ou 6 chaînons, présentant 1, 2 ou 3 hétéroatomes choisis indépendamment les uns des autres dans le groupe constitué d'oxygène, soufre et azote comme chaînon(s) du noyau et pouvant éventuellement être substitués par 1, 2, 3, 4 ou 5 substituants choisis, indépendamment les uns des autres, dans le groupe constitué de F ; Cl ; Br ; I ; -CF₃ ; -OCF₃ ; -SCF₃ ; -SF₅ ; -CN ; -OH ; -SH ; -alkyle en C₁ à C₅ ; -O-(alkyle en C₁ à C₅) ; -S-(alkyle en C₁ à C₅) ; -C(=O)-OH ; -C(=O)-O-(alkyle en C₁ à C₅) ; -NH₂ ; -N (H) - (alkyle en C₁ à C₅) ; -N- (alkyle en C₁ à C₅)(alkyle en C₁ à C₅) ; -C(=)NH₂ ; -C(=O)N(H)(alkyle en C₁ à C₅) ; -C(=O)N(alkyle en C₁ à C₅)-(alkyle en C₁ à C₅), -S(=O)₂NH₂ ; -S(=O)₂N(H) (alkyle en C₁ à C₅) ; -S(=O)₂N(alkyle en C₁ à C₅)(alkyle en C₁ à C₅) ; -S(=O)₂phényle ; -S(=O)₂-(alkyle en C₁ à C₅) ; phényle ; phénoxy ; benzyle ; thiophényle (thiényle), furannyle et pyridinyle,
sous forme du racémate, des énantiomères, des diastéréo-isomères, de mélanges des énantiomères ou des diastéréo-isomères ou sous forme d'un énantiomère ou d'un diastéréoisomère individuel ; des bases et/ou de sels d'acides physiologiquement acceptables.

2. Composés suivant la revendication 1, dans lesquels
R¹, R², R³ et R⁴ représentent indépendamment les uns des autres, dans chaque cas,
H ; F ; Cl ; Br ; I ; -CN ; -NR⁷R⁸ ; -OR⁹ ; -SR¹⁰ ; alkyle en C₁ à C₄, alcényle en C₂ à C₄ ou alcynyle en C₂ à C₄ ;
R⁵ représente
un groupe -C(=S)NR²¹R²²
ou
un groupe (CHR⁶)ₙ-R²⁵
avec n = 1, 2 ou 3
R⁶ représentant H, ou un reste alkyle en C₁ à C₆, et R²⁵ représentant un reste aryle ou hétéroaryle,
R⁷ et R⁸ représentent, indépendamment l'un de l'autre,
dans chaque cas
H, un groupe -C(=O)R¹⁵ ou un reste alkyle en C₁ à C₄, ou bien
R⁷ et R⁸ forment, conjointement avec l'atome d'azote qui les lie en tant que chaînon du noyau, un reste qui est choisi entre morpholine, pipéridine ou pyrrolidine ;
R⁹, R¹⁰, représentent, indépendamment l'un de l'autre, dans chaque cas,
H ; un reste alkyle en C₁ à C₄, alcényle en C₂ à C₄, alcynyle en C₂ à C₄ ; cycloalkyle en C₃ à C₈ ; -(alkylène en C_{1,2 ou 3})-(cycloalkyle en C₃ à C₈) ; hétérocycloalkyle ; -(alkylène en C_{1,2 ou 3})-hétérocycloalkyle ; aryle ; hétéroaryle ; -(alkylène en C_{1,2 ou 3})-aryle ; - (alkylène en C_{1,2 ou 3})-hétéroaryle ;
R²¹ et R²² représentent, indépendamment l'un de l'autre, dans chaque cas
H ; un reste alkyle en C₁ à C₁₀, alcényle en C₂ à C₁₀, alcynyle en C₂ à C₁₀ ; cycloalkyle en C₃ à C₈ ; -(alkylène en C_{1,2 ou 3})-(cycloalkyle en C₃ à C₈) ; hétérocycloalkyle ; -(alkylène en C_{1,2 ou 3})-hétérocycloalkyle ; aryle ; hétéroaryle ; -(alkylène en C_{1,2 ou 3})-aryle ; -(alkylène en C_{1,2 ou 3})-hétéroaryle ;
les restes alkyle en C₁ à C₁₀, alcényle en C₂ à C₁₀ et alcynyle en C₂ à C₁₀ mentionnés ci-dessus étant dans chaque cas linéaires ou ramifiés et pouvant éventuellement être porter 1, 2, 3, 4 ou 5 substituants choisis indépendamment les uns des autres dans le groupe constitué de F ; Cl ; Br ; -CN ; COOH ; COO-(alkyle en C₁ à C₄) ; -OH ; -SH ; -O-(alkyle en C₁ ou C₂) ; -S-(alkyle en C₁ ou C₂) et -NH₂,
les restes alkyle en C₁ à C₄, alcényle en C₂ à C₄ et alcynyle en C₂ à C₄ mentionnés ci-dessus étant dans chaque cas linéaires ou ramifiés et pouvant éventuellement être porter 1, 2, 3, 4 ou 5 substituants choisis indépendamment les uns des autres dans le groupe constitué de F ; Cl ; Br ; -CN ; -OH ; -SH ; -OCH₃ et -NH₂,
les restes cycloalkyle en C₃ à C₈ mentionnés ci-dessus pouvant porter dans chaque éventuellement 1, 2, 3, 4 ou 5 substituants choisis indépendamment les uns des autres dans le groupe constitué de F ; Cl ; Br ; -CN ; -OH ; -CH₃ ; -C₂H₅ ; -OCH₃ et -NH₂,
les restes hétérocycloalkyle mentionnés ci-dessus ayant chacun 4, 5, 6 ou 7 chaînons, comprenant 1 ou 2 hétéroatomes choisis indépendamment l'un de l'autre dans le groupe constitué d'oxygène, soufre et azote comme chaînon(s) du noyau et pouvant éventuellement être substitués avec 1, 2, 3, 4 ou 5 substituants choisis indépendamment les uns des autres dans le groupe constitué de F ; Cl ; Br ; -CN ; -OH ; -CH₃ ; -C₂H₅ ; -OCH₃ et -NH₂,
les restes aryle mentionnés ci-dessus représentant dans chaque cas un reste phényle, anthracényle ou naphtyle, ces restes pouvant porter 1, 2, 3, 4 ou 5 substituants choisis indépendamment les uns des autres dans le groupe constitué de F ; Cl ; Br ; -CF₃ ; -OCF₃ ; -SCF₃ ; 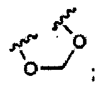 -NO₂ ; -C(=O)(alkyle en C₁ ou C₂) ; cyclohexyle ; -O(C=O)-(alkyle en C₁ ou C₂) ; -SF₅ ; -CN ; -OH ; méthyle ; éthyle ; n-propyle ; isopropyle ; n-butyle ; isobutyle ; sec.-butyle ; tertiobutyle ; méthoxy ; éthoxy ; -NH₂ ; -N(CH₃)₂) ; -N(C₂H₅)₂ ; phényle ; benzyloxy ; phénoxy ; benzyle ; thiophényle (thiényle), furannyle et pyridinyle, ;
les restes hétéroaryle mentionnés ci-dessus représentant dans chaque cas un reste furannyle, thiényle (thiophényle) ou pyridinyle et pouvant porter le cas échant 1, 2, 3, 4 ou 5 substituants choisis, indépendamment les uns des autres, dans le groupe constitué de F ; Cl ; Br ; -CF₃ ; -OCF₃ ; -SCF₃ ; -SF₅ ; -CN ; -OH ; méthyle ; éthyle ; n-propyle ; isopropyle ; n-butyle ; isobutyle ; sec.-butyle ; tertiobutyle ; méthoxy ; éthoxy ; -NH₂ ; -N(CH₃)₂ ; -N(C₂H₅)₂ ; phényle ; phénoxy ; benzyle ; thiophényle (thiényle), furannyle et pyridinyle,
sous forme du racémate, des énantiomères, des diastéréoisomères, de mélanges des énantiomères ou des diastéréoisomères ou d'un énantiomère ou d'un diastéréoisomère individuel ; des bases et/ou de sels d'acides physiologiquement acceptables.

3. Composés suivant l'une des revendications 1 et 2, dans lesquels R⁵ est un groupe -C(=S)NR²¹R²².

4. Composés suivant l'une des revendications 1 et 2, dans lesquels R⁵ est un groupe -(CHR⁶)ₙ-R²⁵.

5. Composés suivant la revendication 3, dans lesquels R²¹ représente H et R²² représente un reste benzyle, phényle, pyridyle, naphtyle ou phénéthyle, non substitué ou substitué une ou plusieurs fois avec un reste méthyle, éthyle, isopropyle, Cl, F, Br, NO₂, acétyle, CN, COOH, COO-(alkyle en C₁ à C₄), méthoxy, éthoxy, N(CH₃)₂, N(C₂H₅)₂, 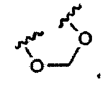 CF₃, SCH₃ ; alkyle en C₁ à C₁₀, ramifié ou non ramifié, saturé ou non saturé, non substitué ou substitué avec un radical OCH₃, COOCH₃ ou COOC₂H₅ ; un groupe cycloalkyle en C₃ à C₆, le groupe cycloalkyle pouvant être lié par l'intermédiaire d'un groupe CH₂ ; un groupe hétérocycloalkyle en C₅ ou C₆, le groupe hétérocycloalkyle pouvant être lié par l'intermédiaire d'un groupe CH₂.

6. Composés suivant la revendication 5, dans lesquels R²¹ représente H et R²² représente un reste benzyle, phényle, 2-méthylphényle, phénéthyle, 2-isopropylphényle, 2-chlorophényle, 4-fluorobenzyle, 1-(4-fluorophényl)éthyle, 4-chlorobenzyle, 4-chlorophénéthyle, 4-nitrophényle, 4-acétylphényle, 3-carboxyphényle, 3-méthylbenzoate, 4-éthylbenzoate, 2,6-diéthylphényle, 3-chloro-4-méthylphényle, 2-méthoxyéthyle, 3-méthoxypropyle, cyclopentyle, cyclohexyle, 3-pyridyle, 4-diméthylaminophényle, 4-diéthylaminophényle, CH₂COOCH₃, CH(CH₃)COOC₂H₅, CH(CH₃)CH₂COOC₂H₅, cyclohexylméthyle, 4-éthoxyphényle, 3,4-diméthoxyphényle, 1-naphtyle, 3,4,5-triméthoxyphényle, 2,3,4,5,6-pentafluorophényle, benzodioxole, 4-fluorophényle, méthyle, éthyle, propyle, isopropyle, tertiobutyle, allyle, 2-méthylprop-2-ényle, 2-nitrophényle, 3-trifluorométhylphényle, 2-trifluorométhylphényle, 4-trifluorométhylphényle, cyclopropyle, 2-méthylsulfanylphényle, 3-méthylsulfanylphényle, 4-méthylsulfanylphényle, 3,5-diméthylphényle, éthylmorpholine, ((4-propyl)-cyclohexyl)phényle, 4-bromo-2-trifluorométhylphényle, n-octyle, n-nonanyle, tétrahydrofurylméthyle, 2-éthylphényle, 4-cyanophényle, 3-cyanophényle, 2,6-diisopropylphényle, n-pentyle, n-hexyle, sec.-butyle, propylmorpholine, 5-chloro-2-méthoxyphényle, 4-chloro-3-trifluorométhylphényle, 3-chlorophényle, 1-phényléthyle, CH(CH₃)COOCH₃, 2-méthoxyphényle, 3-méthoxyphényle, 4-méthoxyphényle, CH₂CH₂COOC₂H₅, 2-méthylbenzoate, 4-méthylbenzoate, 2-éthylbenzoate, 2-fluorophényle, 2-méthoxy-5-chlorophényle ou 2,4-diméthoxyphényle.

7. Composés suivant la revendication 5, dans lesquels R⁶ représente H.

8. Composés suivant la revendication 5, dans lesquels R⁶ représente CH₃.

9. Composés suivant l'une des revendications 4, 7 ou 8, dans lesquels R²⁵ représente un reste phényle, pyridyle, thiényle ou furyle, chacun non substitué ou substitué une ou plusieurs fois avec CF₃, SCF₃, alkyle en C₁ à C₄, Cl, NO₂, O-acétyle, OCH₃, F, O-phényle, OCF₃, Br, O-benzyle, O-allyle, phényle, I, CN ou OH.

10. Composés suivant la revendication 9, dans lesquels R²⁵ représente un reste 4-trifluorométhylphényle, 4-SCF₃-phényle, 2-méthylphényle, phényle, anthracényle, 4-Cl-phényle, 4-OCF₃-phényle, 4-n-butylphényle, 3(3-CF₃-phénoxy)-phényle, 4-OCHF₂-phényle, 3,5-diméthylphényle, 3-bromo-4-méthoxyphényle, 4-benzyloxy-3,5-diméthylphényle, 3-nitrophényle, 3-méthoxy-4-(acétylméthyl)-phényle, 2,4,5-triméthoxyphényle, 4-éthylphényle, 3,4-dichlorophényle, 2,3,5-trifluorophényle, 4-phénoxyphényle, 3-chloro-4-fluorophényle, 3-benzyloxyphényle, 3-bromo-4,5-diméthoxyphényle, 3-fluoro-2-méthylphényle, 2-chloro-3-trifluorométhylphényle, 3-chloro-2-fluoro-5-trifluorométhylphényle, 2-fluoro-4-trifluorométhylphényle, 4-(allyloxy)-phényle, 2-(benzyloxy)-4,5-diméthoxyphényle, 2-phényl-phényle, 2,3,4-trifluorophényle, 2-fluoro-5-trifluorophényle, 4-méthoxy-3-méthylphényle, 2-fluoro-3-chlorophényle, 3,4-difluorophényle, 2,6-dichlorophényle, 3-iodophényle, 3-iodo-4,5-diméthoxyphényle, 2-cyanophényle, 4-hydroxyphényle, 3,4-diméthylphényle ou 3-OCF₃-phényle.

11. Composés suivant la revendication 1, choisis dans le groupe constitué de :
la benzo[d]isoxazole-3-yl-[1-(4-trifluorométhylphényl)-éthyl]amine,
la benzo[d]isoxazole-3-yl-[1-(4-trifluorométhylsulfanylphényl)-éthyl]amine,
la 1-benzo[d]isoxazole-3-yl-3-benzyl-thio-urée,
la 1-benzo[d]isoxazole-3-yl-3-phényl-thio-urée,
la 1-benzo[d]isoxazole-3-yl-3-o-tolyl-thio-urée,
la 1-benzo[d]isoxazole-3-yl-3-phénéthyl-thio-urée,
la 1-benzo[d]isoxazole-3-yl-3-(2-isopropylphényl)-thiourée,
la 1-benzo[d]isoxazole-3-yl-3-(2-chlorophényl)-thio-urée,
la 1-benzo[d]isoxazole-3-yl-3-(4-fluorobenzyl)-thio-urée,
la 1-benzo[d]isoxazole-3-yl-3-[1-(4-fluorophényl)-éthyl]-thio-urée,
la 1-benzo[d]isoxazole-3-yl-3-(4-chlorobenzyl)-thio-urée,
la 1-benzo[d]isoxazole-3-yl-3-[2-(4-chlorophényl)-éthyl]-thio-urée,
la 1-benzo[d]isoxazole-3-yl-3-(4-nitrophényl)-thio-urée,
la 1-(4-acétylphényl)-3-benzo[d]isoxazole-3-yl-thio-urée,
l'acide 3-(3-benzo[d]isoxazole-3-yl-thio-uréido)-benzoïque,
l'ester méthylique d'acide 3-(3-benzo[d]isoxazole-3-yl-thio-uréido)-benzoïque,
l'ester méthylique d'acide 4-(3-benzo[d]isoxazole-3-yl-thio-uréido)-benzoïque,
la 1-benzo[d]isoxazole-3-yl-3-(2,6-diéthylphényl)-thiourée,
la 1-benzo[d]isoxazole-3-yl-3-(3-chloro-4-méthylphényl)-thio-urée,
la 1-benzo[d]isoxazole-3-yl-3-(2-méthoxyéthyl)-thio-urée,
la 1-benzo[d]isoxazole-3-yl-3-(3-méthoxypropyl)-thio-urée,
la 1-benzo[d]isoxazole-3-yl-3-cyclopentyl-thio-urée,
la 1-benzo[d]isoxazole-3-yl-3-cyclohexyl-thio-urée,
la 1-benzo[d]isoxazole-3-yl-3-pyridine-3-yl-thio-urée,
la 1-benzo[d]isoxazole-3-yl-3-(4-diméthylaminophényl)-thio-urée,
la 1-benzo[d]isoxazole-3-yl-3-(4-diéthylaminophényl)-thio-urée,
l'ester méthylique d'acide (3-benzo[d]isoxazole-3-yl-thio-uréido)-acétique,
l'ester éthylique d'acide 2-(3-benzo[d]isoxazole-3-yl-thio-uréido)-propionique,
l'ester éthylique d'acide 3-(3-benzo[d]isoxazole-3-yl-thio-uréido)-butyrique,
la 1-benzo[d]isoxazole-3-yl-3-cyclohexylméthyl-thio-urée,
la 1-benzo[d]isoxazole-3-yl-3-(4-éthoxyphényl)-thio-urée,
la 1-benzo[d]isoxazole-3-yl-3-(3,4-diméthoxyphényl)-thio-urée,
la 1-benzo[d]isoxazole-3-yl-3-(3,4,5-triméthoxyphényl)-thio-urée,
la 1-benzo[d]isoxazole-3-yl-3-pentafluorophényl-thio-urée,
la 1-benzo[d]isoxazole-3-yl-3-naphtalène-1-yl-thio-urée,
la 1-benzo[1,3]dioxole-5-ylméthyl-3-benzo[d]isoxazole-3-yl-thio-urée,
la 1-benzo[d]isoxazole-3-yl-3-(4-fluorophényl)-thio-urée,
la 1-benzo[d]isoxazole-3-yl-3-méthyl-thio-urée,
la 1-benzo[d]isoxazole-3-yl-3-éthyl-thio-urée,
la 1-benzo[d]isoxazole-3-yl-3-propyl-thio-urée,
la 1-benzo[d]isoxazole-3-yl-3-isopropyl-thio-urée,
la 1-benzo[d]isoxazole-3-yl-3-tertiobutyl-thio-urée,
la 1-allyl-3-benzo[d]isoxazole-3-yl-thio-urée,
la 1-benzo[d]isoxazole-3-yl-3-(2-méthyl-allyl)-thio-urée,
la 1-benzo[d]isoxazole-3-yl-3-(2-nitrophényl)-thio-urée,
la 1-benzo[d]isoxazole-3-yl-3-(2-trifluorométhylphényl)-thio-urée,
la 1-benzo[d]isoxazole-3-yl-3-(3-trifluorométhylphényl)-thio-urée,
la 1-benzo[d]isoxazole-3-yl-3-(4-trifluorométhylphényl)-thio-urée,
la 1-benzo[d]isoxazole-3-yl-3-cyclopropyl-thio-urée,
l'ester méthylique d'acide 2-[3-(4-fluorobenzo[d]-isoxazole-3-yl)-thio-uréido]-propionique,
la 1-(4-chlorobenzo[d]isoxazole-3-yl)-3-o-tolyl-thio-urée,
la 1-benzyl-3-(4-chlorobenzo[d]isoxazole-3-yl)-thio-urée,
la 1-(4-chlorobenzo[d]isoxazole-3-yl)-3-(1-phényl-éthyl)-thio-urée,
la 1-(4-diméthylamino-benzo[d]isoxazole-3-yl)-3-isobutyl-thio-urée,
la 1-(4-diméthylamino-benzo[d]isoxazole-3-yl)-3-p-tolyl-thio-urée,
la 1-(3-chlorophényl)-3-(4-diméthylamino-benzo[d]isoxazole-3-yl)thio-urée,
la 1-(4-diméthylamino-benzo[d]isoxazole-3-yl)-3-(3-méthoxyphényl)-thio-urée,
la 1-(4-diméthylamino-benzo[d]isoxazole-3-yl)-3-(2-méthylsulfanylphényl)-thio-urée,
la 1-(4-diméthylamino-benzo[d]isoxazole-3-yl)-3-(3-méthylsulfanylphényl)-thio-urée,
la 1-(4-diméthylamino-benzo[d]isoxazole-3-yl)-3-(4-méthylsulfanylphényl)-thio-urée,
la 1-(4-diméthylamino-benzo[d]isoxazole-3-yl)-3-(2-méthoxyphényl)-thio-urée,
la 1-(4-diméthylamino-benzo[d]isoxazole-3-yl)-3-(4-méthoxyphényl)-thio-urée,
la 1-(4-diméthylamino-benzo[d]isoxazole-3-yl)-3-(3,5-diméthylphényl)-thio-urée,
la 1-benzyl-3-(4-diméthylamino-benzo[d]isoxazole-3-yl)-thio-urée,
la 1-(4-diméthylamino-benzo[d]isoxazole-3-yl)-3-(3-méthoxypropyl)-thio-urée,
l'ester éthylique d'acide 3-[3-(4-diméthylamino-benzo-[d]isoxazole-3-yl)-thio-uréido]-propionique,
l'ester éthylique d'acide 2-[3-(4-diméthylamino-benzo-[d]isoxazole-3-yl)-thio-uréido]-propionique,
l'ester éthylique d'acide 3-[3-(4-diméthylamino-benzo-[d]isoxazole-3-yl)-thio-uréido]-butyrique,
l'acide 3-[3-(4-diméthylaminobenzo[d]isoxazole-3-yl)-thio-uréido]-benzoïque,
la 1-(4-diméthylaminobenzo[d]isoxazole-3-yl)-3-(4-éthoxyphényl)-thio-urée,
l'ester méthylique d'acide 2-[3-(4-diméthylaminobenzo-[d]isoxazole-3-yl)-thio-uréido]-benzoïque,
l'ester méthylique d'acide 3-[3-(4-diméthylaminobenzo-[d]isoxazole-3-yl)-thio-uréido]-benzoïque,
l'ester méthylique d'acide 4-[3-(4-diméthylaminobenzo-[d]isoxazole-3-yl)-thio-uréido]-benzoïque,
l'ester éthylique d'acide 2-[3-(4-diméthylaminobenzo-[d]isoxazole-3-yl)-thio-uréido]-benzoïque,
l'ester éthylique d'acide 4-[3-(4-diméthylaminobenzo-[d]isoxazole-3-yl)-thio-uréido]-benzoïque,
la 1-(4-acétylphényl)-3-(4-diméthylaminobenzo[d]isoxazole-3-yl)-thio-urée,
la 1-(2-chlorophényl)-3-(4-méthoxybenzo[d]isoxazole-3-yl)-thio-urée,
la 1-(4-diéthylaminophényl)-3-(4-méthoxybenzo[d]-isoxazole-3-yl)-thio-urée,
la 1-(4-méthoxybenzo[d]isoxazole-3-yl)-3-(2-morpholine-4-yl-éthyl)-thio-urée,
l'ester méthylique d'acide 2-{3-[4-(2,2,2-trifluoréthoxy)-benzo[d]isoxazole-3-yl]-thio-uréido}-propionique,
la 1-benzo[1,3]dioxole-5-ylméthyl-3-[4-(2,2,2-trifluoréthoxy)-benzo[d]isoxazole-3-yl]-thio-urée,
la 1-[4-(4-propyl-cyclohexyl)-phényl]-3-[4-(2,2,2-trifluoréthoxy)-benzo[d]isoxazole-3-yl]-thio-urée,
la 1-(4-bromo-2-trifluorométhylphényl)-3-[4-(2,2,2-trifluoréthoxy)-benzo[d]isoxazole-3-yl]-thio-urée,
la 1-(4-méthoxyphényl)-3-[4-(2,2,2-trifluoréthoxy)-benzo-[d]isoxazole-3-yl]-thio-urée,
l'ester éthylique d'acide 3-{3-[4-(2,2,2-trifluoréthoxy)-benzo[d]isoxazole-3-yl]-thio-uréido}-propionique,
la 1-[4-(4-méthyl-benzyloxy)-benzo[d]isoxazole-3-yl]-3-octyl-thio-urée,
la 1-[4-(4-méthyl-benzyloxy)-benzo[d]isoxazole-3-yl]-3-nonyl-thio-urée,
la 1-cyclopropyl-3-[4-(4-méthyl-benzyloxy)-benzo[d]-isoxazole-3-yl]-thio-urée,
la 1-cyclopentyl-3-[4-(4-méthyl-benzyloxy)-benzo[d]-isoxazole-3-yl]-thio-urée,
la 1-cyclohexyl-3-[4-(4-méthyl-benzyloxy)-benzo[d]isoxazole-3-yl]-thio-urée,
la 1-cyclohexylméthyl-3-[4-(4-méthyl-benzyloxy)-benzo-[d]isoxazole-3-yl]-thio-urée,
la 1-(4-diméthylaminophényl)-3-[4-(2,2,2-trifluoréthoxy)-benzo[d]isoxazole-3-yl]-thio-urée,
la 1-allyl-3-(5-méthyl-benzo[d]isoxazole-3-yl)-thio-urée,
l'ester méthylique d'acide [3-(5-méthyl-benzo[d]isoxazole-3-yl)-thio-uréido]-acétique,
la 1-(2-isopropylphényl)-3-(5-méthyl-benzo[d]isoxazole-3-yl)-thio-urée,
la 1-(5-méthyl-benzo[d]isoxazole-3-yl)-3-(4-trifluorométhylphényl)-thio-urée,
l'ester méthylique d'acide 2-[3-(5-fluorobenzo[d]-isoxazole-3-yl)-thio-uréido]-propionique,
la 1-(5-fluorobenzo[d]isoxazole-3-yl)-3-(tétrahydrofuranne-2-ylméthyl)-thio-urée,
la 1-(5-bromobenzo[d]isoxazole-3-yl)-3-(2-fluorophényl)-thio-urée,
la 1-(5-bromobenzo[d]isoxazole-3-yl)-3-(2-éthylphényl)-thio-urée,
la 1-(6-chlorobenzo[d]isoxazole-3-yl)-3-(4-fluorophényl)-thio-urée,
la 1-(6-chlorobenzo[d]isoxazole-3-yl)-3-(2-fluorophényl)-thio-urée,
la 1-(6-chlorobenzo[d]isoxazole-3-yl)-3-cyclopentyl-thio-urée,
la 1-(6-chlorobenzo[d]isoxazole-3-yl)-3-(4-cyanophényl)-thio-urée,
l'acide 3-[3-(6-chlorobenzo[d]isoxazole-3-yl)-thio-uréido]-benzoïque,
la 1-(6-chlorobenzo[d]isoxazole-3-yl)-3-(4-méthoxyphényl)-thio-urée,
la 1-(6-chlorobenzo[d]isoxazole-3-yl)-3-(3-méthoxyphényl)-thio-urée,
la 1-(6-bromobenzo[d]isoxazole-3-yl)-3-(3,4-diméthoxyphényl)-thio-urée,
la 1-(6-bromobenzo[d]isoxazole-3-yl)-3-naphtalène-1-yl-thio-urée,
la 1-benzo[1,3]-dioxole[1,3]-5-ylméthyl-3-(6-bromobenzo-[d]isoxazole-3-yl)-thio-urée,
la 1-(6-fluorobenzo[d]isoxazole-3-yl)-3-(2-méthylsulfanylphényl)-thio-urée,
la 1-(3-cyanophényl)-3-(6-fluorobenzo[d]isoxazole-3-yl)-thio-urée,
la 1-(2-chloro-6-méthylphényl)-3-(6-fluorobenzo[d]-isoxazole-3-yl)-thio-urée,
la 1-(2,6-diisopropylphényl)-3-(6-fluorobenzo[d]isoxazole-3-yl)-thio-urée,
la 1-(7-fluorobenzo[d]isoxazole-3-yl)-3-méthyl-thio-urée,
la 1-éthyl-3-(7-fluorobenzo[d]isoxazole-3-yl)-thio-urée,
la 1-(7-fluorobenzo[d]isoxazole-3-yl)-3-propyl-thio-urée,
la 1-(7-fluorobenzo[d]isoxazole-3-yl)-3-pentyl-thio-urée,
la 1-(7-fluorobenzo[d]isoxazole-3-yl)-3-hexyl-thio-urée,
la 1-(7-fluorobenzo[d]isoxazole-3-yl)-3-octyl-thio-urée,
la 1-(7-fluorobenzo[d]isoxazole-3-yl)-3-nonyl-thio-urée,
la 1-(7-fluorobenzo[d]isoxazole-3-yl)-3-isobutyl-thio-urée,
la 1-allyl-3-(7-fluorobenzo[d]isoxazole-3-yl)-thio-urée,
la 1-(7-fluorobenzo[d]isoxazole-3-yl)-3-p-tolyl-thio-urée,
la 1-(5-bromobenzo[d]isoxazole-3-yl)-3-(4-diméthylaminophényl)-thio-urée,
la 1-(7-fluorobenzo[d]isoxazole-3-yl)-3-(2-morpholine-4-yl-éthyl)-thio-urée,
la 1-(7-fluorobenzo[d]isoxazole-3-yl)-3-(3-morpholine-4-yl-propyl)-thio-urée,
la 1-(4-méthoxybenzo[d]isoxazole-3-yl)-3-(1-phényléthyl)-thio-urée,
la 1-(4-chlorobenzyl)-3-(4-méthoxy-benzo[d]isoxazole-3-yl)-thio-urée,
la 1-(4-méthoxybenzo[d]isoxazole-3-yl)-3-(2-méthoxyphényl)-thio-urée,
la 1-(5-bromobenzo[d]isoxazole-3-yl)-3-(4-diméthylaminophényl)-thio-urée,
la 1-(5-chloro-2-méthoxyphényl)-3-(4-méthoxybenzo[d]-isoxazole-3-yl)-thio-urée,
la 1-(4-chloro-3-trifluorométhylphényl)-3-(4-méthoxybenzo[d]isoxazole-3-yl)-thio-urée,
la 1-(2,4-diméthoxyphényl)-3-(4-méthoxy-benzo[d]isoxazole-3-yl)-thio-urée,
la 1-[4-(2,2,2-trifluoréthoxy)-benzo[d]isoxazole-3-yl]-3-(3,4,5-triméthoxyphényl)-thio-urée,
la benzo[d]isoxazole-3-yl-(3-méthyl-butyl)-aminé,
la (5-fluorobenzo[d]isoxazole-3-yl)-(2-méthyl-benzyl)-amine,
la N4,N4-diméthyl-N3-(3-phényl-propyl)-benzo[d]isoxazole-3,4-diamine,
la N3-butyl-N4,N4-diméthyl-benzo[d]isoxazole-3,4-diamine,
l'anthracène-9-ylméthyl-(4-méthoxybenzo[d]isoxazole-3-yl)-amine,
la (4-chlorobenzyl)-(4-méthoxybenzo[d]isoxazole-3-yl)-amine,
la (6-fluorobenzo[d]isoxazole-3-yl)-(3-nitrobenzyl)-amine,
l'ester 4-[(6-chlorobenzo[d]isoxazole-3-ylamino)-méthyl]-2-méthoxyphénylique d'acide acétique,
l'ester 4-[(6-bromobenzo[d]isoxazole-3-ylamino)-méthyl]-2-méthoxyphénylique d'acide acétique,
la benzo[d]isoxazole-3-yl-(3,4-dichlorobenzyl)-amine,
la benzo[d]isoxazole-3-yl-(2,4,5-triméthoxybenzyl)-amine,
la benzo[d]isoxazole-3-yl-(4-éthylbenzyl)-amine,
la (6-chlorobenzo[d]isoxazole-3-yl)-(3,4-dichlorobenzyl)-amine,
la benzo[d]isoxazole-3-yl-(2,3,5-trifluorobenzyl)-amine,
la (6-chlorobenzo[d]isoxazole-3-yl)-(4-phénoxybenzyl)-amine,
la (3-chloro-4-fluorobenzyl)-(7-fluorobenzo[d]isoxazole-3-yl)-amine,
la benzo[d]isoxazole-3-yl-(4-trifluorométhylbenzyl)-amine,
la (7-fluorobenzo[d]isoxazole-3-yl)-(2-méthylpentyl)-amine,
la N4,N4-diméthyl-N3-(2,3,4-trifluorobenzyl)-benzo[d]-isoxazole-3,4-diamine,
la N3-(2-fluoro-5-trifluorométhylbenzyl)-N4,N4-diméthyl-benzo[d]isoxazole-3,4-diamine,
la N3-(4-méthoxy-3-méthylbenzyl)-benzo[d]isoxazole-3,4-diamine,
la N3-(4-méthoxy-3-méthylbenzyl)-benzo[d]isoxazole-3,4-diamine,
la benzo[d]isoxazole-3-yl-(4-trifluorométhoxybenzyl)-amine,
la (5-fluorobenzo[d]isoxazole-3-yl)-(4-trifluorométhoxybenzyl)-amine,
la benzo[d]isoxazole-3-yl-(4-trifluorométhylsulfanylbenzyl)-amine,
la (4-butylbenzyl)-(6-chlorobenzo[d]isoxazole-3-yl)-amine,
la (5-fluorobenzo[d]isoxazole-3-yl)-(4-trifluorométhylsulfanylbenzyl)-amine,
la benzo[d]isoxazole-3-yl-(2-fluoro-4-trifluorométhylbenzyl)-amine,
la (7-fluorobenzo[d]isoxazole-3-yl)-(4-trifluorométhoxybenzyl)-amine,
la (7-fluorobenzo[d]isoxazole-3-yl)-[3-(3-trifluorométhylphénoxy)-benzyl]-amine,
la (4-difluorométhoxybenzyl)-(4-méthoxybenzo[d]isoxazole-3-yl)-amine,
la (3,5-diméthylbenzyl)-(7-fluorobenzo[d]isoxazole-3-yl)-amine,
la (3-bromo-4-méthoxybenzyl)-(6-fluorobenzo[d]isoxazole-3-yl)-amine,
la (3,5-diméthylbenzyl)-(6-fluorobenzo[d]isoxazole-3-yl)-amine,
la (4-benzyloxy-3,5-diméthylbenzyl)-(6-fluorobenzo[d]-isoxazole-3-yl)-amine,
la (4-butylbenzyl)-(6-fluorobenzo[d]isoxazole-3-yl)-amine,
la (6-fluorobenzo[d]isoxazole-3-yl)-(4-trifluorométhylsulfanylbenzyl)-amine,
la (3-benzyloxybenzyl)-(6-fluorobenzo[d]isoxazole-3-yl)-amine,
la N3-(3,5-diméthylbenzyl)-benzo[d]isoxazole-3,4-diamine,
la N3-(4-butylbenzyl)-benzo[d]isoxazole-3,4-diamine,
la (5-bromobenzo[d]isoxazole-3-yl)-(4-trifluorométhylsulfanylbenzyl)-amine,
la (3-bromo-4,5-diméthoxybenzyl)-(7-fluorobenzo[d]-isoxazole-3-yl)-amine,
la (7-fluorobenzo[d]isoxazole-3-yl)-(2-fluoro-4-trifluorométhylbenzyl)-amine,
la N3-(3-fluoro-2-méthylbenzyl)-N4,N4-diméthyl-benzo-[d]isoxazole-3,4-diamine,
la N3-(2-chloro-3-trifluorométhylbenzyl)-N4,N4-diméthylbenzo[d]isoxazole-3,4-diamine,
la N3-(3-chloro-2-fluoro-5-trifluorométhylbenzyl)-N4,N4-diméthyl-benzo[d]isoxazole-3,4-diamine,
la (6-fluorobenzo[d]isoxazole-3-yl)-(2-fluoro-4-trifluorométhylbenzyl)-amine,
la (4-allyloxy-benzyl)-(6-fluorobenzo[d]isoxazole-3-yl)-amine,
la benzo[d]isoxazole-3-yl-(2-benzyloxy-4,5-diméthoxybenzyl)-amine,
la (2-benzyloxy-4,5-diméthoxybenzyl)-(6-chlorobenzo[d]-isoxazole-3-yl)-amine,
la N3-(2-benzyloxy-4,5-diméthoxybenzyl)-N4,N4-diméthylbenzo[d]isoxazole-3,4-diamine,
la N3-biphényl-2-ylméthyl-N4,N4-diméthylbenzo[d]isoxazole-3,4-diamine,
la (6-fluorobenzo[d]isoxazole-3-yl)-(3-iodo-benzyl)-amine,
la (2-benzyloxy-4,5-diméthoxybenzyl)-(4-méthoxybenzo-[d]isoxazole-3-yl)-amine,
la (4-fluorobenzo[d]isoxazole-3-yl)-(3-iodo-4,5-diméthoxybenzyl)-amine,
le 2-[(5-méthyl-benzo[d]isoxazole-3-ylamino)-méthyl]-benzonitrile,
la butyl-[4-(2,2,2-trifluoréthoxy)-benzo[d]isoxazole-3-yl]-amine,
la (3-bromo-4,5-diméthoxybenzyl)-(5-méthyl-benzo[d]-isoxazole-3-yl)-amine,
le 4-[(4-chlorobenzo[d]isoxazole-3-ylamino)-méthyl]-phénol,
la (6-chlorobenzo[d]isoxazole-3-yl)-(3,4-diméthylbenzyl)-amine,
la (4-chlorobenzo[d]isoxazole-3-yl)-(3-chloro-2-fluorobenzyl)-amine,
la (3,4-difluorobenzyl)-(5-fluorobenzo[d]isoxazole-3-yl-amine,
la (6-bromobenzo[d]isoxazole-3-yl)-(2,6-dichlorobenzyl)-amine,
la (7-fluorobenzo[d]isoxazole-3-yl)-(3-trifluorométhoxybenzyl)-amine,
ainsi que dans chaque cas leurs sels correspondants, en particulier leurs sels d'addition consistant en chlorhydrates, et le cas échéant, leurs produits de solvatation correspondants.

12. Procédé de production des composés substitués de benso[d]isoxazole-3-yl-amine conformes à l'invention, conformément auquel un composé de 2-fluorobenzonitrile éventuellement substitué de formule générale II 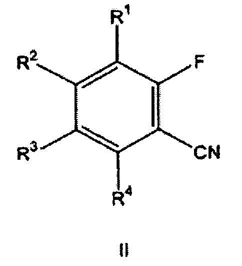 dans laquelle les restes R¹, R², R³ et R⁴ ont la définition indiquée ci-dessus, est amené à réagir, dans un milieu de réaction préférentiellement choisi dans le groupe constitué d'éther de diéthyle, tétrahydrofuranne, acétonitrile, diméthylsulfoxyde, diméthylformamide et dichlorométhane, en présence d'une base, avantageusement en présence d'au moins un alcoolate sel de métal alcalin, très avantageusement en présence d'un alcoolate sel de métal alcalin choisi dans le groupe constitué de méthanolate de potassium, méthanolate de sodium, tertiobutylate de potassium et tertiobutylate de sodium, avec l'acide acéthydroxamique de formule III 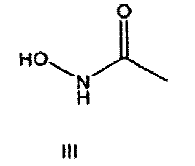 avantageusement à des températures de 20°C à 100°C pour former un composé de formule générale I 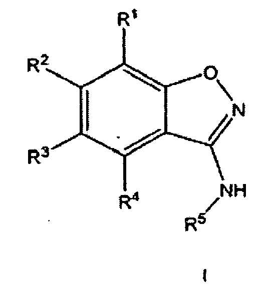 dans laquelle les restes R¹-R⁴ ont la définition indiquée ci-dessus de même que le reste R⁵ représente un reste hydrogène, on purifie éventuellement ce composé et on l'isole le cas échéant,
ensuite on le fait éventuellement réagir dans un milieu de réaction avantageusement choisi dans le groupe constitué d'acétonitrile, toluène, diméthylformamide, benzène, éthanol, méthanol et des mélanges correspondants, avec au moins un isothiocyanate de formule générale S=C-N-R2², dans laquelle R²² a la définition indiquée ci-dessus, éventuellement en présence d'au moins une base, préférentiellement en présence d'au moins une base choisie dans le groupe constitué de triéthylamine, 4,4-diméthylaminopyridine et diisopropyl-éthylamine, pour obtenir au moins un composé de formule générale I dans laquelle R¹, R², R³ et R⁴ ont la définition mentionnée ci-dessus et R⁵ représente un groupe -C(=S)-NR²¹R²² où R²² a la définition indiquée ci-dessus et R²¹ représente un reste hydrogène, on purifie éventuellement ce composé et on l'isole le cas échéant,
et le cas échéant on fait réagir au moins un composé de formule générale I, dans laquelle R¹, R², R³ et R⁴ ont la définition indiquée ci-dessus, R⁵ représente un groupe -C(=S)-NR²¹R²² où R²² a la définition indiquée ci-dessus et R²¹ représente un reste hydrogène, dans un milieu de réaction, avantageusement choisi dans le groupe constitué d'acétonitrile, toluène, diméthylformamide, benzène, éthanol, méthanol et des mélanges correspondants, en présence d'au moins une base, préférentiellement en présence d'au moins un hydrure métallique ou d'un sel qui est un alcoolate métallique, très avantageusement en présence d'un hydrure métallique ou d'un alcoolate métallique choisis dans le groupe constitué d'hydrure de sodium, hydrure de potassium, tertiobutanolate de potassium, tertiobutanolate de sodium, méthanolate de potassium, méthanolate de sodium, éthanolate de sodium et éthanolate de potassium, avec au moins un composé de formule générale LG-R²¹ dans laquelle LG représente un groupe partant, préférentiellement un atome d'halogène, très avantageusement un atome de chlore, et R²¹ a la définition indiquée ci-dessus à l'exception de l'hydrogène, pour obtenir au moins un composé de formule générale I qu'on purifie éventuellement et/ou qu'on isole le cas échéant,
ou bien
on fait réagir éventuellement au moins un composé de formule générale I, dans laquelle R¹, R², R³ et R⁴ ont la définition indiquée ci-dessus et R⁵ représente H, dans un milieu de réaction, avantageusement choisi dans le groupe constitué d'acétonitrile, toluène, diméthylformamide, benzène, éthanol, méthanol, DCM, acide trifluoracétique ou des mélanges correspondants, en présence d'au moins une base, préférentiellement en présence d'au moins un sel formé d'un hydrure métallique ou d'un alcoolate métallique ou de triéthylsilane, très avantageusement en présence du triéthylsilane, d'un sel formé d'un hydrure métallique ou d'un alcoolate métallique choisis dans le groupe constitué d'hydrure de sodium, hydrure de potassium, tertiobutanolate de potassium, tertiobutanolate de sodium, méthanolate de potassium, méthanolate de sodium, éthanolate de sodium et éthanolate de potassium, avec au moins un composé de formule générale R³⁰C(=O)H ou R⁶C(O)R²⁵, où R⁶ et R²⁵ ont la définition indiquée ci-dessus et R³⁰ représente un reste aliphatique linéaire ou ramifié, saturé ou non saturé, non substitué ou substitué au moins une fois ; un reste aryle ou hétéroaryle non substitué ou substitué au moins une fois, et peut être condensé avec un système de noyau monocyclique ou polycyclique ; ou un reste aryle ou hétéroaryle non substitué ou substitué au moins une fois, qui peut être condensé avec un système de noyau monocyclique ou polycyclique et qui est lié par l'intermédiaire d'un groupe alkylène linéaire ou ramifié, pour obtenir au moins un composé de formule générale I qu'on purifie le cas échéant et/ou qu'on isole éventuellement.

13. Médicament contenant au moins un composé suivant la revendication 1 et, le cas échéant, une ou plusieurs substances auxiliaires physiologiquement acceptables.

14. Utilisation d'au moins un composé suivant la revendication 1 pour la préparation d'un médicament destiné au traitement de la douleur, de la migraine, d'états d'anxiété, d'incontinence urinaire ou d'épilepsie.

15. Utilisation d'au moins un composé suivant la revendication 1 pour la préparation d'un médicament destiné au traitement de la douleur choisie dans le groupe constitué de douleur aiguë, douleur chronique, douleur neuropathique, douleur musculaire et douleur inflammatoire.
